# EUROPEAN PATENT SPECIFICATION

(11) **EP 3 003 375 B1**
(45) Date of publication and mention of the grant of the patent: **20.11.2019**
(21) Application number: 14733966.7
(22) Date of filing: 04.06.2014
(51) Int. Cl.: A61K 31/498, A61L 2/08, A61K 31/4709, A61K 31/4745, A61K 31/496, A61N 5/06, A61K 41/00, A61K 31/704, A61K 31/65, A61K 31/5383, A61K 31/7048, A61B 17/00, A61F 9/008, A61N 5/00, A61B 90/00

(54) **METHOD OF APPLYING A COMPOSITION AND PHARMACEUTICAL COMPOSITION WITH A REGIMEN OF ADMINISTERING IT**
VERFAHREN ZUM AUFBRINGEN EINER ZUSAMMENSETZUNG UND PHARMAZEUTISCHE ZUSAMMENSETZUNG MIT EINEM SCHEMA ZUR VERABREICHUNG DAVON
PROCÉDÉS D'APPLICATION D'UNE COMPOSITION, ET COMPOSITION PHARMACEUTIQUE ET SON RÉGIME D'ADMINISTRATION

(30) Priority: 05.06.2013 CH 10682013
(43) Date of publication of application: 13.04.2016
(73) Proprietor: Hafezi, Farhad, 6340 Baar (CH)
(72) Inventor: HAFEZI Farhad, 6340 Baar (CH); RICHOZ Oliver, 2300 La Chaux-de-Fonds (CH)
(74) Representative: Frei Patent Attorneys
(86) International application number: PCT/CH2014/000075
(87) International publication number: WO 2014/194435

(56) References cited:
- WO-A1-2009/042159
- WO-A1-2009/124189
- WO-A1-2012/013270
- WO-A1-2012/122210
- WO-A2-2005/034997
- JP-A- 2008 079 510
- US-A1- 2003 153 962
- US-A1- 2008 131 968
- US-A1- 2011 054 305
- DATABASE WPI Week 200835 Thomson Scientific, London, GB; AN 2008-F14405 XP002728474, -& JP 2008 079510 A (UNIV YAMAGUCHI) 10 April 2008 (2008-04-10)
- BECERRA M C ET AL: "Light effect and reactive oxygen species in the action of ciprofloxacin on Staphylococcus aureus", JOURNAL OF PHOTOCHEMISTRY AND PHOTOBIOLOGY B: BIOLOGY, ELSEVIER SCIENCE S.A., BASEL, CH, vol. 76, no. 1-3, 25 October 2004 (2004-10-25), pages 13-18, XP004602797, ISSN: 1011-1344, DOI: 10.1016/J.JPHOTOBIOL.2004.06.003
- HIRO YASUKAWA ET AL: "Photomanipulation of antibiotic susceptibility and biofilm formation of Escherichia coli heterologously expressing photoactivated adenylyl cyclase", THE JOURNAL OF GENERAL AND APPLIED MICROBIOLOGY, vol. 58, no. 3, 1 January 2012 (2012-01-01), pages 183-190, XP055134741, ISSN: 0022-1260, DOI: 10.2323/jgam.58.183
- YANAGAWA T ET AL: "Photodynamic bactericidal effect of tetracycline on methicillin resistant Staphylococcus aureus", LASERS IN THE LIFE SCIENCE, HARWOOD ACADEMIC PUBLISHERS, GB, vol. 6, no. 3, 1 January 1994 (1994-01-01) , pages 187-196, XP008171251, ISSN: 0886-0467

## Description

The invention relates to a method of applying a composition to an object or a surface area and to a pharmaceutical composition with a regimen of administering it to a human or non-human individual. In particular the invention relates to the issue of resistance to drugs and chemical agents which have partially or fully lost their effectiveness in killing or retarding proliferation of living cells and organisms. The method and pharmaceuticals find applications in various technical fields such as human and veterinary medicine, and industries including sanitary, food and agricultural and other technologies.

The invention is defined in the claims, other embodiments being merely exemplary. In particular, methods of treatment of a human or animal body by surgery or therapy do not constitute a part of the invention.

It is well known that antimicrobial treatments, disinfection and pest control provoke the development of cells, viruses and organisms which are resistant to the drugs or chemical agents used, especially if they are used repeatedly or over long periods of time. Some multi-drug resistant bacteria and other pathogens acquired resistance to almost all of the currently available antibiotics and antimicrobials and pose a threat to the treatment of infectious disease. Similarly, cancer cells resistant to antineoplastic drugs are known and often impede successful treatment of cancer. Furthermore, large numbers of microbes, fungi, weeds, insects and other pest are known which are resistant to agricultural pesticides and / or disinfectants used in industries including such as agriculture, food processing and packaging industries.

A number of different mechanisms have been described, by which living cells can acquire resistance to drugs and chemical agents such as disinfectants or pesticides. One such resistance mechanism involves a pump and/or pumps located in the cell membrane of the targeted cells, which efficiently pump out drug molecules as soon as they have entered the target pathogenic, infected or neoplastic target cell. A further mechanism involves reduced drug uptake for example by modification of the receptor or transport proteins involved in drug uptake into the cell. An even further mechanism involves modification or degradation of the active ingredient of a drug by enzymes within the target cell. Yet a further mechanism involves a decreased affinity of the molecule which is targeted by the antibiotic, e.g. due to a mutational change of the target cell resulting in a targeted molecule with decreased affinity for the antibiotic.

Drug resistance of pathogens and neoplastic or infected cells prevents or procrastinates the healing of individuals suffering from infectious disease and cancer. Furthermore, resistant pathogens are hard to control and to prevent from spreading to new host individuals, thus posing a public health risk. Drug resistance not only causes loss of life but also material loss. For example multi-drug resistant pathogens cause nosocomial transfer of disease and infections in hospital e.g. during or after surgery, thereby besides causing human suffering also causing higher cost due to prolonged medical treatment at the hospital. Other examples of material loss include lost crop due to pesticide resistant organisms and spoiled food due to resistant microbes. Therefore, continuing expensive research efforts are directed for the development of new active ingredients for medical treatment, disinfection and pest control in the battle against multi-resistant viruses, living cells and organisms.

A technical object of the current invention is a reduction of loss in human and animal life and a reduction in material loss due to resistance against drugs and other chemical agents. In particular, it is an object of the invention to kill or retard proliferation of living cells and organisms, which have acquired resistance to a drug or chemical agent.

It is thus one object of the invention to provide an effective therapeutic regimen of pharmaceuticals for the treatment of disease caused by drug-resistant pathogens and cancer cells. In particular, it is an object of this aspect of the invention to provide a therapeutic regimen for pharmaceuticals, which overcomes or reduces drug resistance.

It is the object of a further aspect of the invention to provide methods of application of chemical agents against pathogens, pest and tumor cells, in particular where the chemical agents otherwise have lost their effect or have a lower effect in the elimination of pathogens, pest or cancer cells. Such methods, according to this aspect of the invention, include but are not limited to disinfection, sterilization, sanitary and pest control methods and they relate to technical fields including but not limiting to e.g. medicine, agriculture, food and packaging industries and other.

It is the object of yet another aspect to provide equipment useable in the therapeutic regimen for pharmaceuticals.

The technical problem is solved according to the independent claims. The dependent claims relate to some embodiments of the invention.

A method is provided of applying a composition to a human or non-human individual or to an object or a surface area and a pharmaceutical composition with a regimen of administering it to a human or non-human individual is provided, wherein the composition comprises an active chemical component for killing of or retarding proliferation of target cells including pathogens, infected cells and cancer cells. The application of the composition and the regimen of administration of the composition are accompanied by at least one exposure to electromagnetic radiation in a range of wavelength, in which the active chemical component absorbs, and which has a lower limit of 190 nm. This absorption of electromagnetic wavelength by the active component results in its photo-activation. The electromagnetic radiation is applied in short pulses in a range of 0.1 fs to 200 ms.

The condition that absorption of electromagnetic range in a wavelength range with a lower limit of 190 nm does not, of course, exclude that the component also absorbs radiation below 190 nm. Neither does it imply that the component must absorb at 190 nm. Rather, it means that the absorption spectrum of the active chemical component includes substantial absorption at wavelengths longer than 190 nm, and that the exposure takes place by radiation that includes radiation portions that have wavelengths above 190 nm and are in a part of the spectrum where the active chemical component exhibits substantial absorption.

Substantial absorption takes place at wavelengths corresponding to energies that correspond to energy differences between energy states of the active chemical component molecule (or in case of multiple-photon-processes, a well-defined fraction thereof). "Substantial absorption" is restricted to absorption by the molecule itself, not by impurities, vessels, etc.

Exposure to radiation generally means exposure by an actively controlled, targeted, for example electrically powered radiation source, and in this it is different from the mere exposure to ambient light. Generally, it will be substantially higher than the exposure to the corresponding spectral portion of ambient light, for example by at least a factor 2, at least a factor 10, or at least a factor 100. Nevertheless, in certain situations the radiation intensity can be very low and still satisfy this condition, for example if the exposure takes place in an interior of a human or non-human body.

In embodiments, the step of exposing the composition to radiation comprises using a radiation source as defined and described in more detail hereinafter.

According to a one aspect of the invention, a pharmaceutical composition comprising an active chemical component is provided, which composition kills or retards proliferation of target cells, including, but not limited, to pathogens, infected cells or neoplastic tumor cells, and which composition absorbs electromagnetic radiation in a range of wavelength with a lower limit of 190 nm, for therapeutic or preventive treatment of infectious disease and/or tumors of a human or non-human individual. The treatment is accompanied by at least one exposure of the individual to electromagnetic radiation of a wavelength or a range of wavelengths, at least some of which is absorbed by the active chemical component and the electromagnetic radiation is applied in short pulses in a range of 0.1 fs to 200 ms.

The term 'pharmaceutical composition' in this text relates to the pharmaceutical composition administered with this regimen of treatment including exposure to electromagnetic radiation. The regimen of medical treatment includes therapeutic, preventive and surgical applications.

According to a further aspect of the invention, a method of applying a composition to an object or a surface area is provided, wherein the composition comprises an active chemical component for killing of or for retarding proliferation of target cells wherein the application or administration of the composition is accompanied by at least one exposure to electromagnetic radiation in a range of wavelength, in which the active chemical component absorbs and which wavelength or range of wavelengths has a lower limit of 190 nm. The electromagnetic radiation is applied in short pulses in a range of 0.1 fs to 200 ms.

In some embodiments of the method of applying a composition to an object, surface area or a human or non-human individual, the method serves an industrial purpose of obtaining a germ and pest free environment by killing or reducing the proliferation of target cells including but not limited to applications for a sanitary purpose or for disinfection, in particular in the fields of medicine, in the food industry or agriculture.

According to some embodiments of the method of applying a composition including the step of photo-activation of an active component of the composition, the composition is administered to a human or non-human individual but does not serve the medical treatment of this human or non-human individual, which is subjected to the method. For example, in some of these embodiments, the method is used for disinfection of human individuals e.g. staff in a hospital or in other industries where a germfree environment is desired or in agriculture for disinfection of animals prior to harvesting an animal product such as e.g. milk or wool. In one exemplary embodiment multi-drug resistant pathogens responsible for nosocomial transfer of disease and infections are killed or reduced in a hospital setting.

According to some further embodiments of the method of applying a composition including the step of photo-activation of an active component, the composition is not applied to an individual but to an object and in particular to the surface of an object.

According to some further embodiments of the method, the composition is applied to exterior surfaces and objects including for example fields and other farm land, sport fields, parks, streets exterior surfaces of objects as well as and interior surfaces including interior surfaces e.g. of buildings and vehicles.

According to some embodiments of the method of applying a composition including a step of photo-activation of an active component, the method serves the medical treatment of human or non-human individuals including preventive and therapeutic applications and applications during surgery.

In this text, unless specifically indicated to the contrary, the term 'method' refers to methods of applying a composition, which can be a pharmaceutical or another composition, and it applies to medical and non-medical methods. Similarly, in this text, unless specifically indicated to the contrary, the term 'composition' refers to both pharmaceutical compositions and to further compositions used in the method such as chemical agents used as pesticides, disinfectants etc. Thus, the compositions can be used for a medical or a non-medical purpose or both. Sometimes in this text the term 'pharmaceutical composition' or 'pharmaceutical' is used to emphasize the medical application of the method.

The term 'active component' - of the pharmaceutical composition administered or of the composition applied - refers to at least one active ingredient of the composition, which interacts with the target cells. Thus, it is the active component of a pharmaceutical administered to an individual or of a composition applied to an object or a surface or an individual, which interacts with the target cells or more particularly with a targeted molecule, i.e. a molecular component of the target cells and thereby kills or reduces the proliferation the target cells. In some particular embodiments the active component can be a toxine secreted by bacteria such as e.g. the pyocyanin secreted pseudomonas. In these embodiments the bacterial toxin attacks resistant target cells.

The term 'photo-activation' - of an active component - refers to a step of the method of applying the composition to the human or non-human individual or to the object or the surface area and/or to a step of the regimen of administrating the pharmaceutical composition, which step comprises the exposure to electromagnetic radiation in a range of wavelength, in which the active chemical component absorbs (exhibits substantial non-zero absorption) and which has a lower limit of 190 nm. This absorption of electromagnetic wavelength by the active component results in its photo-activation, and accordingly the term 'photo-activated active component' - of the pharmaceutical composition administered or of the composition applied - refers to an active component which has absorbed at least some of the electromagnetic radiation to which it has been exposed to during the therapeutic regimen of the pharmaceutical composition or during the method of applying a composition.

In this text the term 'target cell' refers to cells of a unicellular or multi-cellular living organisms at which a particular pharmaceutical drug or a particular chemical composition is targeted. Target cells include pathogens and cells of a human or animal individual, which are targeted by drugs. Target cells such as bacterial, fungal, parasitical, and infected cells, which may be pathogenic and further include e.g. neoplastic tumor cells, in particular cancer cells. The term 'pathogen' refers to viruses, as well as living cells and organisms such as bacterial cells and eukaryotic cells of living organisms including protists, fungi, plants, or animals such as e.g. insects or helminthes. The term 'infected cells' refers to cells of the human or non-human individual subjected to the method or the pharmaceutical composition, which cells are infected by virus or another infectious, in particular pathogenic, agent such as e.g. a prion or a protist.The term "anti-infectant" refers to a pharmaceutical drug, a particular chemical compound or a particular chemical composition acting against infection by inhibiting the spread of an infectious agent or by killing the infectious agent. The term anti-infectant encompasses antibiotics (including antibacterials, antifungals, and antiprotozoans), and antivirals.

The term "antibiotic" stands for low molecular substances of natural, semi-synthetic or synthetic origin that inhibit or abolish the growth of microorganisms, such as bacteria, fungi or protozoans. An antibiotic may also be administered for treatment of a tumour or cancer cell.

One embodiment of the present invention comprises administering an pharmaceutical compound or composition such as for example an anti-infectant to a target cell, wherein a target cell is for example, as described above, a bacterial, fungal, parasitical or infected cell. In embodiments, the active component of the composition is an anti-infectant. In specific embodiments, the active component is an antibiotic, especially an antibacterial substance.

In one specific embodiment an antibiotic is administered to a target cell as defined above.

In a further embodiment of the present invention a cytostatic compound or composition may be administered to target cells as defined above, i.e. to treat tumour cells, infected cells, bacterial, fungal or parasitic cells. Cytostatics inhibit cell growth and cell proliferation.

In another group of embodiments, the active component is documented to exert its effect from within a cell. As an example the active component is a component the documented effect of which is inhibition of mitosis and/or meiosis or inhibition of the function or synthesis of certain enzymes within the target cell or to change or destroy the structure of genetic material (for example DNA, or RNA) or other vital molecules, such as amino acids or proteins, such as enzymes within the target cell.

Some embodiments of the pharmaceuticals and methods are directed at only one type of target cell and some embodiments are directed against several types of target cells at the same time, e.g. for the treatment of multiple infections.

The term 'resistant target cells' refers to target cells which have developed a resistance mechanism to escape the detrimental effect of at least one active component, which effect the drug would exert on non-resistant target cells, i.e. to the same cells prior to acquisition of a resistance mechanism.

In particular, the pharmaceuticals and methods are also applicable to combat multi-drug resistant target cells including but not limited to multi-drug resistant bacteria or multi-drug resistant microbes. In particular, some embodiments relate to a pharmaceutical composition or to a method comprising a composition for overcoming the resistance of resistant target cells. In some of these embodiments the regimen of the pharmaceutical or the method overcomes the resistance of resistant target cells which resistance is mediated in particular by a pump preventing or reducing the uptake of the active component into the target cells or by an enzyme degrading the active component, wherein the enzyme can be an intracellular enzyme of the resistant target cell or an enzyme which is secreted by the resistant target cell or by decreasing the affinity of the targeted molecule of the target cell.

In general, effectiveness of a compound or a composition such as an anti-infectant varies with the location of its target cell, the ability of said anti-infectant to reach said target cell and the ability of the target cell (for example a tumor cell or a microorganism) to inactivate or to excrete the drug.

Access of a pharmaceutical compound or composition as defined above into a cell is usually achieved by passive or facilitated diffusion or by active transport processes. In case of microorganisms with an additional outer membrane (for example Gram-negative bacteria) aqueous transmembrane channels, so called porins, may be used for example by antibiotics to gain access to said microorganisms.

Excretion or efflux of said compounds or compositions into the external environment of a cell or microorganism may be carried out, among other possibilities, by so called transport proteins within the membrane of a target cell. These transport proteins may act as pumps of the mentioned kind, specific for certain compounds or compositions or may transport a range of structurally dissimilar compounds or compositions. These transport proteins may be associated with resistances in the context of commonly applied compounds and compositions such as for example tumour or cancer cells in respect to cytostatic and microorganisms in respect to antibiotics.

Some embodiments of the pharmaceutical and the methods are applicable for both resistant target cells and target cells, which are prone to acquire a resistance mechanism.

It is an important advantage of some embodiments that the pharmaceutical compositions and the methods of applying compositions are also applicable to known and currently available compositions of chemical agents and drugs that have become useless due to resistance acquired by target cells. With the administration of previously useful compositions such as antibiotics, antimicrobials, antifungal and anti-neoplastic compositions according to some embodiments, and applications of chemical agents, e.g. pest control agents according to further embodiments, otherwise useless compositions are effective in killing or reducing proliferation of target cells. Obviously, this is very cost-efficient compared to the development of new compositions and drugs. Furthermore such known compositions have already been tested, are well characterized, e.g. side effects, dosage regimens previously in the desired medical effect in non-resistant target cells, maximal non-toxic levels or environmental effects are already known, and they are admitted to the market.

The active component of the compositions for their photo-activation must absorb within the range of wavelengths of the electromagnetic radiation to which the individual or the object or the surface is exposed. In some embodiments, the photo-activation involves a two- or multi-photon absorption process. The applied electromagnetic radiation does not necessarily have to include an absorption peak of the active component. Pharmaceuticals or compositions with active components lacking aromatic groups and conjugated double bonds do not significantly absorb electromagnetic radiation in the range of UV and Visible light of wavelengths with a lower limit of 190 nm, and they are therefore not suitable. Some embodiments involving a two- or multi-photon absorption process photo-activation of the active compound may be achieved by electromagnetic radiation of wavelength up to e.g. 2000 nm.

UV /Vis spectroscopy is routinely used in the fields of biochemistry and analytical chemistry. The absorption of aromatic and conjugated organic compounds and biochemical macromolecules is easily measured and it is generally well predictable from the structural formula of a chemical compound. Chemical compounds with at least one aromatic system and or conjugated double bonds are highly desirable, due to their high chemical stability of the aromatic system. In particular, the capacity to have delocalized electrons can enable different types of chemical reactions e.g. both oxidation and reduction reactions with two radicals localized in distant parts of the molecule. Accordingly, it is easily determined, that e.g. quinolones and tetracyclines are very good examples of active components in pharmaceuticals for administration according to the dosage regimen of some embodiments of the invention, whereas most penicillins are not suitable due to their lack of aromatic rings and conjugated double bonds and the concomitant lack of significant absorption of electromagnetic radiation of a wavelength above around 190 nm and in particular lack of absorption in a range of wave length between 190 nm and 790 nm.

Some resistance target cells may currently not be susceptible to the pharmaceutical composition or the composition comprising the photo-activated active component, because they have evolved to become resistant by a resistance mechanism which is not overcome by the photo-activated active component. Such an example of non-susceptible mechanism is resistance due to a genetic modification rendering a receptor mediating the passive transport of the active component into the cell ineffective or another example is a change in the permeability of a bacterial cell wall, which block the uptake of the active component. However, other target cells which are resistant to the same active component but due to one or more mechanisms which are susceptible to be overcome by photo-activated active components of the pharmaceutical composition and the method of applying the composition. Such susceptible resistant target cells possess for example pumps transporting the active components out of the target cell and thereby lowering the intracellular concentration so low that the active component cannot kill or reduce proliferation of the resistant target cell. The latter resistant target cell is susceptible to the method and regimen of the same activated component, because the activated active component by covalently binding to the pumps prevents the pumps from transporting further molecules of active component out of the cell. After a while, the intracellular concentration of the active component is high enough stop the target cells from growing, resulting in their reduced proliferation and/or killing.

In the state of the art, UV light treatment in combination with antibiotic treatment has been described to have a phototoxic, and in particular a germicidal effect due to the generation of free radicals. Free radicals aggressively modify nucleic acids and thereby interfere with the replication of the germs, see e.g. D.Trisciuoglio et al. (2002): Phototoxic effect of fluoroquinolones on two human cell lines, Toxicology in vitro 16, 449-456 or Umezawa et al. (1997) Archives of Biochem. and Biophys, 342, 275 - 281, or M.H. Cruz de Carvalho (2008): Drought stress and reactive oxygen specie, Plant Signalling & Behaviour, 156-165.This oxidative effect is short-lived and lasts only as long as the target cell is exposed to both the antibiotic and the radiation. Free radical damage to nucleic acid by UV photo-activated antibiotics is the result of antibiotic molecules breaking up generating free radical compounds and requires a high level of energy applied by electromagnetic radiation, namely a level corresponding to a wavelength of below 400 nm for breaking up most chemical bonds. Furthermore, a high concentration of UV photo-activated antibiotic molecules is necessary, because they are so short lived, that many excited molecules are dissipated even before they have caused damage to nucleic acids due to strand breaks.

In contrast, the effect of overcoming resistance by a photo-activated active compound e.g. due to covalent binding of the active compound to a resistance conferring molecule such as a pump, lasts much longer and continues to have an effect even after the exposure to electromagnetic radiation. In some embodiments of the methods and the pharmaceutical, the exposure to electromagnetic radiation may, in addition to the effect of overcoming a resistance mechanism, add a free radical effect. In other embodiments a free radical effect may be excluded, e.g. by adjusting the wavelength of electromagnetic radiation and/or by lowering the concentration of the antibiotic and/or by lowering the total amount of energy delivered to the target cells.

For the pharmaceutical composition and for the method of applying the composition, the energy delivered during photo activation of the active component can be dosed e.g. by adjustment of the range of wavelength of the electromagnetic radiation, by adjustment of the radiation intensity, and/or adjustment of the time of the exposure to electromagnetic radiation e.g. by pulsing the electromagnetic radiation so as to limit or prevent tissue damage surrounding the target cells. For example the number of pulses per unit of time, the energy per pulse, the duration of each pulse, the switch-off time between the pulses, and total duration of treatment with pulse can be dosed with variability for suiting the particular application.

UV light at wavelengths below 240 nm is known to kill bacteria by itself even in the absence of an active component of a pharmaceutical or a chemical agent. This is because light at wavelenths below 240 nm has the capacity of damaging DNA. However, the effectiveness of UV treatment alone is limited, because bacteria are quite efficient at repairing DNA that is damaged by UV light. Due to surviving bacteria, UV sterilization alone is therefore often unsatisfactory. Also, radiation at wavelengths below 240 nm has a considerable toxicity for tissue that is to remain undamaged, and for most treatments, application of such radiation is not an option. It is an important advantage of many embodiments of the invention that radiation with a lower wavelength limit of 240 nm may be applied.

Furthermore, in the state of the art, UV light treatment of donated blood or blood components for the elimination or reduction of pathogens in blood products is known. This treatment is based on riboflavin, a natural component of blood. The blood products are exposed to UV light which must comprise radiation of a wavelength around 365 nm, an absorption peak of riboflavin, which leads to its excitation and ability to chemically modify nucleic acids. Modification of nucleic acids interferes with the replication of viruses and living cells and therefore pathogens in blood products are inactivated by exposure to UV light in the presence of riboflavin at a wavelength in the range of 365 nm. Therefore, some embodiments including a range of wavelength around 365 nm may benefit from a dual mechanism of killing or reducing proliferation of target cells, namely the mechanism based on riboflavin and the mechanism based on overcoming resistance by a photo-activated active component.

Independent of the riboflavin effect and unless by chance the active component absorbs and is activated only at the same wavelength as riboflavin, compositions and methods including the photo-activation of an active component readily work with electromagnetic radiation at wavelengths differing from 365 nm and differing from a range around 365 nm. Thus, the effect of killing or reducing the proliferation of target cells by the pharmaceutical composition and the method according to the invention function independently of this known mechanism based on activated riboflavin.

It is conceived that the pharmaceuticals and methods are rendered effective in reducing or destroying the resistance of living cells and organisms to drugs and chemical agents due to the exposure of the target cells to a combination of the active component and electromagnetic radiation at the same time and / or exposure to electromagnetic radiation after exposure to the active component by overcoming mechanisms of resistance including such mechanisms as are discussed above.

The conceived exemlary mechanism inhibits excretion of pharmaceutical compounds or compositions such as anti-infectants or cytostatica from a target cell as described above. The conceived mechanism thereby overcomes the undesired excretion of pharmaceutical compounds or compositions from target cells such as a bacterial, fungal, parasitical or infected cell or even tumour or cancer cells.

In more detail, e.g. in one such conceived exemplary mechanism, a microbial strain is resistant to a particular antimicrobial drug owing to pumps with which the resistant microbes efficiently and continuously pump the drug molecules out of the microbes cellular interior to the exterior of the cell. Thereby, the resistant microbes escape the killing or harming effect caused by a drug, which would otherwise be effective in non resistant strains lacking such a pump activity. During the application of the pharmaceutical composition or the method, the active component of the composition is activated to an energetically higher level by absorbing electromagnetic radiation. This activation renders the active component capable of covalently attaching to and thereby obstructing the pump, which is responsible for transporting the active component out of the resistant microbe. By this irreversible binding of the drug or the active component to the pump, only one single such molecule of the active component is inactivated by the pump, while all other molecules, which are not bound to a pump in the outer membrane of the resistant microbe, are still available to exert the intended effect of the drug inside of the target microbe and cause its death or at diminish its proliferation. Accordingly, the exposure of the target cell to the active component in the presence of electromagnetic radiation overcomes the resistance of the target cell and renders the therapeutic regimen of the pharmaceutical or method of applying the composition effective.

According to a further conceived mechanism of overcoming drug resistance, the activated active component binds covalently to an enzyme which enzyme generates the resistance by modification or degradation of the active component, thereby causing inactivation of the drug in the resistant target cells. Again, as soon as all enzyme molecules are bound to one drug molecule each for a prolonged period of time, the enzyme can no longer save the cell from the detrimental effect of all the other drug molecules.

Most of the resistant target cells use more than one resistance mechanisms, e.g. a pump pumping the active component out of the target cell thereby lowering its intracellular concentration and additionally e.g. a modification by genetic mutation of the targeted molecule for decrease its affinity by the active component. The methods and pharmaceuticals comprising the photo-activated active compound first block the pump by covalent binding of the photo-activated active component, thereby increasing the intracellular concentration of the active component. Thus, even if the mutated targeted molecule has a low affinity to the active component, due to its photo-activation once the active component has made contact with the targeted molecule they are covalently linked. Therefore, in some applications the effect of photo activation is observed only with some delay in the time during which a first resistance mechanism is overcome.

There is a further advantageous effect: The resistant target cells cannot easily escape the combined action of an active component with concomitant exposure to electromagnetic radiation. This is because both, the resistance mechanism based on pumps and intracellular enzymes, exploit the specific binding affinity between active component and the pump or enzyme which mediates the resistance to the active component. Any escape strategy, as known from evolutionary principles, would decrease the affinity of the pump or of the enzyme for the active component - thereby escaping the prolonged binding caused by activation of the activated component by electromagnetic radiation -, and such decreased activity would at the same time also decrease the activity of the pump or the enzyme for pumping out or degrading the drug, and thereby it would inevitably also lower or eliminate the original drug resistance.

Although the scope of the present invention is not limited to or bound by certain explanations of the effects observed , the conceived mechanisms predict that the herein described pharmaceuticals and methods are less likely to overcome resistance to drugs or active components which operate from the outside of cells. For example, there are antibiotics which attack the bacterial cell from the outside or which target the bacterial cell wall (e.g. penicillins and cephalosporins) or the cell membrane (polymixins). However, the conceived mechanisms predict that the herein described pharmaceuticals and methods are less likely to overcome resistance due to a structural modification of the bacteria leading to a decrease of the permeability of the antibiotics
Some embodiments of the pharmaceutical composition and the method of applying the composition comprise a photo-activation of the active component wherein the electromagnetic radiation has a range of wavelength with a lower limit of 193 nm, or of 200 nm, or of 240 nm, or of 280 nm, or of 350 nm or of 365 nm.

In some exemplary embodiments of methods and pharmaceuticals of the invention, the electromagnetic radiation includes a range of wavelengths of up to about 240 nm in which a combination of the germicidal effect by UV light itself, the effect of free radical damage caused by a general mechanism of a combination of antibiotic and UV light and an additional killing effect due to specific effect of binding of activated active compounds to molecules such as pumps or enzymes otherwise conferring resistance to the active component all of which may contribute to the killing and reduced proliferation of the target cells. In some embodiments of the method or pharmaceuticals, the surface or object or individuals are exposed to electromagnetic radiation including a range of wavelength with a lower limit of around 180 nm to 200 nm or in particular of 190 nm for photo-activation of the active component and these embodiments include also the above described effects of UV radiation. Some embodiments of the invention particularly include photo-activation with ranges of wavelength with a lower limit of 240 nm or particularly include a range of wave length around 240 nm, where all aromatic organic compounds absorb, however where cell damage to UV exposure is lacking or much reduced.

In some embodiments, the radiation exposure is chosen to minimize damage to adjacent tissue surrounding the target cells by applying a range of wavelength with a lower limit of above 240 nm, e.g. 260 nm, 280 nm, 300 nm, 320 nm, 330, nm 340 nm, 350 nm or 360 nm. Particularly, in the visible spectrum and in the UVA above 350 nm atomic or electric valence electrons are not or only barely excited. However, even for the embodiments with an exposure to radiation in a range of wavelength with a lower limit around 350 nm, eyes should still be shielded from exposure to radiation to prevent damage of the retina due to molecular electron excitation of pigment molecules.

In some exemplary embodiments, a level of intensity of the applied radiation is increased to compensate for an application of less energetic radiation in a range of longer wavelength. In all embodiments of the invention the selected range of wavelength to which the individual, the object or the surface, in particular to which the target cells, are exposed must include a range of the electromagnetic wavelengths within which the active component attacking the target cells is absorbing. It is not required that the peak of absorbance by the active component is included in this range of wavelengths. Rather, the requirement is that the molecular absorption spectrum of the active component overlaps the emission spectrum of the radiation source used.In some exemplary embodiments of the pharmaceuticals and methods, the lower limit of the range of wavelength to which the target cells are exposed is as low as 190 nm, which corresponds to the lower limit of radiation emitted by excimer lasers, also called exciplex lasers. In some of these and other embodiments, the lower limit is kept above 200 nm, as UV light below 200 nm is particularly aggressive and prone to cause tissue damage. In some embodiments, the lower limit is raised to another value above 200 nm, e.g. 220 nm or 240 nm or 260 nm in order to adjust the wavelength and energy transmitted to the physiological sensitivity of the exposed host tissue of the target cells.

In some exemplary embodiments the lower limit of the range of wavelength is kept at 280 nm, which is known to be the upper limit of significant UV light absorption by nucleic acids. Below 280 nm electromagnetic radiation is known to be carcinogenic. By keeping the lower limit of wavelength around 280 nm or 290 nm, potential damage to nucleic acids and in particular to the DNA of host cells is avoided. Therefore, in some embodiments the lower limit of wavelength is kept at 280 nm or above 290 nm or 300 nm to avoid damage to DNA potentially causing mutations and cancer.

In some embodiments of the pharmaceutical composition and the method of applying the composition, comprise a photo-activation wherein the electromagnetic radiation has a range of a wavelength with an upper limit of 800 nm, of 700nm, of 600 nm of 500 nm or of 450 nm.

An increase of the range of the wavelengths selected for the exposure to electromagnetic radiation, and in particular an increase of the lower limit of the range, decreases the amount of energy transmitted to the target cells and to the individual, object or surface area. In particular, the effects of electromagnetic radiation on the active compound, the target cells and the surrounding area of a living individual or tissue or object can be influenced by adjustment of the lower limit of wavelength, because certain cellular or chemical processes are dependent on the wavelength of the electromagnetic exposure and/or the total energy transmitted as will be discussed in more detail below.

Some embodiments of the pharmaceutical composition and the method of applying the composition comprise a photo-activation of the active component, wherein the electromagnetic radiation has a wavelength between 200 nm and 500 nm or in particular in a range with a lower limit between 200 nm and 240 nm or between 200 nm and 280 nm, at 280 nm or at a value between 280 nm and 400 nm especially at 300 nm or higher to avoid substantial absorption by DNA. There is not necessarily an upper limit for the radiation, but often longer wavelength radiation tends to become inefficient leading to the need for very high intensities. For practical purposes, therefore, an upper limit may be selected to be at the IR threshold (at 700 nm) or below, for example at between 450 nm and 500 nm. In a particular example, a lower limit of approx. 350 nm or 365 nm or 370 nm, and an upper limit of approx. 450 nm may be chosen.

In some exemplary embodiments the upper limit of the range of wavelength is selected around 790 or 700 nm or 600 nm so as not to include wavelengths, which may induce molecular oscillations. In some embodiments the upper limit of the range of wavelength is around 500 nm or in particular around 450 nm. For some embodiments of the pharmaceutical composition, in particular, the individual is exposed to electromagnetic radiation in a range of wavelength with a lower limit between 200 nm and 240 nm or 280 nm, especially at any one of these values, or at 300 nm or at 320 nm and an upper limit between 450 nm and 500 nm or more particularly with a lower limit of approx. 350 nm and an upper limit of approx . 450 nm or in particular in a range around 400 nm (thus in the region of the UV/blue transition of the spectrum).

Some embodiments of the pharmaceutical composition and the method of applying the composition comprise a photo-activation comprising a multi-photon process. In these embodiments the active component is activated in a manner involving more than one photon. Some of these embodiments with absorption of more than one photon by the active compound may include a transient intermediate level of the activated compound. In further embodiments with absorption of more than one photon by the active component involve a concurrent or simultaneous absorption of more than one photon by the activated component (nonlinear process; two- or multiple photon absorption).

In some of these embodiments with photo-activation involving a multi-photon process, the range of wavelength of the electromagnetic radiation has an upper limit of up to 2000 nm and in particular the range has a lower limit of 800 nm and an upper limit of 2000 nm, more particularly in a range of 800 to 1700 nm. In some of these embodiments with photo-activation comprising a multi-photon process the exposure to electromagnetic radiation is pulsed, especially very short radiation pulses of less than 1 ps pulse length, e.g. with a pulse length in a range with a lower limit of 0.1 fs or 1 fs and an upper limit of 1 ps.

More in general, for all embodiments, including embodiments where multi-photon processes do not play a significant role, pulsed radiation sources are used for photo activation. In the embodiments of the pharmaceutical composition and the method of applying the composition the electromagnetic radiation is applied with pulses of a length in a range of 0.1 fs to 200 ms, more particularly in a range of 1 fs up to 100 fs or of 1 fs up to 1 ms. An advantageous effect of a pulsed administration of electromagnetic radiation is to limit the amount of heat applied to treated individuals and objects with nevertheless high peak intensities, and in particular to be able to control the temperature increase of tissue surrounding the target cells e.g. so as not to exceed 40°C in mammals. In some exemplary embodiments direct feedback infrared cameras are used to control the warming up of surrounding tissue; such cameras in embodiments may be controlled to operate intermittently, alternating with the pulses so as to make sure that no reflected radiation distorts the temperature measurement.

More in particular, it has been found that at least in some set-ups the effect of the pulsed radiation on overcoming resistances is higher than that of continuous radiation of the same average intensity - even if the pulse peak energy density at the target does not lead to any multi-photon processes with significant probability. While this phenomenon is not yet fully explained, it is currently assumed that it has to do with the process speed and time constants of the biochemical processes within the target cell, especially the of the biochemical processes connected to the pumping effect discussed hereinbefore. For example, one may assume that the effect depends on what state the system comprising the transport proteins and the active component molecule is in when a first photon is incident, and it may also depend on the state the system is in when a second photon is incident. For example, it may be important that the transport protein and the molecule are in a state of strong interaction when the (first) photon is incident, and it cannot be ruled out that a second photon incident on the system that is in an excited state is required to ultimately block the pump. The probability that a second photon meets the system in an excited state is higher for higher intensities, which means that for a given average intensity (that is limited by the condition that the tissue is not to be heated above for example 42°C or 40°C) this probability is higher for pulsed radiation.

This kind of effect of the pulsed radiation (that does not rely on multi-photon processes, i.e. processes in which several photons act simultaneously) has been found to be at least present for pulses with repetition frequencies between 0.1 kHz and 100 kHz and pulse lengths in the microsecond region, i.e. between 100 ns and 500 microseconds. More in general, the ratio between the on-time and the off-time of the pulsed radiation should be at least 10, and often more preferred at least 50 or at least 100 or even at least 200, with no upper limit or an upper limit of 10⁶.

The amount of total energy transferred to the target cells and to the individual, object or surface area can further be dosed by the time of the exposure to electromagnetic radiation e.g. by pulsing the electromagnetic radiation e.g. to limit or prevent tissue damage surrounding the target cells. For example, the pulse energy, number of pulses per unit of time, the duration of each pulse, the switch-off time between the pulses, and total duration of treatment with pulse can be dosed with variability for suiting the particular application.

In some embodiments of the pharmaceutical composition and the method of applying the composition with a duration of time of exposure to the electromagnetic radiation either continuously or pulsed with a lower limit of between 1 s and 30 s or 1 s and 3 min and an upper limit between 1 min and 30 min, in particular an upper limit between 1 min and 10 min or an upper limit between 1 min and 3 min (or, as always in this text, at any one of these limit values).

In some embodiments a long term treatment is applied, lasting for more than 1, or 2 or 6 or 12 or 24 hours. In some embodiments of the long term treatment, the duration of the exposure time to the electromagnetic radiation applied either continuously or pulsed may be 1 or more hours, 1 or more days, 1 or more weeks or even months Some embodiments of a long term treatment comprise a regimen or a method with a radiation exposure e.g. in a range with a lower limit of 0.0001 or 0.001 W/cm² and with an upper limit of 1 W/cm² or 10 W/cm², wherein in case of a pulsed application or regimen of exposure to electromagnetic radiation source only the ON but not the OFF time during the exposure is counted. The amount of energy applied has to be adjusted so as not to damage living tissue or organisms outside of the target cells. Exemplary applications of long term treatments include e.g. administration of an active component which is photo-activated with catheter, intravenous device such as tubing e.g. until the ablation of the catheter or IV device to block the colonisation of the catheter with resistant bacteria or for example for chronic bone infection with a light implantable delivery system witch increases the effect of the active component on bacteria having a low metabolic activity. Long term treatments not only accompany implantable medical devices but include also e.g. long term treatments of plants, farm animals or industrial applications of the pharmaceuticals and the method, all by an active, controllable illumination light source.

In some exemplary embodiments of the method and the pharmaceutical composition the electromagnetic radiation is applied with careful monitoring of the amount of energy transmitted to the surrounding tissue by adjusting the duration of the radiation exposure and intensity of the radiation emitted from a radiation source such as a lamp or a laser. In some exemplary embodiments of the method and the pharmaceutical composition the total amount of energy absorbed per minute and per volume of material or tissue of a human or non-human individual or of a tissue outside of a living individual or of material of an object during the exposure to the electromagnetic radiation source does not exceed approx. 30 J per cm³ or 100 J per cm³ or 300 J per cm³ per minute for a tissue of a living individual and/or it does not exceed approx. 150 J per cm³ or 300 J per cm³ or 600 J per cm³ per minute per volume of a tissue outside of a living organism or per volume of a material of an object. The energy absorbed per volume tissue or material depends e.g on the heat capacity of the material, on the water content of the tissue, on the capacity of the surface to dissipate heat, on the presence and effectiveness of a cooling system and in some embodiments it is selected, so as not to cause a temperature increase by more than 3°C or 4°C or 5°C. The temperature increase which is measurable by a temperature feed back system, like an infrared camera according to methods known in the art. In some of these and other embodiments the radiation intensity resulting from the power of the laser during one second of actual exposure thus only counting the ON but not the OFF time during the exposure in case of a pulsed application or regimen - is selected to be in a range with a lower limit of 0.1 mW/cm² to 0.1 W/cm² and an upper limit of e.g. up to 10 W/cm² or up to 50 W/cm² or up to 500 W/cm² or particularly in a range of approx. 5 W/cm² to 25 W/cm² or 10 W/cm² to 20 W/cm². For some embodiments of the pharmaceuticals and methods with the application of radiation in of a range of wavelength with a lower limit of below 300 nm or below 350 nm, the intensity is selected in rather a lower range to compensate for the higher energy of the radiation known to have a potentially damaging effect on the tissue surrounding the target cells. In other embodiments, in particular when the radiation comprises a range of wavelength with a lower limit of above 300 nm or above 350 nm or 400 nm, the radiation intensity is selected to be in a range of 5 to 500 W/cm² or 10 to 350 W/cm² or 10 to 250 W/cm².

Thus, the range of wavelength, the intensity and the duration of the radiation exposure for particular embodiments of the invention are selected based on the amount of energy that shall be transmitted to the tissue or surface of the individual or object around the target cells and on the absorption spectrum of the active component which is administered or applied.

In some exemplary embodiments, the radiation intensity and duration of irradiation is monitored and adjusted so that the temperature of the surrounding tissue or surface or object does not rise by more than e.g. 1° or 2° or 5° C.

In some embodiments, the active component of the pharmaceuticals and methods is exposed to radiation of a range of wavelengths some of which it absorbs for a duration and a level of intensity at which the active component remains stable or degrades to less then 10%, 30% or 50% of the applied or administered active component. To test this, for some active components when exposed in vitro under comparable conditions to the same range of wavelength at the same level of intensity and with the same duration as in the regimen of the pharmaceutical or in the method, the percentage of absorbed radiation does not decrease by more than 10%, 30% or 50%.

Another significant advantage of photo-activation of active components of pharmaceuticals and other compositions is that they are effective at overcoming the resistance of resistant target cells even at low concentrations of the active component, which is not elevated above the concentration of the active component as known in the state of the art, in particular not a higher concentration than in a comparative pharmaceutical treatment or in a comparative method of application of a composition without concomitant exposure to electromagnetic radiation according to standards described for the same active component in the pertinent literature or according to standard drug prescription

In some embodiments, the effective concentration of the active component which reduces the growth rate of target cells is even lower by a factor of up to two, or up to five or up to ten or up to one hundred or up to one thousand when compared to the concentration known in the state of the art to be necessary for achieving the desired medical effect. Such low effective concentrations reduce side effects particularly in systemic treatment of individuals. For example, in vitro analysis of several antibiotics added to cell cultures at low concentrations, e.g. at 4 mg/L with subsequent exposure to electromagnetic radiation revealed a bacterial growth rate according to a standard test which was reduced by a factor of 100. At the same time other cells surrounding the bacteria such as fibroblasts, epithelial cells of the lungs or endothelial cells, (e.g.HUVEC (Human Umbilical Vein Endothelial Cells) were not reduced by more than 1%.

In some embodiments, the active component of the composition comprises an organic compound with an aromatic or at least one conjugated double bond. All embodiments of the invention comprise one or more active components absorbing electromagnetic radiation in a range of wavelengths with a lower limit of at least 190 nm. The absorption is based in particular on a chemical structure of the active component, which comprises a conjugated system of delocalized electrons. The chemical structure of the active compound may be cyclic, in particular aromatic, acyclic, linear or mixed. In some embodiments the active component comprises an aromatic group or at least one pair of conjugated double bonds in a linear substructure or both. Since compounds with conjugated systems of delocalized electrons generally absorb in the UV to visible spectrum of electromagnetic radiation, structural analysis of chemical compounds is useful for the identification of active components suitable for the pharmaceuticals and methods of the invention.

In some exemplary embodiments, particularly for killing or reducing the proliferation of bacteria and other pathogens or pest, the active component comprises or consists of a quinolone, in particular according to Formula I or II 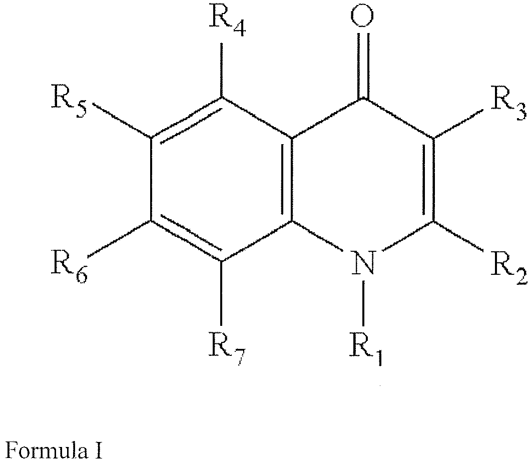 or

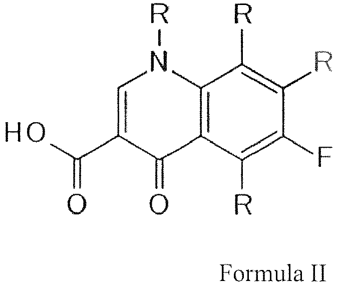

In some further exemplary embodiments the active component comprises or consists of a derivative of the group of tetracyclines, which are polycyclic naphthacene carboxamides in particular, according to Formula III or IV, 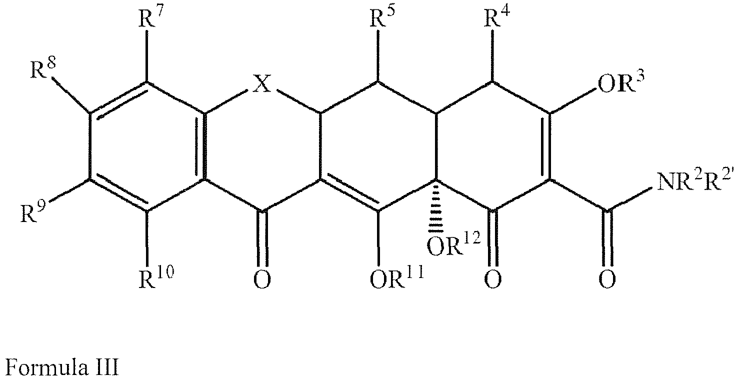 e.g.

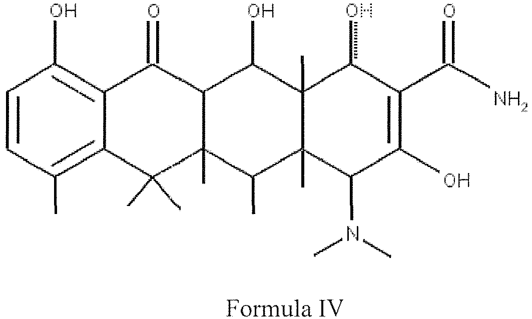

In some further exemplary embodiments the active component comprises a system of conjugated double bonds, e.g. a non-saturated carbon tail. Such a system of conjugated double bonds comprises 2 to e.g. 12 or more conjugated double bonds and in particular it comprises at least 3, 4, 5, 6 or 7 conjugated double bonds.

In some exemplary embodiments one or more active component are selected from one of the following lists of known pharmaceuticals:
- First generation quinolones such as cinoxacin, nalidixic acid, oxolinic acid, piromidic acid, pipemidic acid, rosoxacin.
- Second generation quinolones such as ciprofloxacin, enoxacin, fleroxacin, lomefloxacin, nadifloxacin, norfloxacin, ofloxacin, pefloxacin, rufloxacin.
- Third generation quinolones such as balofloxacin, grepafloxacin, levofloxacin, pazufloxacin, sparfloxacin, temafloxacin, tosufloxacin.
- Fourth generation quinolones such as clinafloxacin, gatifloxacin, gemifloxacin, moxifloxacin, sitafloxacin, trovafloxacin, prulifloxacin.
- Quinolones which are still in a developmental stage such as delafloxacin, JNJ-Q2.
- Quinolones, which currently are used in particular for veterinary applications such as danofloxacin, difloxacin, enrofloxacin, ibafloxacin, marbofloxacin, orbifloxacin, sarafloxacin.
- Cyclines such as tetracycline, demeclocycline, doxycycline, minocycline, oxytetracycline, tetracycline, methacycline, lymecycline, tigecycline.
- Drugs against mycobacteria such as clofazimine, ethionamide, rifampicin (rifampin), rifabutin, rifapentin.
- Other drugs such as amphotericin B

In some exemplary embodiments, the composition comprises one or more quinolone or tetracycline selected from following list A: Ofloxacin, Moxyfloxacin, Ciprofloxacin, Levofloxacin, Tetracycline, Doxycycline. In further exemplary embodiments, the composition comprises one or more quinolone or tetracycline selected from following list B: Gatifloxacin, Norfloxacie, Minocycline, Oxytetracycline. In further embodiments the composition comprises at least two active component selected of the above lists A and/ or B.

In some exemplary embodiments, particularly for killing and reducing proliferation of neoplastic cells, the active component comprises or consists of a planar pentacyclic ring structure in particular according to Formula V

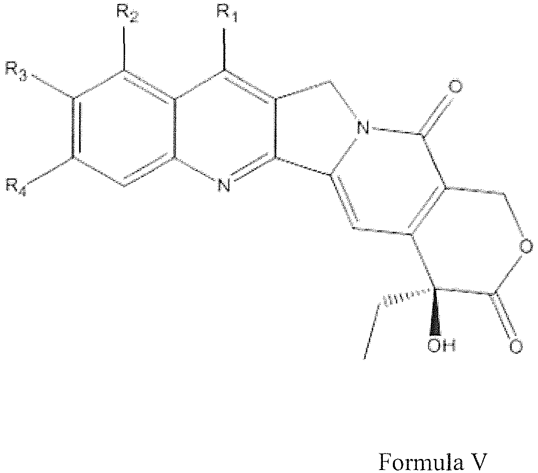

In some further exemplary embodiments, particularly for killing and reducing proliferation of neoplastic cells, the active component comprises or consists of a derivative of the group of anthracyclines, in particular according to Formula VI:

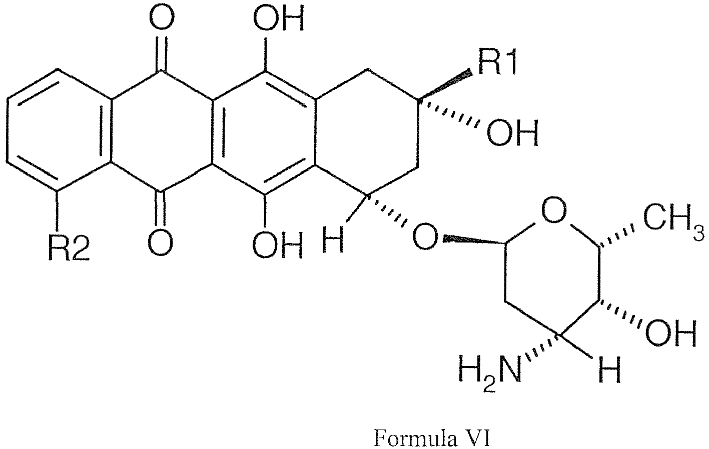

In some further exemplary embodiments, particularly for killing and reducing proliferation of neoplastic cells, the active component comprises or consists of a derivative of acridine according to Formula VII

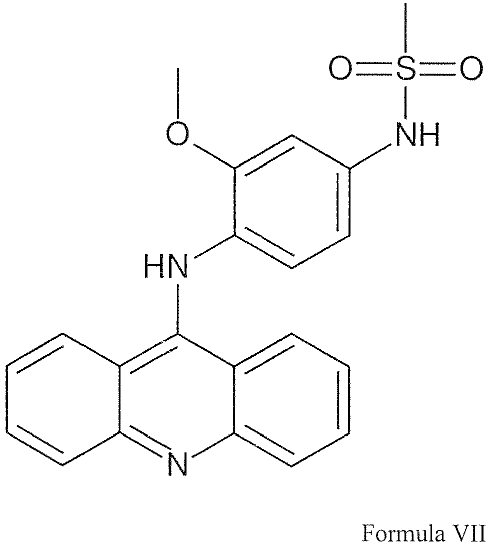

In some exemplary embodiments of the method and the pharmaceutical one or more active components are selected from the following lists of known pharmaceuticals:
- an antracycline such as daunorubicin, doxorubicin, epirubicin, idarubicin, valrubicin
- an camptothecin derivative such as topotecan, irinotecan
- Amsacrine

In some embodiments of the method and the pharmaceutical the active component comprises a system of conjugated double bonds, e.g. a non-saturated carbon tail. Such a system of conjugated double bonds comprises 2 to e.g. 12 or more conjugated double bonds and in particular it comprises at least 3, 4, 5, 6 or 7 conjugated double bonds.

In some embodiments of the pharmaceutical composition and the method of applying the composition, it is applied to an outer or an inner surface of a living organism, e.g to the skin or lungs or intestinal tract or ear or bone or bladder. In some embodiments the composition is administered to an individual systemically, e.g. by oral administration, a drug-delivery system or intravenous administration, and combined with a topical exposure to electromagnetic radiation. In some embodiments both the application of the composition and the exposure to the electromagnetic radiation are topical. In some embodiments the exposure to the electromagnetic radiation is administered during an invasive, in particular a minimally invasive procedure e.g. optic fiber adminstration.

In some exemplary embodiments of the pharmaceuticals and methods, the composition is administered to individuals for preventive or therapeutic topical application, in particular during of after surgical procedures, and/or for example including the treatment of eyes, e.g. corneal infections, or infections of ears, nose, skin, mouth, throat, and other inner epithelial tissue surfaces, as well as e.g. topical treatment of dermatological and veneral disease and applications in dental medicine and as yet further example for a topical application per-operative treatment of the skin prior to surgical incision as well as during surgical procedures for treatment at the site of the surgical procedure, and infections or tumors in bones and joints. Obviously, in topical applications of the composition or the method, particularly to outer surfaces of an individual, the exposure of the target cells to electromagnetic radiation is particularly simple.

The pharmaceuticals and methods are suited for further applications in a hospital setting such as for preventing the transfer of nosocomial pathogens e.g. pre- and post operatively and during surgery and also for the prevention or treatment of wound infections as well as for disinfection purposes including e.g. disinfection of surgical tools and operating rooms.

In some exemplary embodiments of the pharmaceuticals or the methods where the target cells are not as readily accessible to an external source of radiation, exposure to electromagnetic radiation is achieved by a minimally invasive instrument transmitting the electromagnetic radiation to proximity of the target cells. For example, in some embodiments of the pharmaceutical composition for the treatment of a localized tumor or source of infection tissue comprising the target cells are irradiated by a minimally invasive laser light. For example infections in bones or joints may be treated by endoscopic delivery of an active component and electromagnetic radiation. Further, applications of the pharmaceuticals and methods particularly include but are not limited to applications in the respiratory tract, in the reproductive system, urinary tract and in the gastrointestinal tract.

According to a further aspect of the description, a medical device is disclosed, the device comprising a source of electromagnetic radiation of a spectal composition that includes spectral components of a wavelength above 190 nm and that are matched to the pharmaceutical composition in that they are within the absorption spectrum of the active component. The device is configured to irradiate tissue portions of tissue that has been treated by the pharmaceutical composition (by superficial application or ingestion, including injection). To this end, the device may be adapted to specific situations.
- For example for the treatment of an open wound, the device may be adapted to evenly illuminate a comparably large area from a distance of typically 10-100 mm.
- In another example, for the treatment of tissue in an orifice or of unexposed tissue, the device may comprise an applicator introducible in the orifice/body, which applicator (applicator head/catheter, etc.) may be sterilizeable or single-use sterilized or have a sterilizeable or single-use sterile outer cover.
- In an even further example, for ophthalmic treatments, the device may comprise an applicator that is compatible with standard ophthalmic equipment and/or designed to be suitable to specifically illuminate the human
- cornea while exposing other tissue to the radiation only minimally.
   The device may be adapted to be held by an appropriate mounting system (such as standard equipment). For example, the device - or an applicator thereof - may be shaped to cooperate with a mount of a standard Goldmann applanation tonometer.
   In ophthalmic applications, the device may comprise a camera placed to monitor the cornea during treatment. Such a camera - or an other sensor device - may s be sensitive to radiation at frequencies at which Riboflavin emits fluorescent radiation when illuminated by UVA radiation. The radiation source may be chosen to emit radiation (also) in the UVA range, around 365 nm, where Riboflavin absorbs. A dose of Riboflavin may be applied to the Cornea. By this, firstly it is ensured that the radiation is sufficiently absorbed and does not adversely affect other parts of the eye, such as the retina. Secondly, in combination with the camera or other device, it allows to control the even irradiation.
   A camera for ophthalmic applications may be placed in an arrangement where it monitors the eye from a non-perpendicular angle. In this, it may optionally be configured to satisfy the Scheimpflug condition with respect to the surface of the eye. In accordance with an alternative, it may be arranged to monitor the eye from a perpendicular angle.
   In addition or as an alternative, also the illumination equipment, for example comprising a plurality of radiation sources, may be arranged to satisfy the Scheimpflug condition.

While the radiation source itself may be a standard radiation source - especially an LED (the definition of LED used herein includes OLED sources) or a superluminescent light emitting diode (SLED) emitting at wavelengths in the UVA and/or visible (especially blue) range of the spectrum, and more in generally satisfies the conditions discussed hereinbefore and hereinafter that hold for the electromagnetic radiation used for the regimen/method - the device is specifically adapted for the purpose described herein. To this end, it also comprises information on the interplay between the radiation and the active component, and more particularly explicitly refers to the pharmaceutical composition/active component that absorbs the radiation.

Generally, the information may be present in any form, such as written on a leaflet or in a user manual, or electronically.

The radiation output by the device may be tunable, and the device allows for selecting a radiation output depending on a chosen substance. For example, the device may comprise user interface allowing the user to select between different substances or groups of substances as the active component an adjusts the radiation output to the selection.

The radiation source may be a source of pulsed radiation. In this, the radiation pulses may have a pulse repetition frequency between 0.1 kHz and 100 kHz, particularly between 05 kHz and 40 kHz.

More in general, the radiation source may be configured to output radiation having pulses and/or other properties of the kind discussed in this text referring to the method and the regimen.

The radiation source is a source of incoherent radiation, thus not a laser. This is because for some applications, especially if even irradiation is desired, interferences may be undesired. Also, in contrast to coherent laser radiation, incoherent radiation is less dangerous for the operating person.

The device will also comprise a control that controls the energizing of the radiation source.

The device may further comprise a temperature sensor, such as an infrared sensor, for sensing the temperature of irradiated tissue or tissue immediately adjacent the irradiated tissue. The control may be configured to control the radiation source in a manner that the temperature - that may rise due to heating by the radiation - does not rise above a certain limit of for example 42°C, 41°C, or 40°C.

The device may further comprise a cooler, such as an active cooler. Such a cooler may be mounted to cool the radiation source. It may comprise a flowing cooling agent (such as cooling water), or it may comprise a Peltier cooler or other suitable cooling means. The cooler may cool the radiation source, firstly because semiconductor-based radiation sources must not be operated above a certain maximum operation temperature, as is known in the art. Secondly, cooling may prevent substantial Infrared radiation from the radiation source and its mount onto the tissue, which Infrared radiation would cause additional heat impact on the tissue, especially if the radiation source is close to the tissue. Thus, the cooler may be configured to cool the radiation source to substantially below the normal operation temperature of the radiation source.

The medical device comprising an optic fiber as part of the radiation source arrangement, is equipped for the combined delivery of an active component and electromagnetic radiation, in particular according to the above described pharmaceutical composition and the method. Examples of such a device include a drug delivery device and in particular a catheter comprising the optical fiber or directly the source emitting electromagnetic radiation. Medical devices according to this aspect of the invention have the advantage that their colonization by resistant target cells can be prevented and therefore they can be left within an individuals body for a longer time period than corresponding medical devices lacking an optic fiber. The advantage of a catheter comprising an optic fiber would be that the catheter would not have to changed during the treatment of an individual or at least not have to be changed as frequently compared to currently available catheters not comprising an optic fiber and not accompanied by the administration of a photo-activated active component.

The device has a catheter with a pharmaceutical composition dispensing unit arranged at its distal end, so that the device serves both, as applicator for the targeted dispensing of the composition and for the irradiation that overcomes possible resistances. Embodiments of this sub-group may for example be interesting for oncological applications where the pharmaceutical composition comprises a cytostatic, and it is desired to not flood the entire body of the patient with the composition. Especially interesting applications for this kind of devices are the treatment of uterine cancer or of cancer of the intestinal tract or of other regions accessible through body orifices.

The description also discloses a non-invasive method of using a radiation device after a treatment by a pharmaceutical composition, the method comprising irradiating a treated tissue by the radiation with a spectral composition and in a dose that does not substantially affect untreated living cells and that does not heat the tissue to a temperature above 42°C.

The description also contains a use of a medical device that has at least one source of electromagnetic radiation of a spectral composition including radiation portions of a wavelength above a lower limit of 190 nm, for irradiating a tissue portion that has been treated with a pharmaceutical composition comprising an active component which kills or retards proliferation of target cells within the tissue portion, and which tissue portion absorbs electromagnetic radiation of the radiation portion.

Especially, in this the irradiation is done with a spectral composition and in a dose that does not substantially affect untreated living cells and that does not heat the tissue to a temperature above 42°C.

More in particular, the non-invasive method and the use may be carried out in a manner as described referring to the regimen and method described in this text.

Some exemplary embodiments of the method and pharmaceuticals are further explained below and some experiments are presented to further describe some embodiments of the invention also including the following figures.

The figures show:
**Fig.** 1 Absorption Spectra of exemplary active components for pharmaceuticals and methods of the invention;
Fig. 2 Results of a toxicity test showing human skin after an exemplary exposure to electromagnetic radiation in the UV / Vis range;
**Fig. 3****,** **Fig. 4** **and** **Fig.** 5 Results of Experiments 7, 8 and 9, respectively testing the effect of some exemplary photo-activated antibiotics on two different multi resistant bacterial strains;
**Figs. 6-8** Examples of medical devices.

### Examples of absorption spectra of exemplary active components:

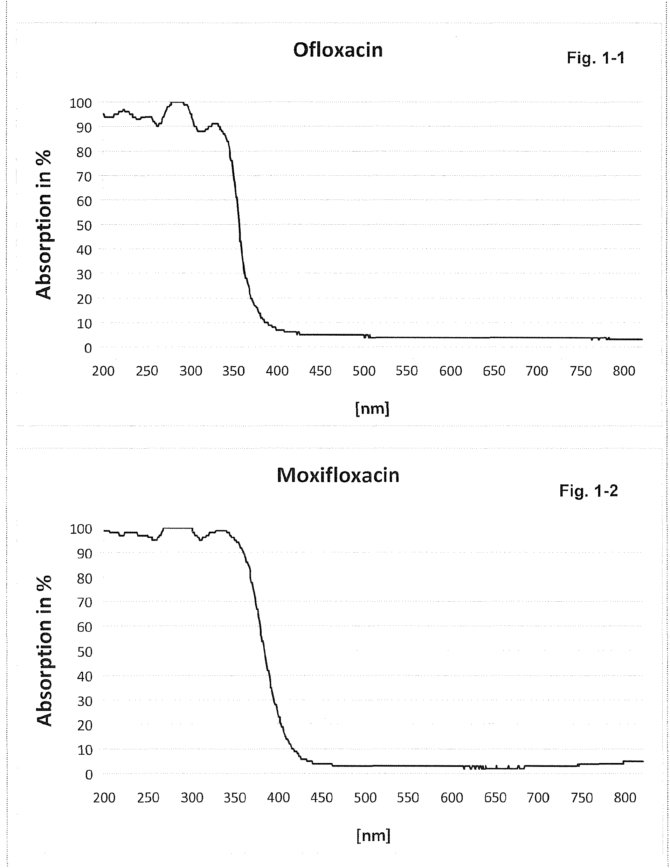

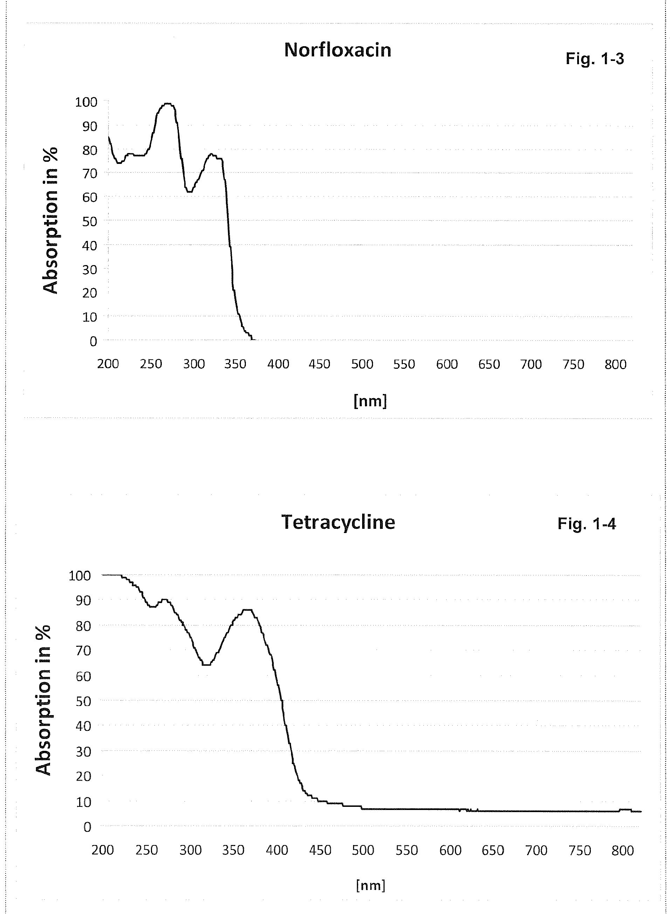

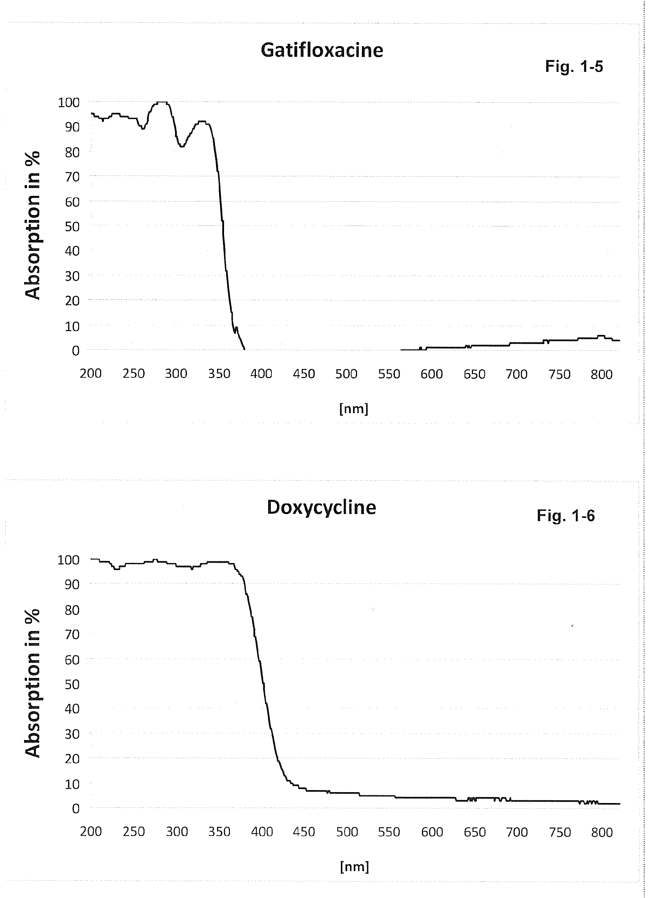

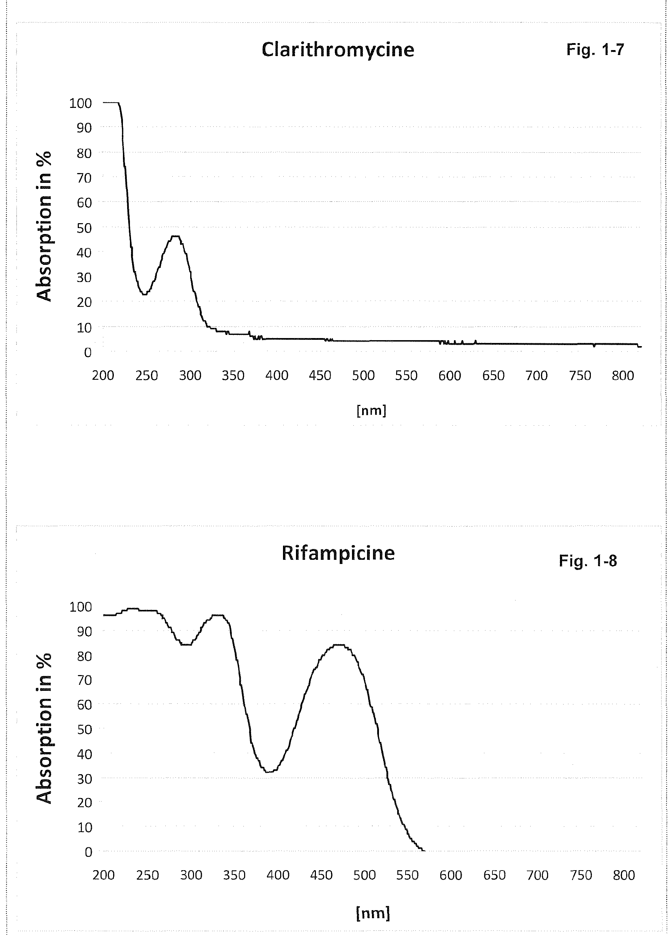

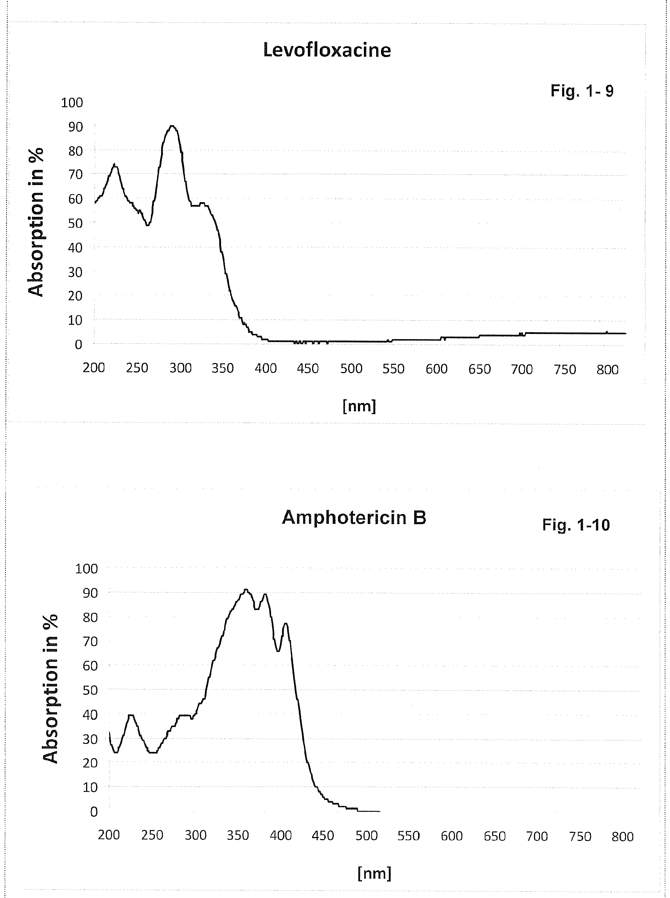

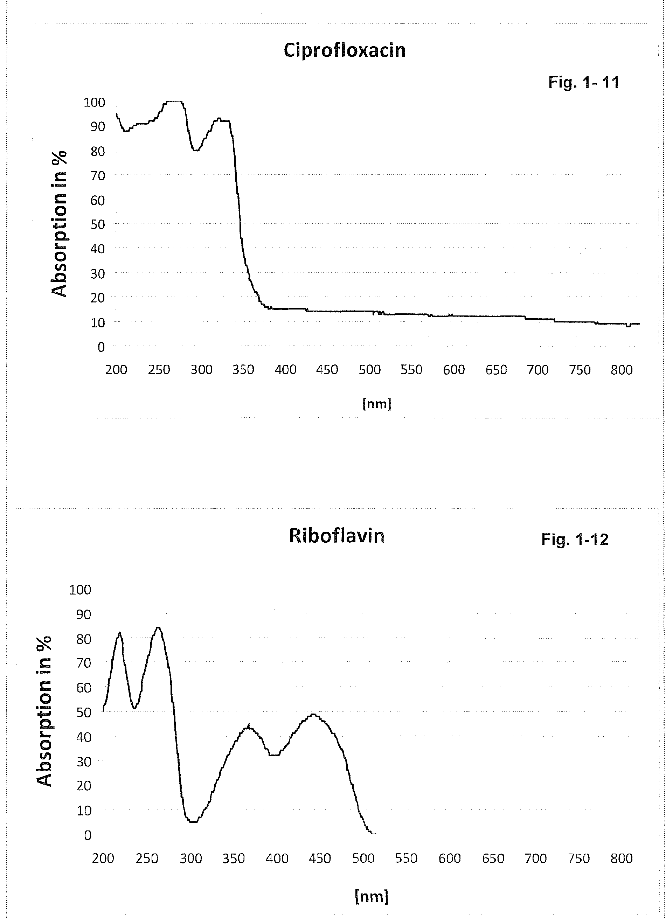

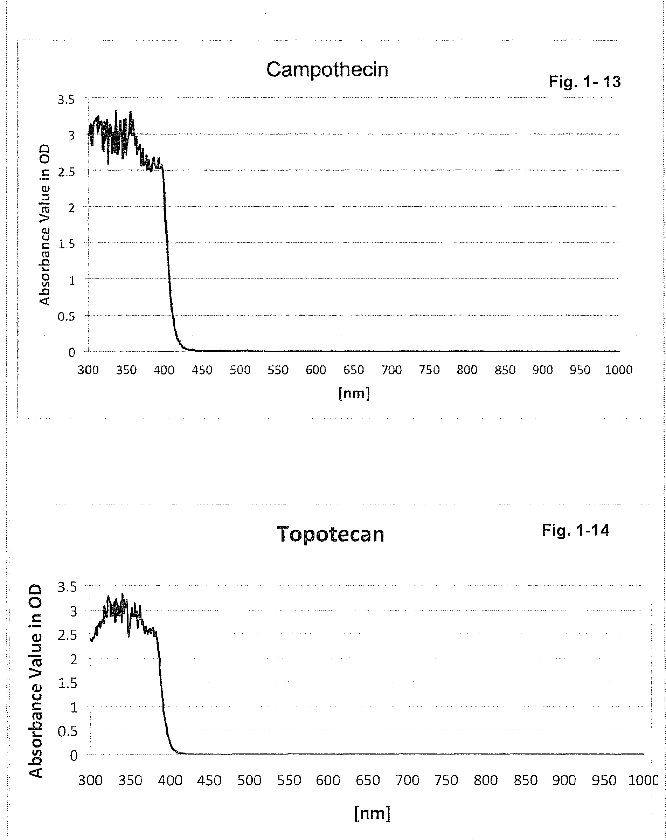

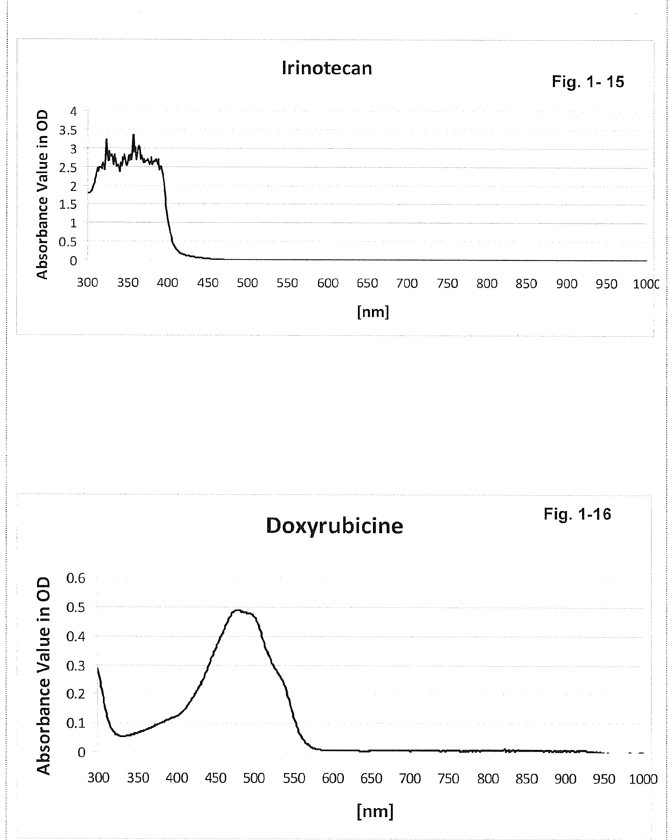

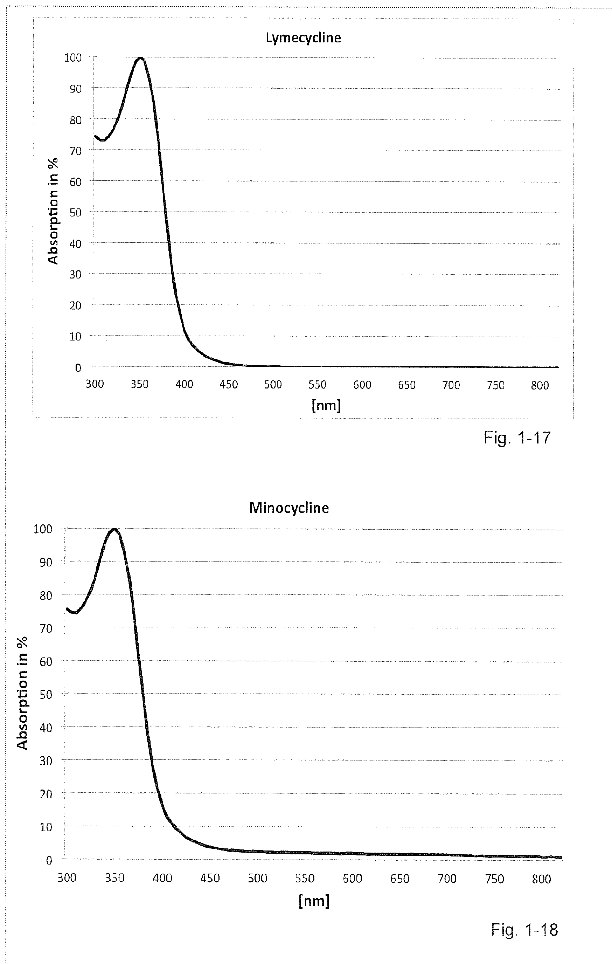

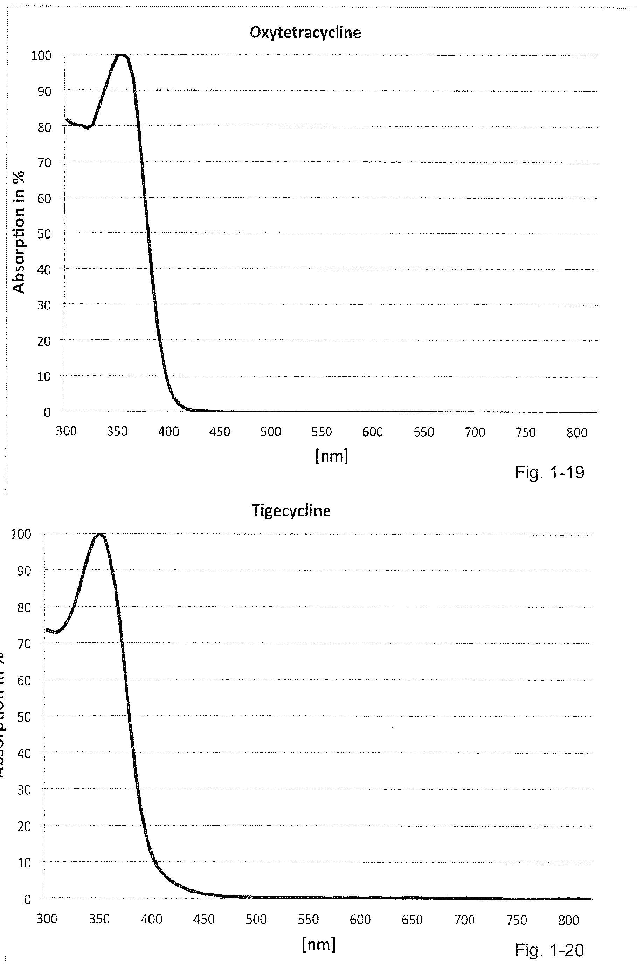

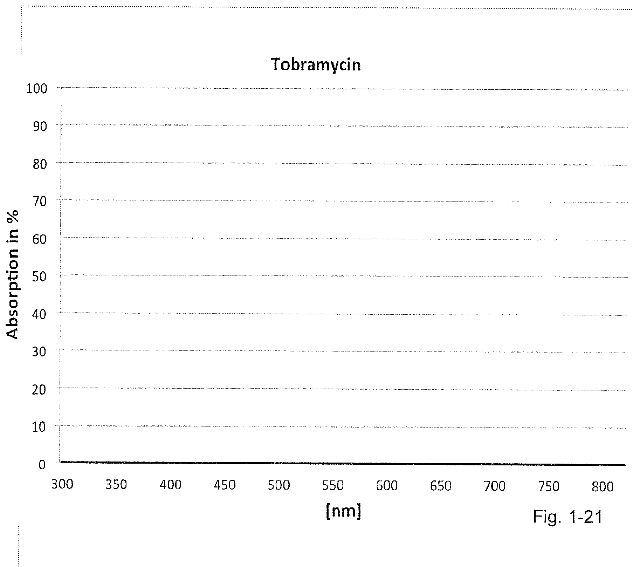

In Figure 1, absorption spectra of some exemplary antibiotic active components comprising a conjugated system of delocalized electrons are presented: Ofloxacin Fig. 1-1, Moxifloxacin Fig. 1-2, Norfloxacin Fig. 1-3, Tetracyline Fig. 1-4, Gatifloxacin Fig. 1-5, Doxycycline Fig. 1-6, Clarithromycin Fig. 1-7, Rifampicin Fig. 1-8, .Levofloxacin Fig. 1-9, Amphotericin B Fig. 1-10, Ciprofloxacin Fig. 1-11, Furthermore, for comparison also the absorption spectrum of the above discussed absorbing vitamine B₂, Riboflavin Fig. 1-12, is shown. Additionally absorption spectra of four exemplary antineoplastic drugs, Campothecin Fig. 1-13, Topotecan Fig. 1-14, Irinotecan Fig. 1-15, and Doxyrubicin Fig. 1-16.

Further, Fig. 1-17 shows the absorption spectrum of Lymecycline, Fig. 1-18 of Minocycline, Fig. 1-19 of Oxytetracycline, Fig. 1-20 of Tigecycline, and 1-21 of Tobramycin. Tobramycin does not exhibit any substantial absorption in the investigated frequency range.

All absorption spectra were taken by a commercially available measurement device, namely a FLUOstar Omega by BMG LABTECH. Some measurement results (with per cent values given) are shown in normalized units.

For illustration, structural formulas are presented for the following exemplary active components of suitable for some embodiments of the method and the pharmaceuticals.

The following exemplary active components comprise a conjugated system of delocalized electrons as shown in the structures below for some the exemplary antibiotics belonging to the quinolone group or the tetracycline group comprising at least one aromatic ring, e.g.
1) Ofloxacin 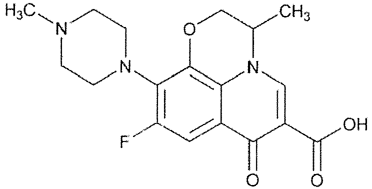
2) Tetracycline 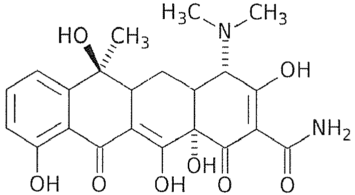
3) Doxycycline 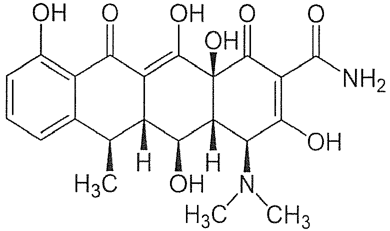 or with a non-saturated carbon tail comprising conjugated double bonds:
4) Amphotericin B 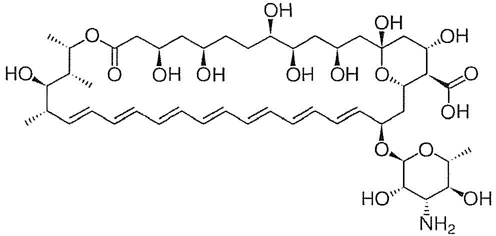 or of the rifamycin group of antibiotics such as
5) Rifampicin 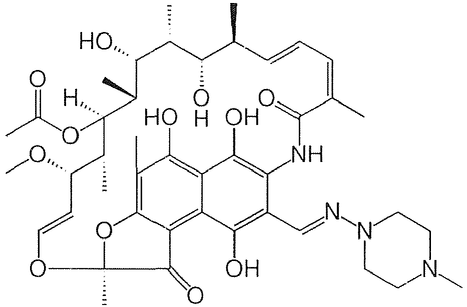 or antineoplastic drugs such as cycline derived anthracyclines, e.g.
6) Doxorubicin 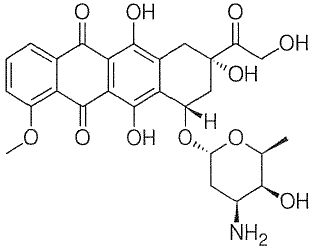
7) Daunorubicin 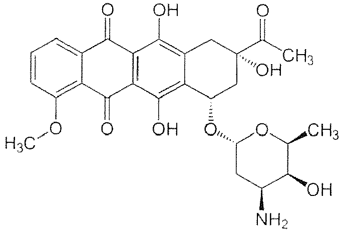
8) Epirubicin 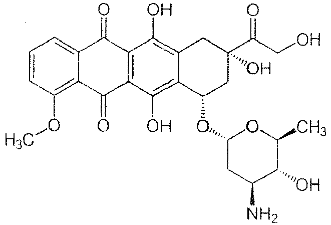
9) Idarubicin 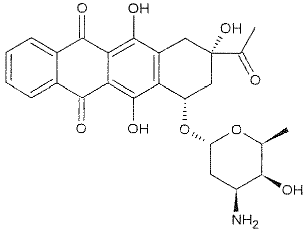
10) Valrubicin 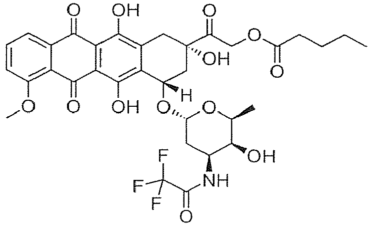 or antineoplastic drugs with structural similarity to quinolones, such as
11) Irinotecan 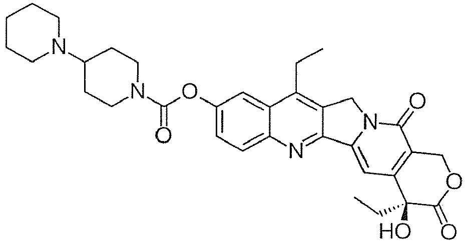
12) Topotecan 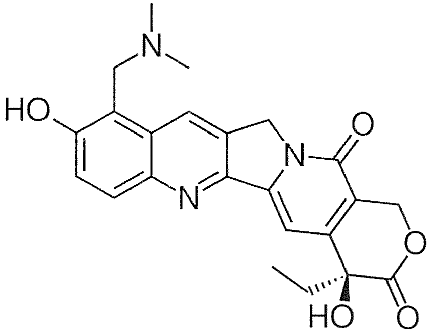
13) Campothecin 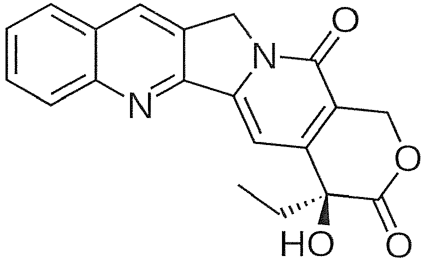
14) Amsacrine

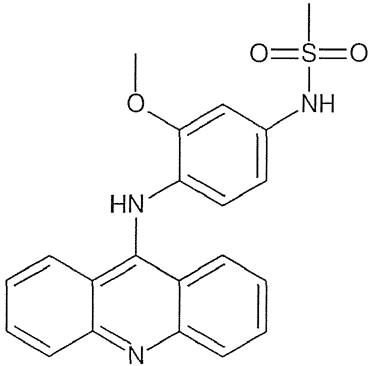

The above shown structures show only a few examples of active components, which absorb electromagnetic radiation, in particular in the UV / VIS spectrum, and which are suitable for some embodiments of the methods or the pharmaceuticals. The intensity and duration of the exposure to the electromagnetic radiation is adjustable according to the application.

The ionizing energy of electromagnetic radiation in a range of wavelength between 400 and 790nm is not sufficient for breaking up most types of chemical bonds: The energy of one photon necessary in nm (wavelength) for breaking the chemical bond can be calculated according to the formula E=hv (h=plank constant, v= frequency). Thus, photons of a wavelength above 400 nm only have an energy of approx. 3 eV = 408.31 nm or less. The bond energy of common chemical bonds is presented in the table below in one photon energy in nm.

| | | | |
|---|---|---|---|
| C-H | 289.67 | O-H | 258.39 |
| C-C | 343.78 | C=N | 194.53 |
| C-O | 334.17 | C=O | 194.53 |
| C-F | 246.67 | C-N | 408.31 |
| C=C | 194.84 | C-S | 461.91 |
| N-H | 305.97 | O-H | 258.39 |

This shows that for breaking most of the bonds, photons with a wavelength of less than 400 nm are required. Since the pharmaceuticals and methods work very well also at wavelength above 400 nm, the effect of the invention clearly goes beyond the known mechanism of free radical generation by antibiotics and UV light.

Below, a further calculation is presented explaining why the method of applying a composition requires only a very low concentration of the active component for the exemplary antibiotic as an exemplary active component, ofloxacin, which is photo-activated by exposure to electromagnetic radiation. A common concentration in the body fluids during antibiotic treatment is 4 mg /l. The molecular weight of ofloxacin is 361 g/mol (→4 mg is approx. 0.00001 mol), thus the concentration of ofloxacin is approx. 0.00001 mol/L which equals approx. 6 x 10¹⁸ molecules of ofloxacin per liter of body fluid. Exemplary embodiments of the method and pharmaceuticals comprise concentration of the active component in the range of 0.001 micromol/L to max 0.01 mol/L.

From the concentration of the active component such as e.g. ofloxacin a theoretical approximate value for the distance between two neighbouring molecules of it can be calculated: assuming that the distance between molecules is always the same, it corresponds to the cube root of 6 x 10¹⁸ molecules of ofloxacin per liter (=dm³) of body fluid: 1.82 10⁶ x 1.82 10⁶ x 1.82 10⁶ molecules per 10 cm x 10 cm x 10 cm. Thus, over the distance of 1 micrometer → 18 molecules of ofloxacin are distributed, corresponding to one molecule every 55 nm. Ofloxacin has the size of approx a sucrose molecule, which is approx. 0.44 nm and from this a rough estimation of a distance between two molecules of ofloxacin is about 100 times as large as the size of a single molecule in both directions.

The distance for one activated molecule to diffuse in a target cell until it hits a target molecule to attack accordingly is approximately in the range of 50 nm. Considering that with the phototoxic effect, there is no selective affinity of an antibiotic for the DNA damaged in such a free radical attack of an activated antibiotic, the probability appears extremely low, that an antibiotic molecule actually gets into contact with the DNA at such a low concentration of the antibiotic and catch a photon at this very moment to create free radical and damage the DNA.

In contrast, with the pharmaceuticals and the methods according to the invention there is a very high affinity and a strong contact between the targeted molecule which is e.g. a pump in resistant target cells, or a targeted molecule of the target cell. Accordingly, photo-activation of the active component such as ofloxacin has a much higher probability to interact and form a covalent bond with the targeted molecule such as a pump.

In the above described example of ofloxacin at 0.01 mol/L the distance between the molecule will be decreased to about the 10 times the size of one molecule. Then the probability of a phototoxic free radical effect is much higher. In some embodiments of the invention, the concentration of the antibiotic is elevated to achieve both a free radical attack of DNA and covalent linking to targeted molecules and pumps or other molecules mediating resistance of resistant target cells.

### Experiments:

For some of the experimental tests MU50 bacteria were used. MU50 is a multidrug-resistant Staphylococcus aureus strain (MRSA). MRSA are bacteria which have naturally evolved under antibiotic selection pressure and which are known to be resistant to antibiotics including penicillins (methicillin, dicloxacillin, nafcillin, oxacillin, etc.) and cephalosporins. MU50 is additionally resistant to macrolides, quinolones and aminoglycosides.

For experiments 1 to 3 MU50 inocula were prepared from fresh subcultures grown on Mueller Hinton Agar at a titer of 0.5 McFarland, corresponding to a cell density of 1 x 10⁸ bacterial cells/ml. The minimum inhibitory concentration (MIC), which inhibits the visible growth of a microorganism after an overnight incubation was determined for the following antibiotics :

| Drug | MIC (µg/ml) |
|---|---|
| Quinolone (Cirpofloxacin) | > 16 |
| Gentamicin | > 16 |
| Oxacillin | > 32 |
| Penicillin | > 8 |
| Quinupri stin/dalfopri stin | 0.12 |
| Teicopanin | 8 |
| Tetracycline | 32 |
| Timeth/sulfa* | 0.12 |
| Timeth/sulfa* | 2.28 |
| Vancomycin | 8 |

| | |
|---|---|
| *Trimethoprim/sulfamethoxazole combination of trimethoprim and sulfamethoxazole, in the ratio of 1 to 5. | |

**Experiment 1:** The first experiment compares the effect of two antibiotics incubated with multi-drug resistant MU 50 bacterial cells accompanied by exposure to UVA light at 365 nm. This first antibiotic used was Ofloxacin, a quinolone absorbing in the UV/Vis range and the second antibiotic used was Tobramycin, an aminoglycoside, which does not absorb in the UV/Vis range. Furthermore, the antibiotics were administered in the presence and absence of riboflavin, to test the free radical effect of riboflavin.

### Method:

A MU50 inoculum with a cell density of 1 x 10⁸ bacterial cells /ml was tenfold diluted to 1x10⁷ cells /ml, for preparing samples comprising bacteria, antibiotic and optionally riboflavin as well as negative controls lacking antibiotic in 1 mm thick UV transparent chambers according to the following scheme:
Experiment 1A:

| | |
|---|---|
| Vol. bacterias / chamber | 1 µl |
| Vol. antibiotic ofloxacin 600 µg/ml | 4 µl |
| Vol. NaCl 0.9 % or Ribo 2 mg/ml | 5 µl |
| Vol Total chamber | 10 µl |

and Experiment 1B:

| | |
|---|---|
| Vol. bacterias / chamber | 0.5 µl |
| Vol. antibiotic tobramycin 300 µg/ml | 4.5 µl |
| Vol. NaCl 0.9 % or Ribo 2 mg/ml | 5 µl |
| Vol Total chamber | 10 µl |

After addition of the antibiotic and optionally riboflavin to the bacterial samples they were incubated for 1 or for 30 minutes in Experiment 1A and for 30 minutes in Experiment 1B prior to the UV-A exposure at 365 nm with 9 mW/cm² for 10 minutes.

In Experiment 1A: the added antibiotic was ofloxacin at a concentration of 240 µg/ml. In Experiment 1B the added antibiotic was tobramycin at a concentration of 135 µg/ml.

The results of Experiment 1A are presented in the following table:

| No: | Incubation | Riboflavin | ofloxacin | UV A | CFU first run | CFU second run | Killed first run | Killed second run |
|---|---|---|---|---|---|---|---|---|
| 1 | 1 min | - | - | - | 576 | 409 | 0% | 0% |
| 2 | 1 min | + | + | + | 144 | 98 | 75% | 76% |
| 3 | 1 min | - | + | + | 9 | 9 | **98%** | **98%** |
| 4 | 1 min | + | + | - | 574 | 391 | 0% | 4% |
| 5 | 1 min | - | + | - | 505 | 351 | 12% | 14% |
| 6 | 30 min | + | + | + | 171 | 193 | 70% | 53% |
| 7 | 30 min | - | + | + | 7 | 15 | **99%** | **96%** |
| 8 | 30 min | + | + | - | 570 | 363 | 1% | 11% |
| 9 | 30 min | - | + | - | 490 | 350 | 15% | 14% |

Conclusions from Experiment 1A with ofloxacin which has a UV-A absorption peak at 335nm, i.e not at the same wavelength as riboflavin which has a UV-A absorption peak at 365nm:
- The maximal killing effect of 98% occurs in the presence of ofloxacin with exposure to UV-A at 365 nm and in the absence of riboflavin (samples No. 3), even though the radiation source used is at the peak of riboflavin absorbance of 365nm and not at the peak of ofloxacin of 335 nm.
- In the presence of riboflavin and ofloxacin with exposure to UV-A at 365 nm results in a killing effect of only 75% and 76% (samples No. 2)
- In the absence of exposure to UV-A at 365 nm the killing effect is reduced to 12% and 14% in the first and second run, respectively (samples No. 5).
- In the presence of riboflavin and ofloxacin without exposure to UV-A at 365 nm the killing effect is even reduced to only 0% and 4% (samples No. 4)

Clearly experiment 1A demonstrates that the exposure to UV-A is essential for effective killing the multidrug resistant MU50 bacterial cells. And furthermore, it shows that riboflavin which is known to be very efficient in the generation of free radicals and causing DNA damage, the killing rate of ofloxacin decreases. This effect is attributed to competition for the absorption of electromagnetic radiation in the same range with a peak of at 365 nm for riboflavin at of approx. 340 nm for ofloxacin. Importantly, this experiment demonstrates photoactivated ofloxacin alone is more effective in killing and reducing proliferation of MU 50 cells than photoactivated riboflavin. This is a further indication showing that the method with photoactivation of ofloxacin is working by another mechanism than the free-radical effect.

The reduction of the killing effect of ofloxacin with UV-A exposure in the presence of riboflavin results from competition of riboflavin with ofloxacin for UVA at absorption at 365 nm. Thus, riboflavin diminishes the activation of the antibiotic by effectively reducing exposure of the antibiotic to UVA and thereby reduces the killing of the multidrug resistant MU50 cells from 98% to about 75%.

The results of Experiment 1B are presented in the following table:

| No: | Incubation | Riboflavin | tobramycin | UVA | CFU first run | CFU second run | Killed first run | Killed second run |
|---|---|---|---|---|---|---|---|---|
| 11 | 30 min | - | - | - | 645 | 623 | 0% | 3% |
| 12 | 30 min | + | + | + | 144 | 159 | **78%** | **75%** |
| 13 | 30 min | - | + | + | 416 | 481 | 36% | 25% |

Conclusions from Experiment 1B with tobramycin which does not absorb UV-A at 365nm, i.e at the wavelength where riboflavin absorbs:
- The maximal killing effect of 78% and 75% occurs in the presence of tobramycin and riboflavin with exposure to UV-A at 365 nm (samples No. 12) due to activated riboflavin

Thus, in contrast to the results seen above with ofloxacin, the killing effect of tobramycin with the exposure to UV-A in the presence of riboflavin is higher than in its absence. Since tobramycin does not absorb UV-A, exposure of the target bacteria to UV-A only enhances the killing effect contributed by activation of riboflavin. And, the comparison of sample No. 3 with sample No. 12 shows that activated riboflavin is not as efficient as the activated quinolone, ofloxacin, in killing MU50 cells. It is conceived that ofloxacin in combination with UV-A exposure overcomes a specific resistance mechanism to the antibiotic ofloxacin whereas riboflavin in combination with UV-A exposure kills the multi-drug resistant bacteria by the known mechanism of modification of nucleic acids due to free radical generation by activated riboflavin. Thus, the killing effect results from a mechanism which is different from overcoming a specific resistance to a quinolone antibiotic.

Furthermore, the killing effect of tobramycin and riboflavin with UV exposure are additive. Thus, there is a contribution by UV exposed tobramycin to the killing effect of UV exposed riboflavin. This effect is assumed to be caused by non-specific free radical generation as it is known for antibiotics which are exposed to UV light.

### Experiment 2:

### Method:

- A MU50 inoculum with a cell density of 1 x 10⁸ bacterial cells /ml was diluted by a factor of one hundred to 1x10⁶ cells /ml, for preparing samples 1 and 2
- Samples 1 and 2 were exposed to UV-A at 365 nm and 10 mW/cm² in 1 mm thick UV transparent chambers for 10 minutes either in the presence or in the absence of the antibiotic moxifloxacin at a concentration of 5 µg/ml. This low concentration was chosen so as to detect threshold effects at the low edge of a just marginally effective concentration level of the antibiotic.
- After the exposure to UV-A either moxifloxacin (sample 1A) or NaCl (sample 1B as a control) was added and incubated for 2 hours prior to plating an aliquot on Mueller Hinton Agar (Becton Dickinson).
- The number of colony forming units (CFU) was determined after 24 hours of incubation at 37°.

The results show:

| Sample | moxifloxacin during UV / after UV | control: CFU without UVA | CFU with UVA |
|---|---|---|---|
| 1 → 1 A | During 5 µg/ml | 516 | 3 (0 %) |
| | After 7.5 µg/ml | | |
| 1 →1 B | During 5 µg/ml | 400 | 119 (30%) |
| | After 2.5 µg/ml | | |
| 2 | During 0 µg/ml | 480 | 359 (75%) |
| | After 0 µg/ml | | |

### Conclusions:

Surprisingly, when combined with UV-A exposure, at a low concentration of only 5 µg/ml moxifloxacin effectively eliminates or drastically reduces cell proliferation of MU50 multi-drug resistant bacterial cells. In contrast, the control without UV-A exposure confirms as expected, that moxifloxacin has no significant effect on the multidrug resistant MU50 cells.

However, with the UV-A exposure in the presence of moxifloxacin 5 µg/ml during and 2.5 µg/ml after the UV treatment 30% of MU50 cells are rendered susceptible to the antibiotic (sample 1B).

Strikingly, the additional treatment with moxifloxacin at a concentration of still only 7.5 µg/ml for two hours after the UV exposure significantly increases the killing of MU50 cells to a 0 to 1% survival rate (sample 1A).

This result with samples 1A shows that after the initial effect of moxifloxacin during the 10 minutes of activation by UV-A, the killing effect is significantly increased with further moxifloxacin added after the UV-A treatment. This implies the existence of two separate mechanisms of killing the MU50 originally antibiotic resistant target cells:
During the 10 minutes of exposure to UV-A at 365 nm at 10 mW/cm² moxifloxacin was activated. This had two effects: First, excited moxifloxacin produced free radicals. These free radicals caused non-specific damage of the nucleic acids as known of photo-activated antibiotics in the state of the art and destroyed some of the MU50 target cells or at least slowed down their proliferation. This killing effect occurred instantly during the exposure to UV-A, and the effect is short lived, because the free radicals are extremely reactive. For reference see e.g. Cruz de Carvalho, M. H. Drought stress and reactive oxygen species: Production, scavenging and signaling. Plant Signal Behav 3, 156-165 (2008).

The second effect of exposure to UV-A at 365 nm at 10 mW/cm² is consistent with an activation of moxifloxacin molecules rendering them capable of the formation of specific covalent bonds with pump proteins in the cell membrane of MU50 cells. This permanent binding of moxifloxacin permanently destroyed those pump molecules which in the absence of concomitant exposure to UV-A provided for resistance of MU50 cells to moxifloxacin. Therefore, this second effect is not as short lived and not dependent on continuing exposure to UV-A, but instead it re-sensibilises the MU50 target cells to the antibiotic moxifloxacin. This conceived mechanism explains the significant increase in the killing of MU50 cells during the 2 hour incubation after the exposure to UV-A.

### Experiment 3:

### Method:

- An MU50 inoculum with a cell density of 1 x 10⁸ bacterial cells /ml was hundredfold diluted to 1x10⁶ cells /ml
- Equal volumes of bacterial cells and either a solution of antibiotic (2 µg/ml ciprofloxacin or 20 µg/ml tetracycline) or 0.9% NaCl were incubated for 30 min at room temperature
- After incubation an aliquot of 22 µl was placed into a 1 mm thick UV transparent quartz cuvette and followed by one of
   A) exposure to a CXL UV lamp at 18 mW/cm² for 5 min; or
   B) exposure to a laser at 405 nm at 300 mW/cm² for 1min; or
   C) no UV-A exposure
- After the exposure to one of A to C, 10 µl of the cell mix was mixed with an equal volumes of antibiotic (2 µg/ml ciprofloxacin or 20 µg/ml tetracycline) or 0.9% NaCl as a control and incubated for 2 hours
- A 10 µl aliquot of the cell mix was diluted into 390 µl of 0.9% NaCl prior to plating a 100 µl aliquot on Mueller Hinton Agar (Becton Dickinson).
- The number of colony forming units (CFU) was determined after 24 hours of incubation at 37°C.

The results are presented in the following table:

| First Incubation 30 min | Second Incubation 2 hrs | SampleNo: | CFU UV Lamp | CFU Light Laser | CFU No radiation |
|---|---|---|---|---|---|
| tetracycline | tetracycline | 1 A | 297 | 36 | 574 |
| | | IB | 343 | 133 | 591 |
| | | 1 avg | **320** | **85** | 583 |
| tetracycline | NaCl | 2 A | 359 | 62 | 500 |
| | | 2 B | 373 | 210 | 652 |
| | | 2 avg | 366 | 136 | 576 |
| ciprofloxacin | ciprofloxacin | 3 A | 4 | 1 | 159 |
| | | 3 B | 0 | 0 | 147 |
| | | 3 avg | **2** | **1** | 153 |
| ciprofloxacin | NaCl | 4 A | 2 | 5 | 227 |
| | | 4 B | 14 | 12 | 267 |
| | | 4 avg | 8 | 9 | 247 |
| NaCl | NaCl | 5 A | 556 | 337 | 531 |
| | | 5 B | 563 | 425 | 624 |
| | | 5 avg | 560 | 381 | 578 |

### Conclusions:

The third experiment shows that the killing effect of the antibiotic treatment is much increased both by the exposure to electromagnetic radiation in the UV range as well as in the visible range. Particularly, the activation of ciprofloxacin resulted in the killing of multi-drug resistant MU 50 bacterial cells. Tetracycline with exposure UV-A or light seems to be less efficient than ciprofloxacin. One explanation might be that tetracycline is not as efficiently photoactivated, i.e. not absorbing as much light and therefore not disposing as much energy to bind and destroy the pumps or other molecules of the resistant target cells which mediate the resistance.

### Experiment No. 4: First toxicity test:

### Method:

- Mice fibroblast were grown according to conventional in vitro cell culture protocol
- The culture medium was exchanged with one of the following solutions
   1: Tobramycin 75 µg/ml in NaCl 0.9 %
   2: Tetracycline 10 µg/ml in NaCl 0.9 %
   3: Ciprofloxacin 1 µg/ml in NaCl 0.9 %
   4: NaCl 0.9 %
- After 30 minutes of pre-incubation the cultures were exposed to
   A: No treatment
   B: CXL: 18 mW/cm2 5 minutes
   C: Laser 405 nm 300 mW on 6 mm during 1 minutes
   and a blue mark was applied on the petri dish for identification of the region which is irradiated
- Exchange of the solutions 1 to 4 with culture medium and incubation for 24 hours at 37°C
- The plates were visually inspected and documented with photographs (not shown). The number of colony forming units (CFU) was determined after 24 hours of incubation at 37°C. No toxicity in term of necrosis and apoptosis was found.

### Conclusion:

No regions of reduced growth could be identified, indicating no toxicity in terms of necrosis and apoptosis.

### Experiment No. 5: Second toxicity test:

In the second toxicity test fibroblasts were grown in RPMI cell culture medium medium with 10% fetal bovine serum and 1% of penicillin-streptomycin-fungizione (100x).

150 µl of cells were added well plates, antibiotic was added to a final concentration as listed in the following table:

| | | µg/ml in 50 µl | µg/ml final concentration |
|---|---|---|---|
| 1 | Moxifloxacin | 16 | 4 |
| 2 | Ofloxacin | 16 | 4 |
| 3 | Norfloxacin | 16 | 4 |
| 4 | Levofloxacin | 16 | 4 |
| 5 | rifampicin | 40 | 10 |
| 6 | Clarithromycin | 16 | 4 |
| 7 | Ciprofloxacin | 16 | 4 |
| 8 | Tetracycline | 12 | 3 |
| 9 | Doxycycline | 12 | 3 |
| 10 | NaCl 0.9% | NA | NA |

After a 12 hour incubation exposed to a pulse of electromagnetic radiation with 405 nm.
A: pulsed for 10 ms on and 90 ms of for 10 minutes or
B: pulsed for 5 ms on and 45 ms of for 10 minutes
C: no exposure to electromagnetic radiation

The result was obtained by counting viable cells for determination of the percentage of viable cells, with 100% viability being equal to the number of cells delivered to each well. Visual analysis of photographs of the cells from all the wells showed no signs of toxicity on the cells.

The results are shown in the table below
1) Percentage of viable cells:

| Sample No | 1 | 2 | 3 | 4 | 5 | 6 | 7 | 8 | 9 | 10 |
|---|---|---|---|---|---|---|---|---|---|---|
| A | 43 | 48 | 30 | 25 | 37 | 57 | 38 | 28 | 34 | 58 |
| C | 85 | 74 | 87 | 86 | 92 | 85 | 84 | 79 | 74 | 85 |
| B | 45 | 50 | 40 | 24 | 43 | 36 | 35 | 30 | 49 | 57 |

2) Percentage of viable cells normalized for the dead cells in the control sample without light

| Sample No | 1 | 2 | 3 | 4 | 5 | 6 | 7 | 8 | 9 | 10 |
|---|---|---|---|---|---|---|---|---|---|---|
| A | 51 | 65 | 34 | 29 | 40 | 67 | 45 | 35 | 46 | 68 |
| C | 100 | 100 | 100 | 100 | 100 | 100 | 100 | 100 | 100 | 100 |
| B | 53 | 68 | 46 | 28 | 47 | 42 | 42 | 38 | 66 | 67 |

Normalized for the dead cell in NaCl group including light

| Sample No | 1 | 2 | 3 | 4 | 5 | 6 | 7 | 8 | 9 | 10 |
|---|---|---|---|---|---|---|---|---|---|---|
| A | 74 | 95 | 51 | 43 | 59 | 98 | 66 | 52 | 67 | 100 |
| B | 79 | 101 | 69 | 42 | 70 | 63 | 62 | 57 | 99 | 100 |

### Conclusion:

The results show that the toxicity depends on the antibiotic used and that the light treatment has a small toxicity on the fibroblasts.

### Experiment No. 6: Third toxicity test:

In this experiment the toxicity of the exposure to electromagnetic radiation was tested by monitoring the reaction of the skin of a human inner forearm with direct contact of the light source to the skin and a exposure to 405nm with a 10W LED and a pulsed application of 10 ms ON and 90 ms OFF for a duration of 10 minutes.

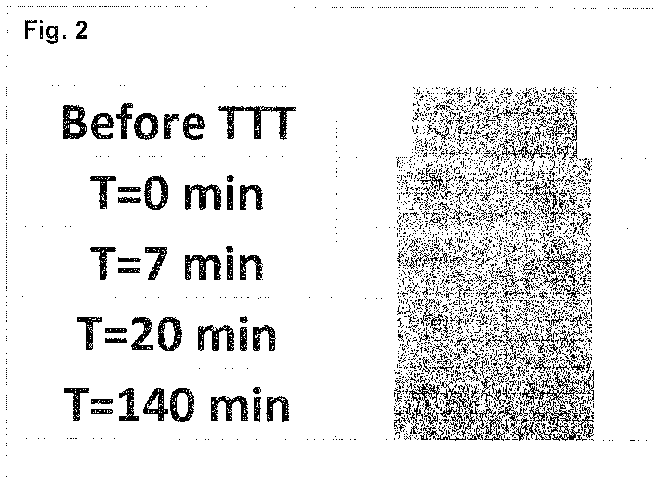

Result: The reaction was analyzed on photographs taken before the exposure to +electromagnetic radiation TTT and at the end of the 10 minutes exposure (T=0) and at T = 7, 20 and 140 minutes as shown in Fig.2. The circular reaction on the left and on the right in this pictures taken of the skin at the indicated times are reactions to an identical treatment with two identical LED lamps.

### Conclusion:

As can be seen on the photographs there is a small vasodilator effect appearing as darkening within the circles of exposure in the pictures above. However, this effect dissipates within 140 minutes. The exposure was not painful and did not cause any skin tissue necrosis nor any tanning of the skin.

### Experiments 7, 8 and 9:

In Experiments 7 to 9 the effect of some exemplary photo-activated antibiotics is tested on two different multi resistant bacterial strains:
In Experiment 7 MU 50 multi-resistant bacteria as described above for Experiments 1 to 3 were tested.
In Experiment 8 and 9 PA01 Pseudamonas aeruginosa MultiR genetically modified bacteria were tested. This PA01 strain is genetically modified with a quinolone pump and conferring resistance to antibiotics of the quinolone group of antibiotics.

The following experimental set-up was done:
For experiment 7 and 8
   A → Pulse ON 10ms energy 0.1 J, OFF 90ms during 10 minutes
   D → Control no Pulse
   G → Pulse ON 5ms energy 0.05 J, OFF 45ms during 10 minutes
For experiment 9
   A → Pulse ON 3ms energy 0.03 J, OFF 27ms during 10 minutes
   D → Control no Pulse
   G → Pulse ON 1ms energy 0.01 J, OFF 9ms during 10 minutes

The following experimental procedure are the same for experiments 7, 8 and 9: The bacterial cells were grown in Mueller Hinton Broth in the presence of an antibiotic at a concentration according to the table below until they reached turbidity of 1 according to the McFarland Standard (= 3 x 10⁸ bacteria / ml) :
The samples were treated with various active components and concentrations according to the tables below:
For experiment 7

| Sample number | antibiotic | final concentration (µg/ml) |
|---|---|---|
| 1 | Moxifloxacin | 4 |
| 2 | Ofloxacin | 4 |
| 3 | Norfloxacin | 4 |
| 4 | Levofloxacin | 10 |
| 5 | Rifampicin | 12 |
| 6 | Clarithormycin | 3 |
| 7 | Ciprofloxacin | 4 |
| 8 | Tetracycline | 3 |
| 9 | Doxycycline | 3 |
| 10 | NaCl 0.9% as a control | NA |

For the experiment 8:

| Sample number | antibiotic | final concentration (µg/ml) |
|---|---|---|
| 7 | Moxifloxacin | 4 |
| 8 | Norfloxacin | 4 |
| 9 | Levofloxacin | 4 |
| 10 | Rifampicin | 10 |
| 11 | Clarithromycin | 3 |
| 12 | NaCl as a control | 3 |
| 7' | Ciprofloxacin | 4 |
| 8' | Tetracycline | 3 |
| 9' | Doxycycline | 3 |

For the experiment 9:

| Sample number | antibiotic | final concentration (µg/ml) |
|---|---|---|
| 7 | Ciprofloxacin | 4 |
| 8 | Tetracycline | 3 |
| 9 | Doxycycline | 3 |
| 10 | Moxifloxacin | 4 |
| 8' | Norfloxacin | 4 |
| 9' | Levofloxacin | 4 |
| 10' | Rifampicin | 10 |
| 11' | Clarithromycin | 3 |
| 12' | NaCl as a control | NA |

At the beginning time t₁ = 0, the bacterial cells are transferred to a well plate and diluted by a factor of 100 with medium and accordingly the antibiotic concentration was also decreased by a factor of 100. For each antibiotic two patterns of pulsed exposure for photo-activation were applied and a control without exposure to radiation. For example in experiment 7 and 8, pulse ON 10 ms energy 0.1 J, OFF 90 ms during 10 minutes and pulse ON 5 ms energy 0.05 J, OFF 45 ms i.e. in both treatments a 10% ON and a 90% OFF pattern and both for a duration of 10 minutes.

Cell growth was regularly measured by analyzing light absorption between 595 and 600 nm with the FLUOstar Omega - BMG Labtech spectrophotometer. Incubation for 2 to up 36 hours at 37°C until time = t₂
At time = t₂ one of the antibiotics is added to obtain the same concentration of antibiotic as during the original growth phase prior to time t₁ = 0 as listed in the table above.

Incubation for further 2 to 4 hours at 37°C in the presence of antibiotic until time = t₃. As expected for a multi-resistant bacterial strain the bacteria continued to grow in the presence of antibiotic, even at the same rate.

At time = t₃ the bacterial cells in the well plate are exposed to a pulse of electromagnetic radiation with a 400nm LED following the previous pattern, Thus, during the ten minutes of exposure the ON time is 10% of the total time of a cycle.

The cell growth was analyzed for approx. ten hours after t₃ until t₄. by measuring the light absorption between 595-600 nm. The absorbance value correlates to the colloidal effect and the number of bacteria in the sample.

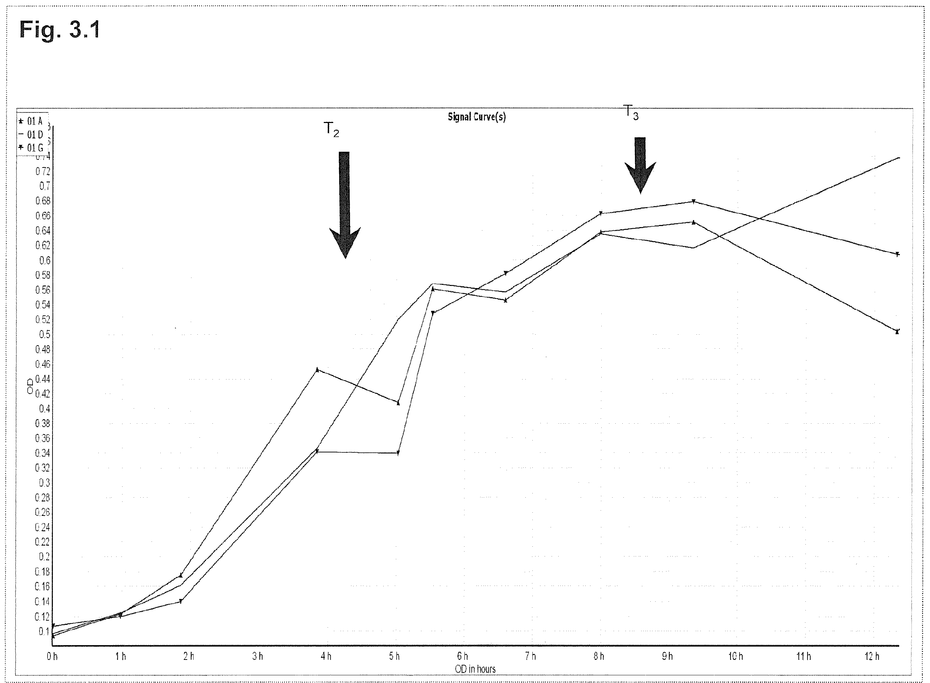

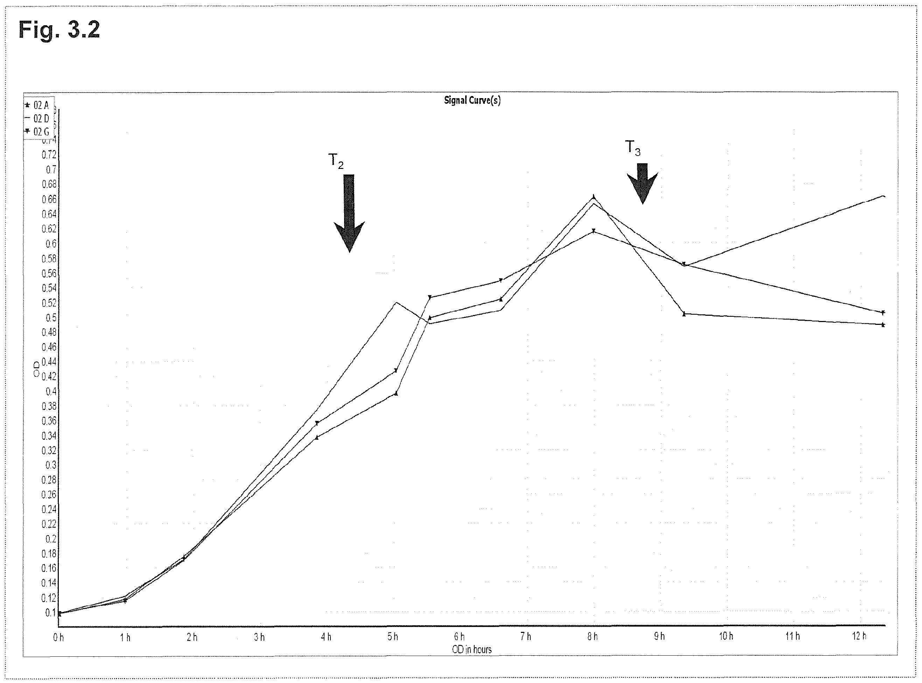

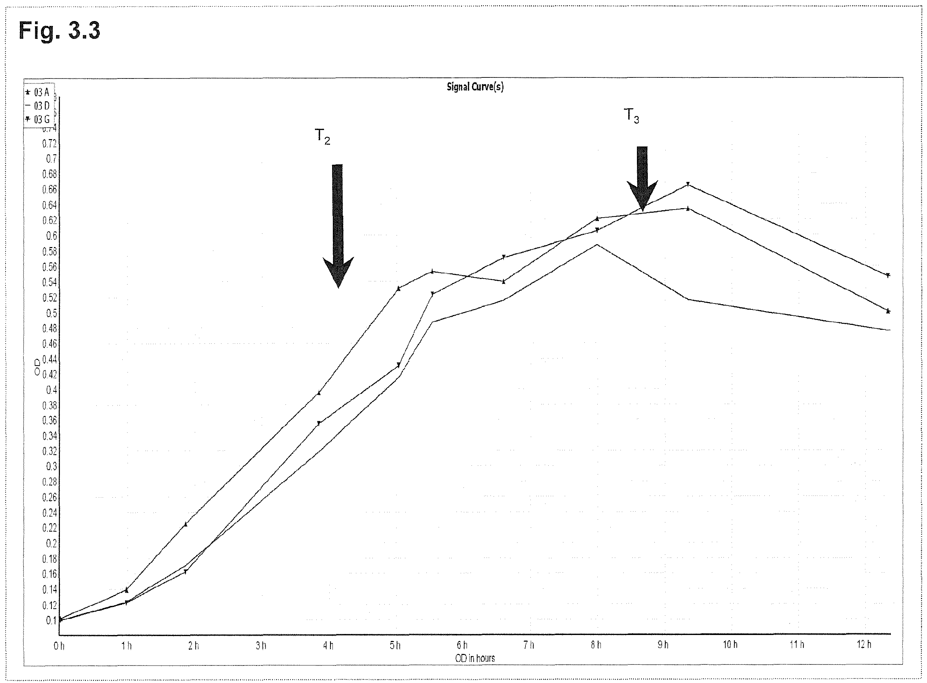

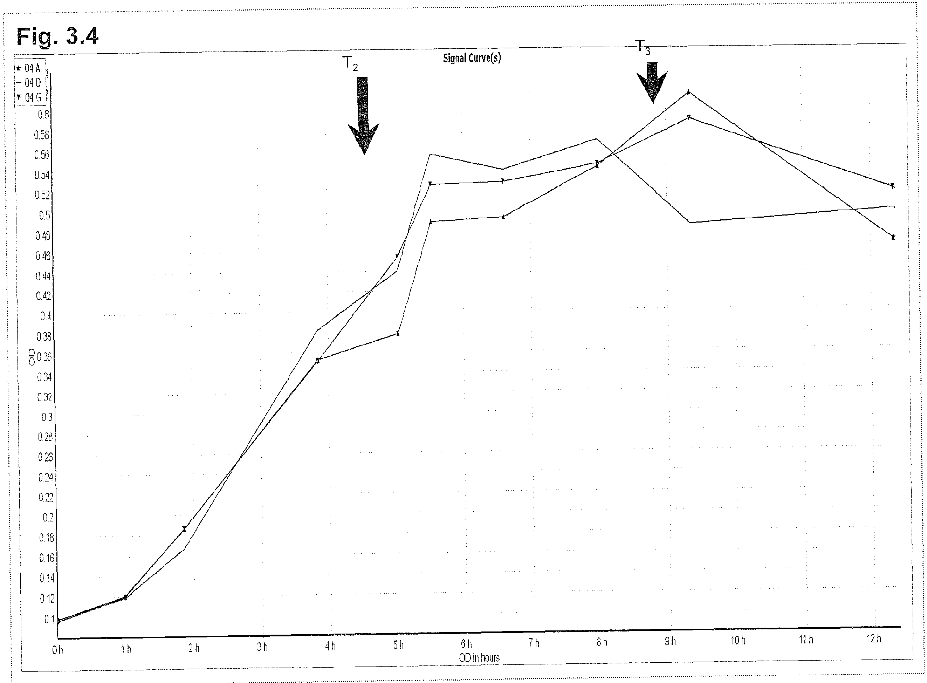

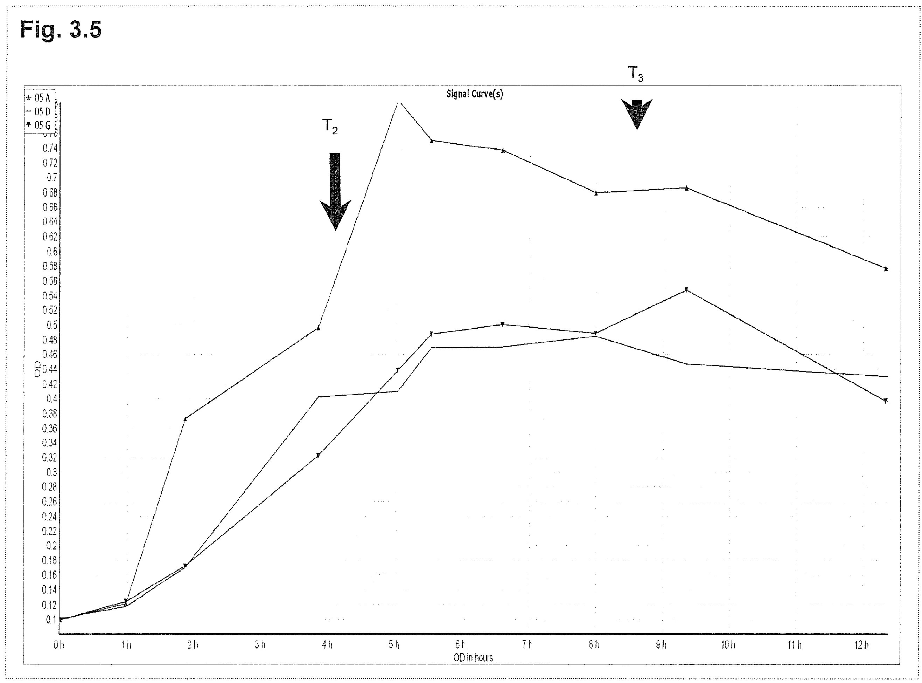

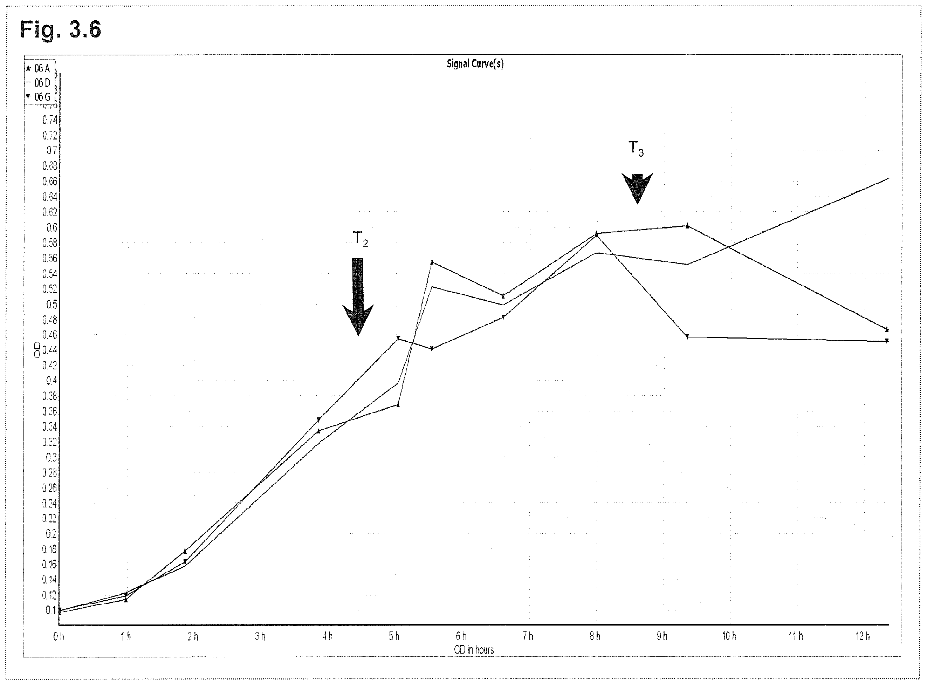

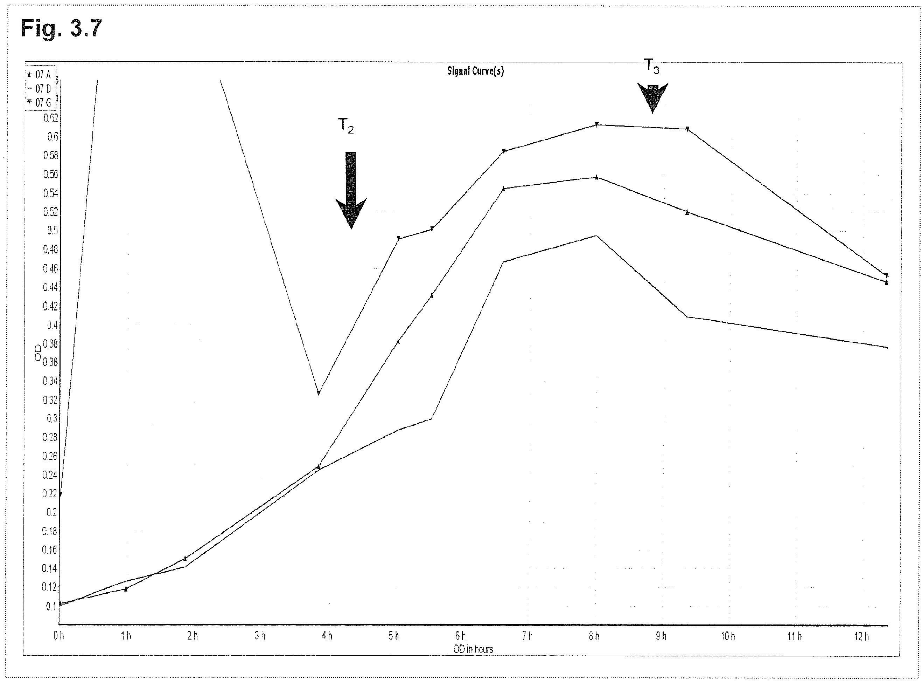

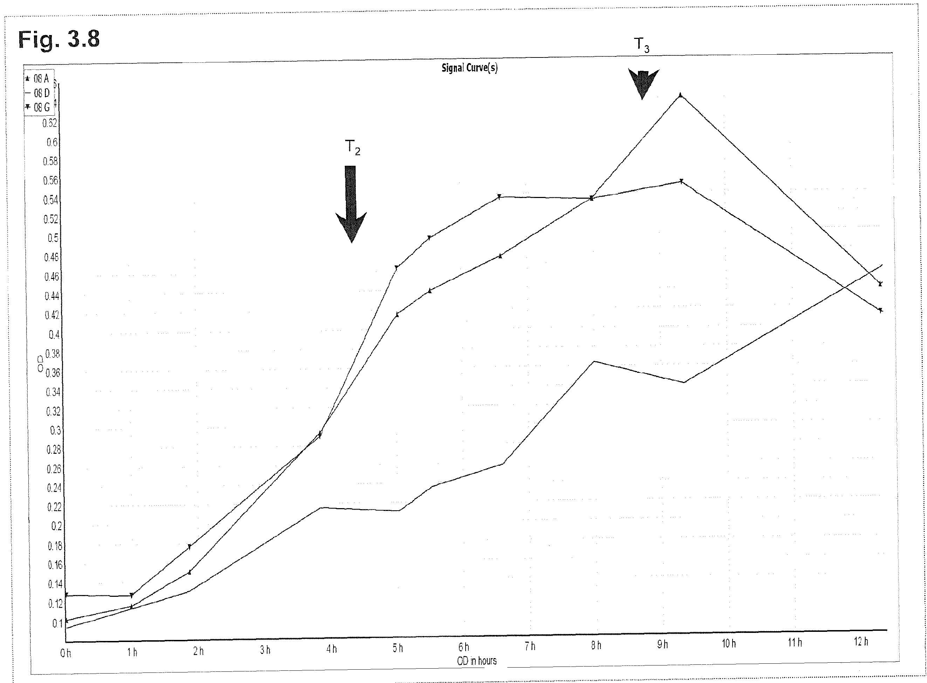

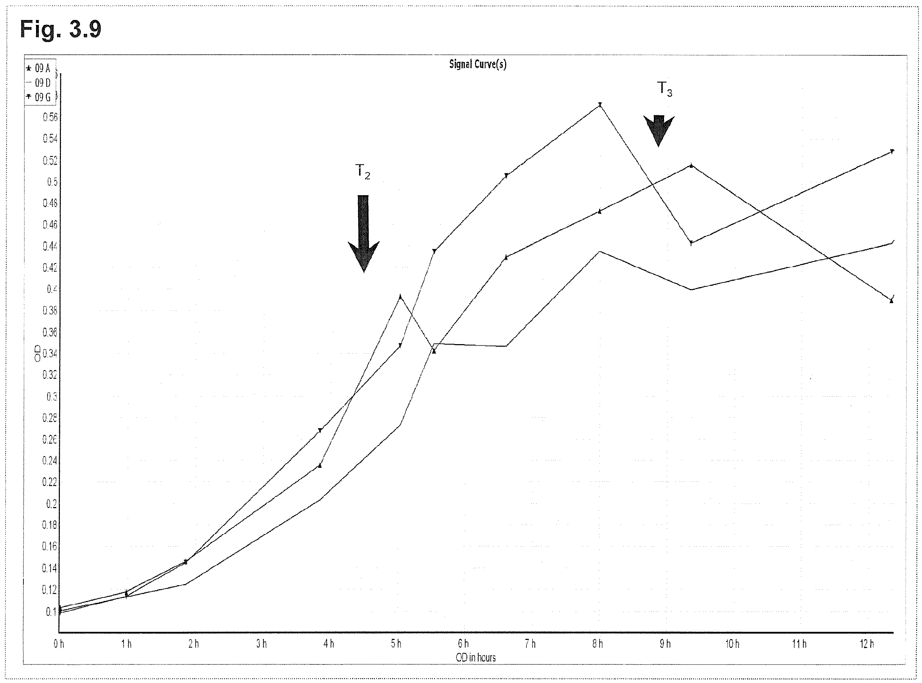

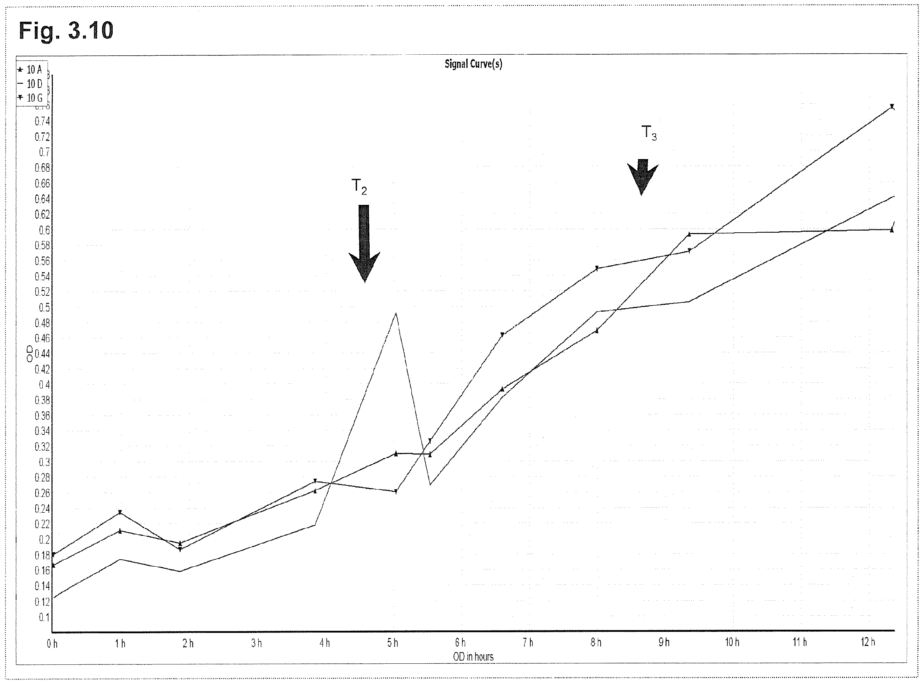

The growth curves of the bacterial samples treated according to experiments 7, 8 and 9 are shown in the graphs of Fig 3, Fig. 4 and Fig 5., respectively.

These tables below list for each bacterial sample the difference in absorption values representing the difference in the number of bacteria present in each sample by subtracting the absorption values at time t₄, which is the last measurement of the growth curve presented in Fig. 3 to 5 from the last absorption value before the treatment with light at time t₃. The values of A and G represent the effect of the treatment of the bacterial samples with two different light patterns for activation of the antibiotic as described above. The values of D correspond to the control samples without photo-activation of the antibiotic:
Experiment 7

| | 1 | 2 | 3 | 4 | 5 | 6 | 7 | 8 | 9 | 10 |
|---|---|---|---|---|---|---|---|---|---|---|
| A | -0.1342 | -0.1741 | -0.1198 | -0.0749 | -0.1027 | -0.1249 | -0.1112 | -0.0939 | -0.083 | 0.1295 |
| D | 0.102 | 0.0107 | -0.1119 | -0.0304 | -0.0542 | 0.0956 | -0.1183 | 0.0942 | 0.0072 | 0.1471 |
| G | -0.0547 | -0.1112 | -0.0589 | -0.027 | -0.0914 | -0.1383 | -0.16 | -0.1218 | -0.0429 | 0.2074 |

Experiment 8

| | 7 | 8 | 9 | 10 | 11 | 12 | 7' | 8' | 9' |
|---|---|---|---|---|---|---|---|---|---|
| A | 0.0034 | -0.0491 | -0.0726 | 0.0137 | 0.0106 | 0.0659 | -0.1665 | -0.0342 | -0.0365 |
| D | 0.1549 | 0.0039 | 0.0676 | 0.0679 | 0.1041 | 0.1246 | 0.0103 | 0.0877 | 0.0735 |
| G | -0.165 | -0.1879 | -0.131 | -0.0678 | -0.0396 | 0.0427 | -0.1551 | -0.061 | 0.0181 |

Experiment 9

| | 7 | 8 | 9 | 10 | 8' | 9' | 10' | 11' | 12' |
|---|---|---|---|---|---|---|---|---|---|
| A | -0.2193 | -0.1186 | -0.153 | -0.2813 | -0.3082 | -0.2926 | -0.0808 | -0.1708 | 0.0101 |
| D | -0.1443 | 0.0485 | -0.0464 | 0.0077 | -0.1441 | 0.0064 | 0.0562 | -0.0024 | 0.3067 |
| G | -0.2785 | -0.1025 | -0.0553 | -0.1624 | -0.2048 | -0.2451 | -0.0196 | -0.0994 | 0.2312 |

Looking at these results it can be seen e.g. for sample 1 of experiment 7 which corresponds to MU50 cells treated with moxifloxacin, that both radiation treatments according to pattern A and G result in a decrease of the number of bacteria and that the treatment according to the pulse pattern A seems to be more effective than the treatment according to pattern G.

In contrast the control sample D in the presence of moxifloxacin without photo-activation shows an increase in the number of bacteria.

The growth curves of bacterial samples treated according to experiments 7 to 9 are shown in the graphs of Fig 3, Fig. 4 and Fig 5.

### Fig. 3:

Figure 3.1 to 3.10 show growth curves of bacterial samples treated according to experiment 7 and with Fig. 3.1 corresponding to sample 1, Fig. 3.2 corresponding to sample 2 etc. In the graphs the values of optical density at 595-600 nm are plotted on the y- axis against the time in hours on the x-axis. The triangles show treated samples according to pattern A (up triangles) and G (down triangles) and the lines without triangles is the control (D = no irradiation). The sample number 10 is the control sample without antibiotic (NaCl).

Conclusion: As can be seen at time t₄, the absorption in the samples with radiation treatment has significantly decreased compared to the control sample without radiation treatment for all of the antibiotics. Furthermore, before time t₂ before the antibiotic was added the bacterial cells were growing well in the exponential phase, and the addition of antibiotic at time t₂ did not affect the growth rate. Only after the exposure to electromagnetic radiation at time t₃, and only in the samples with antibiotic, the rate of growth decreased.

### Fig 4:

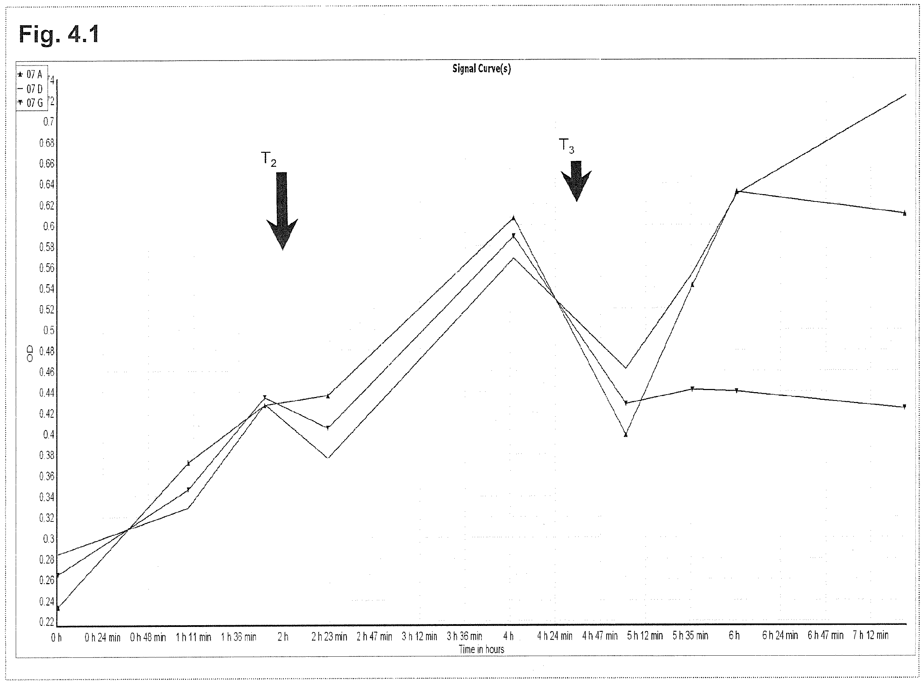

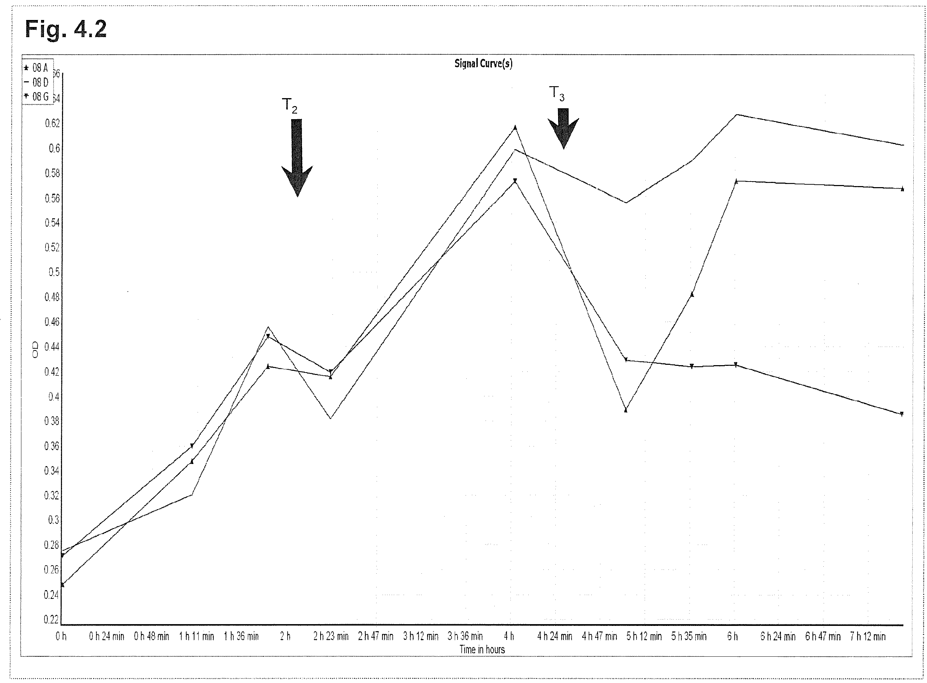

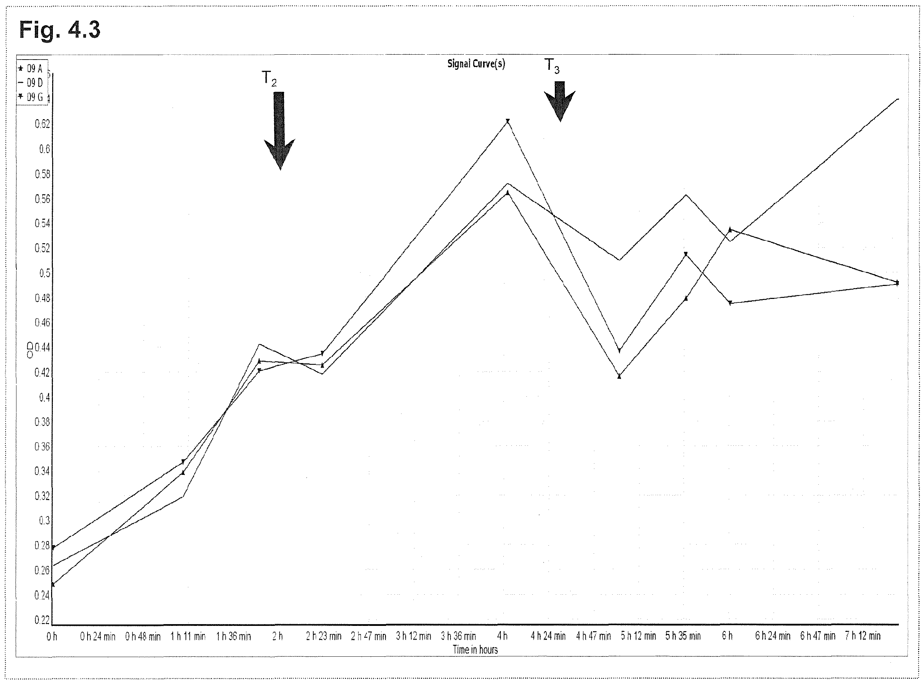

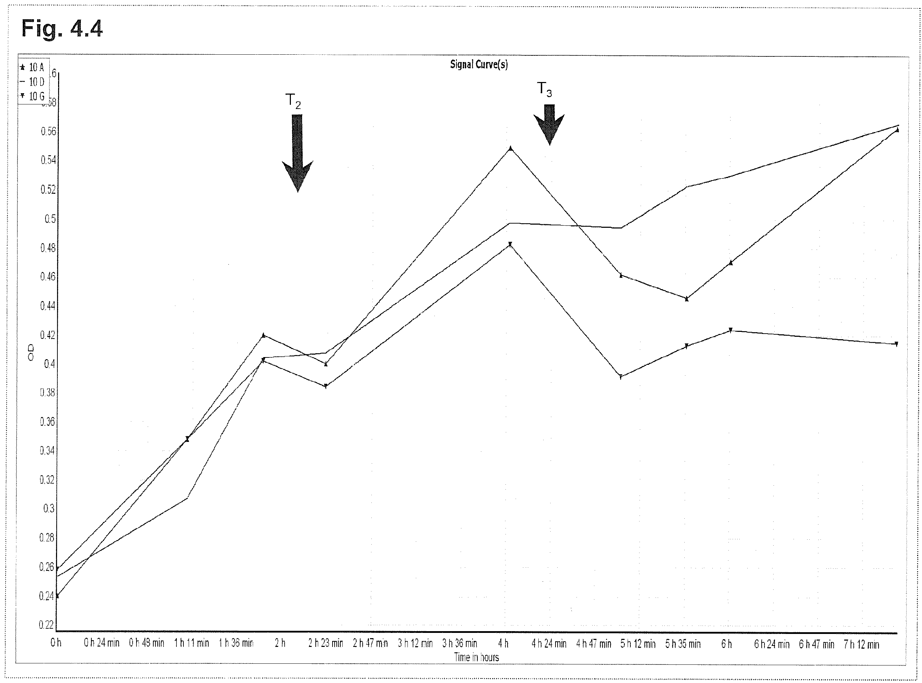

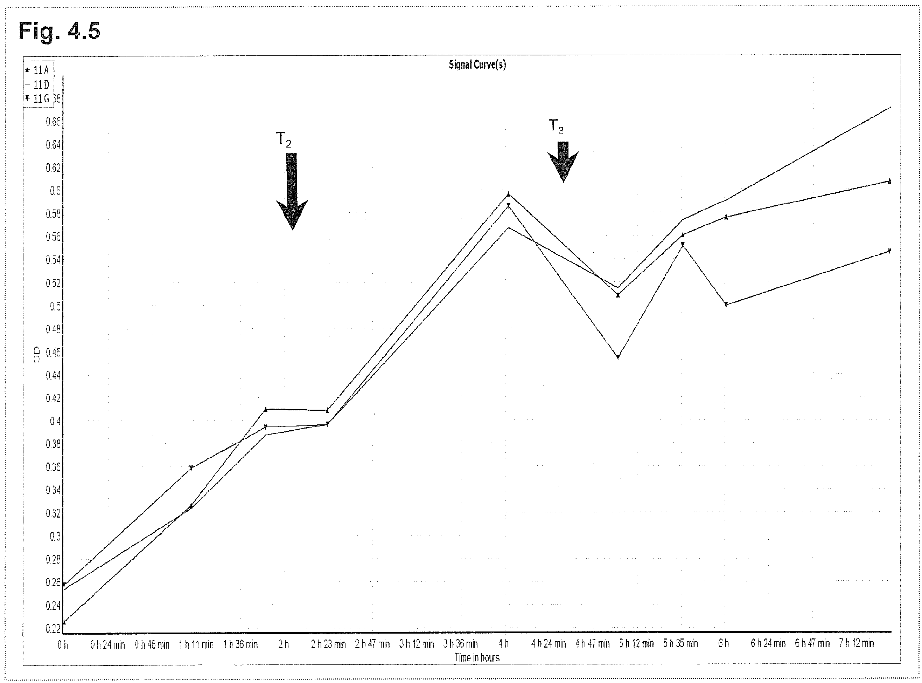

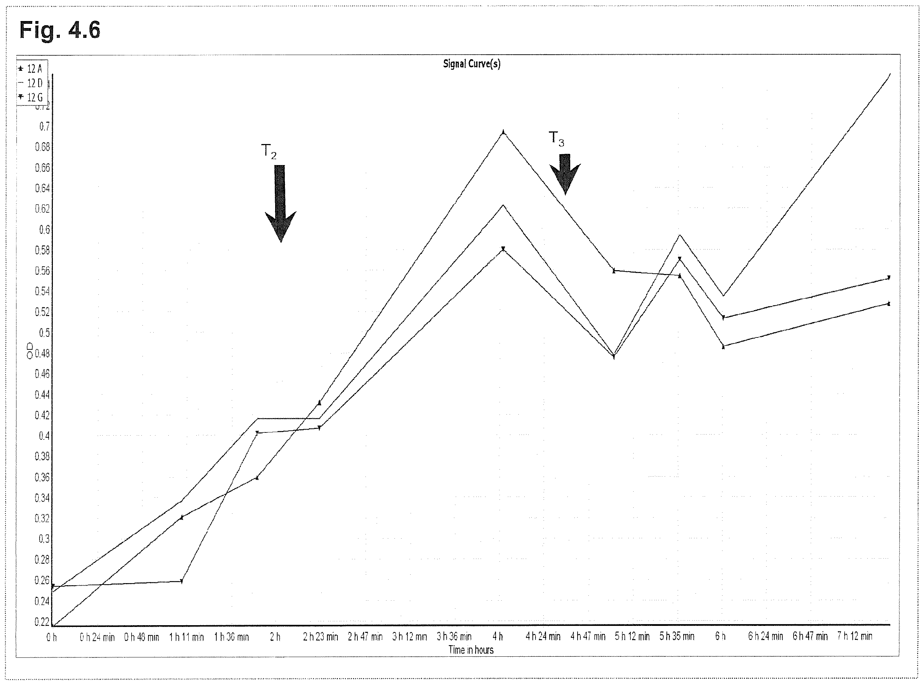

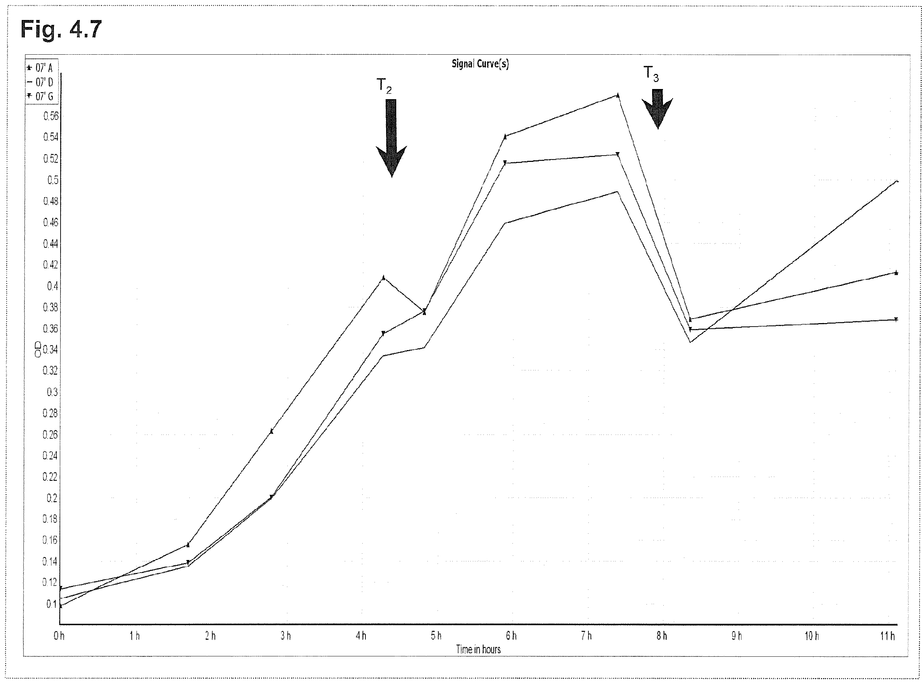

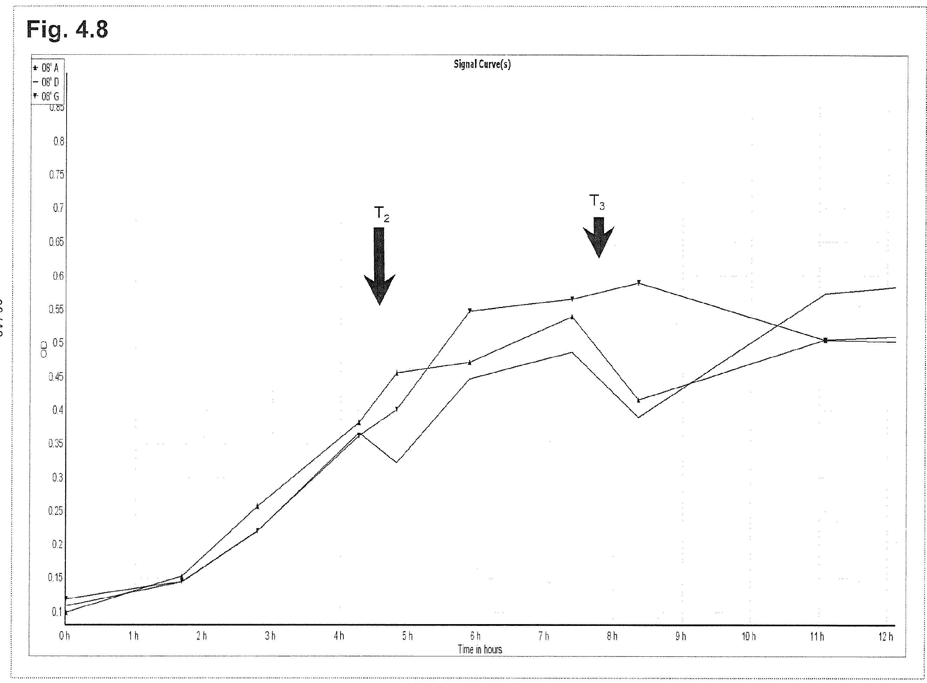

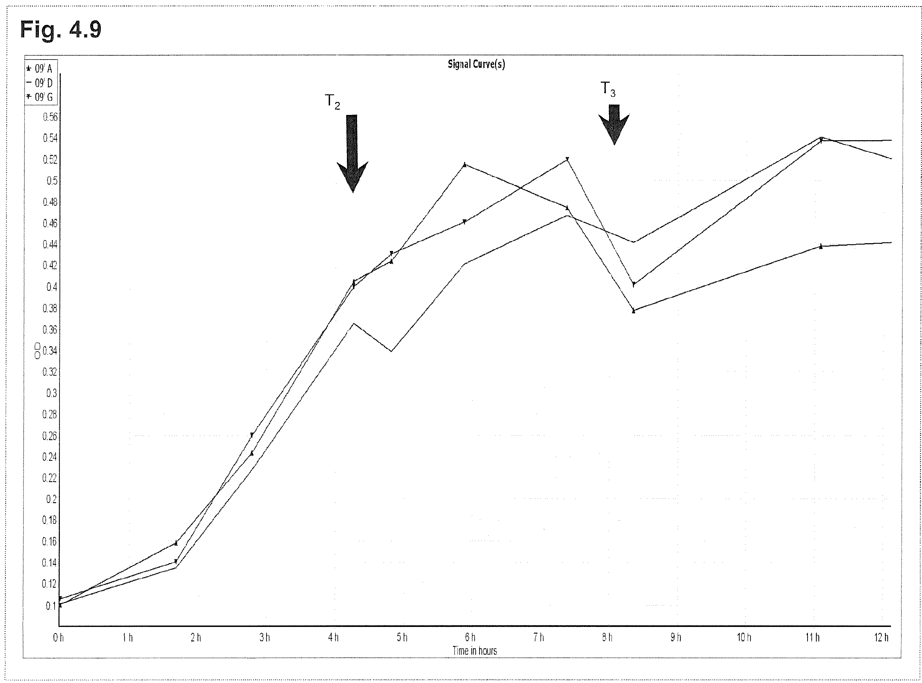

Figure 4.1 to 4.9 show growth curves of bacterial samples treated according to experiment 8 and with Fig. 4.1 corresponding to sample 1, Fig. 4.2 corresponding to sample 2 etc. In the graphs, the values of optical density at 595-600 nm are plotted on the y-axis against the time in hours on the x-axis. The triangles show treated samples according to pattern A (up triangles) and G (down triangles), and the line without triangles represents the control measurement (D = no irradiation). The sample number 6 is the control sample without antibiotic (NaCl). The same conclusions can be drawn as discussed above for Experiment 7, with the additional following interesting observation: In the sample number 6 (control) we can see the effect of a decreased growth rate. We can explain this phenomenon by the secretion of the toxin pyocyanin by Pseudomonas. After the treatment with electromagnetic radiation, even pseudomonas themselves become sensitive to their secreted toxin pyocyanin, which has a structure with delocalized electrons and therefore is capable of being photo-activated by the exposure to electromagnetic radiation.

### Structural formula of pyocyanin:

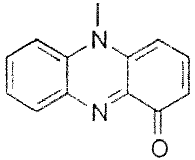

### Fig 5:

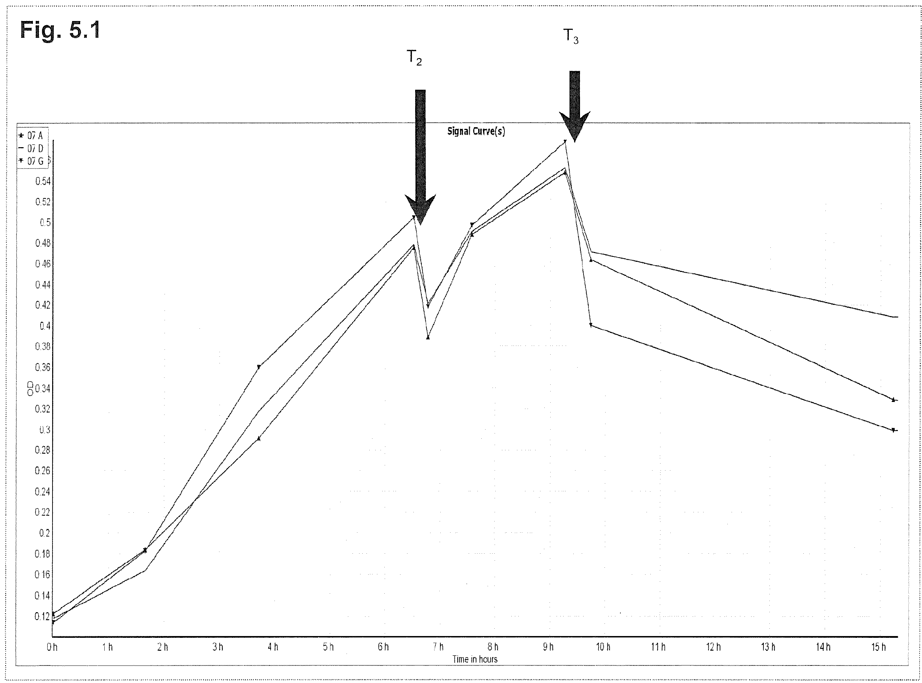

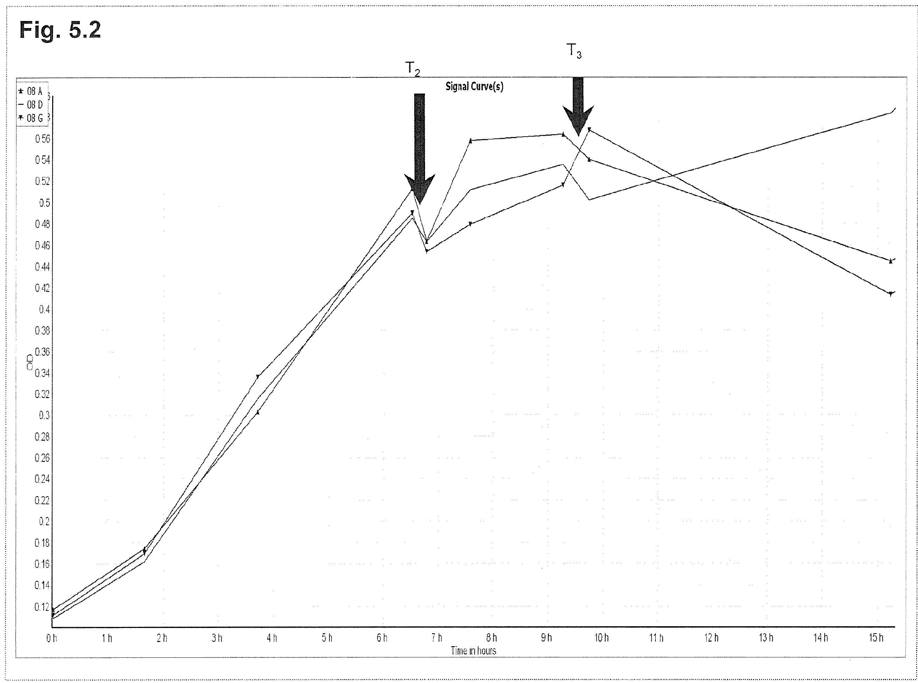

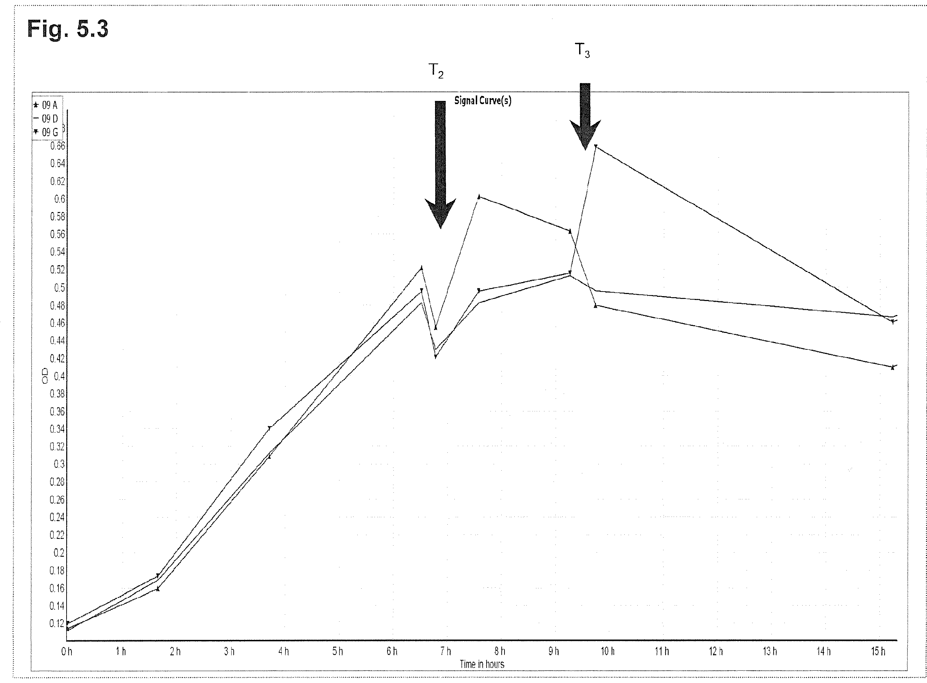

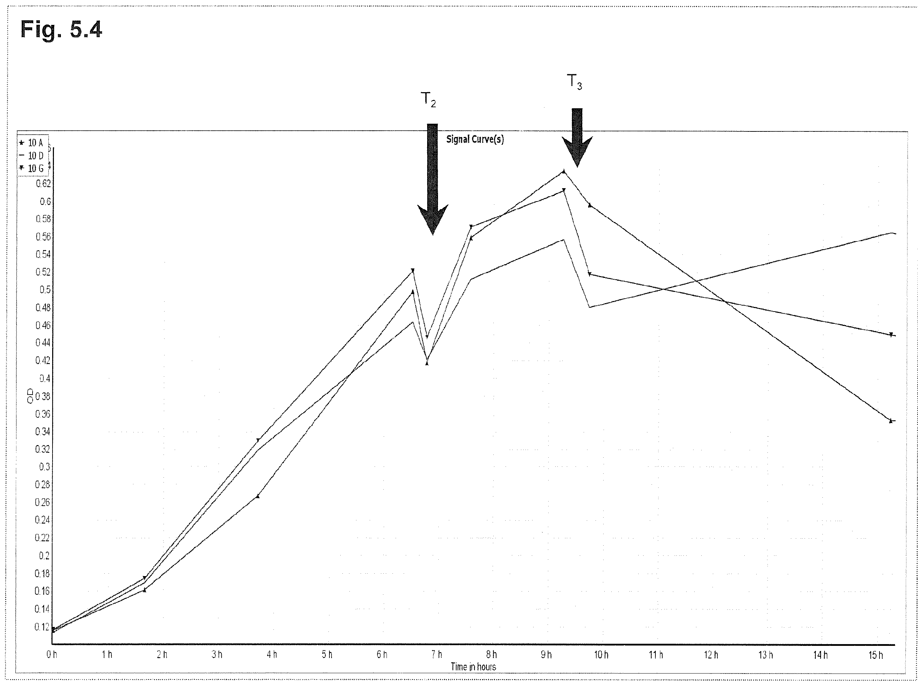

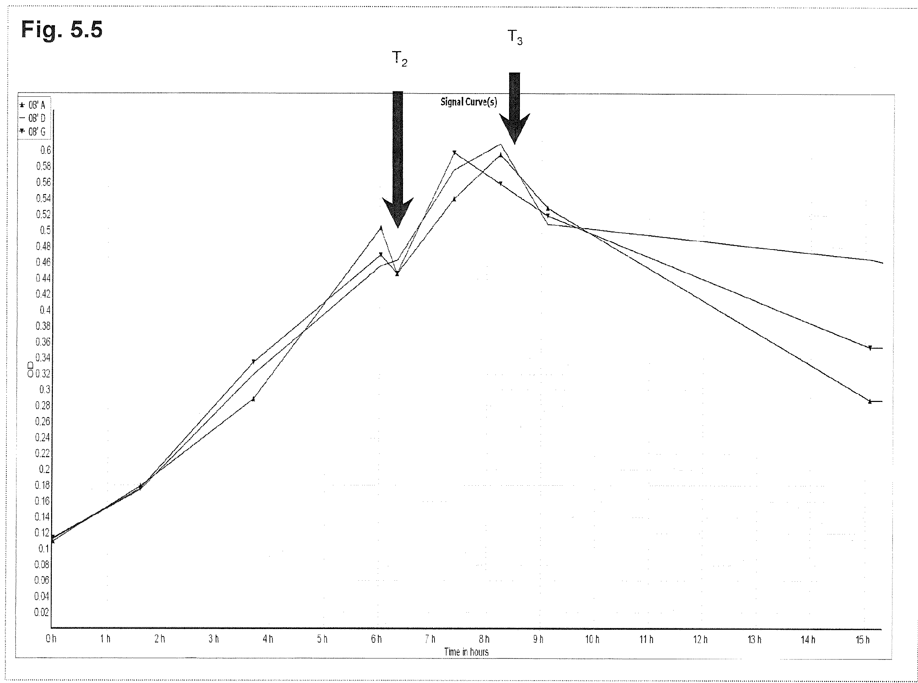

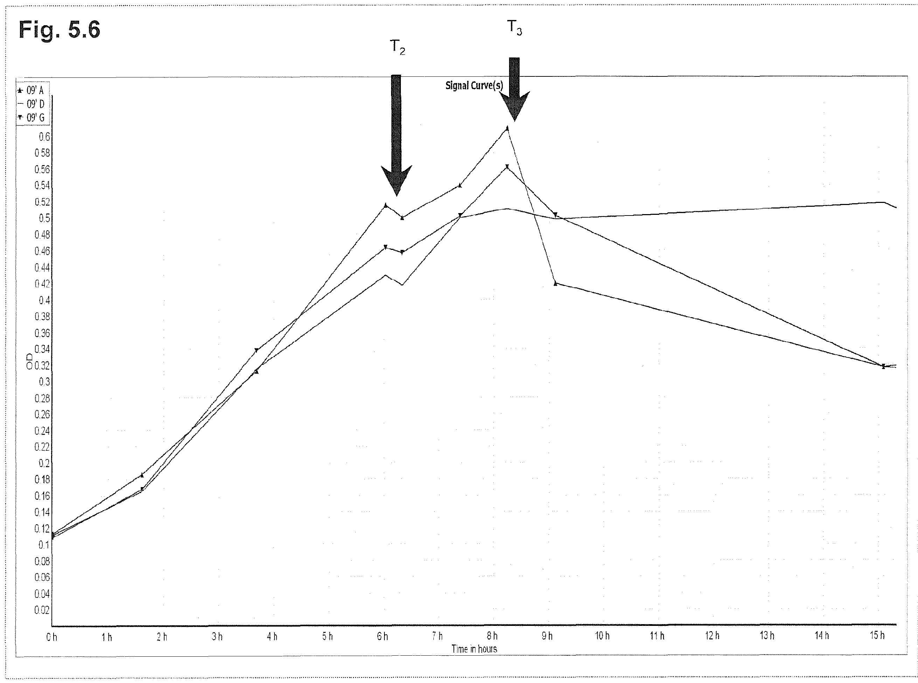

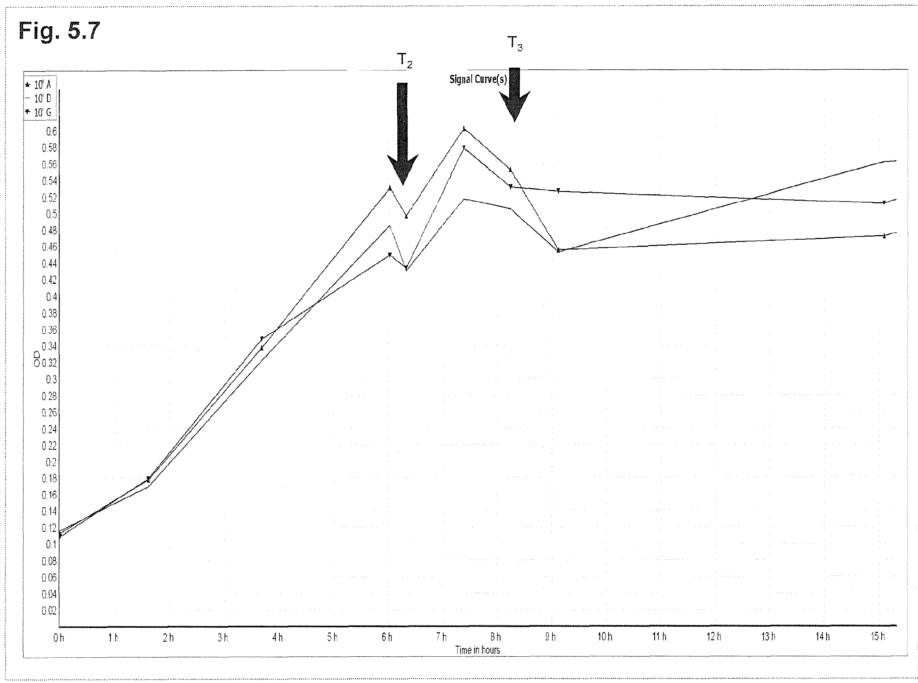

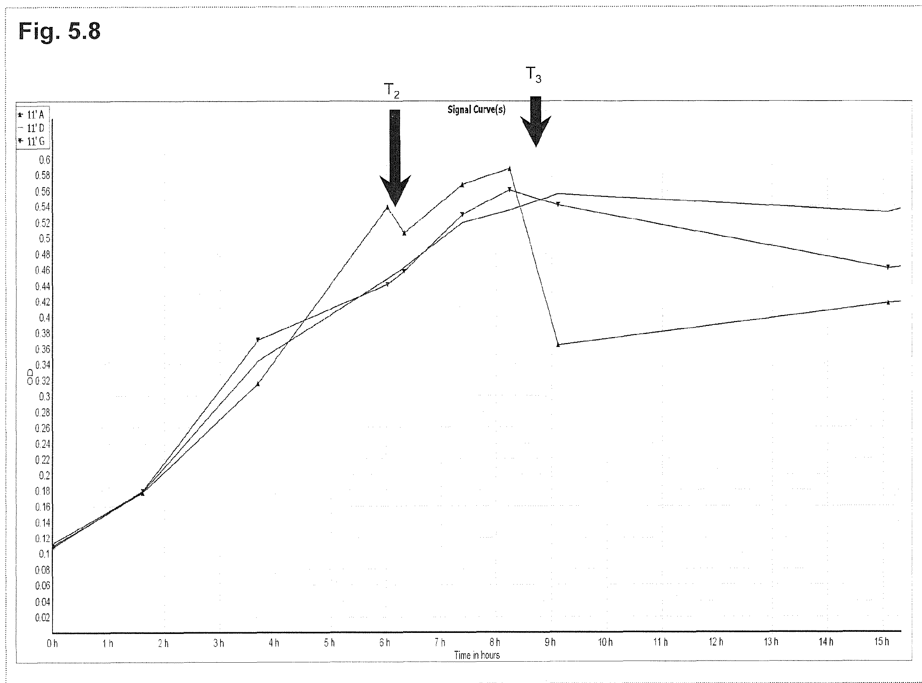

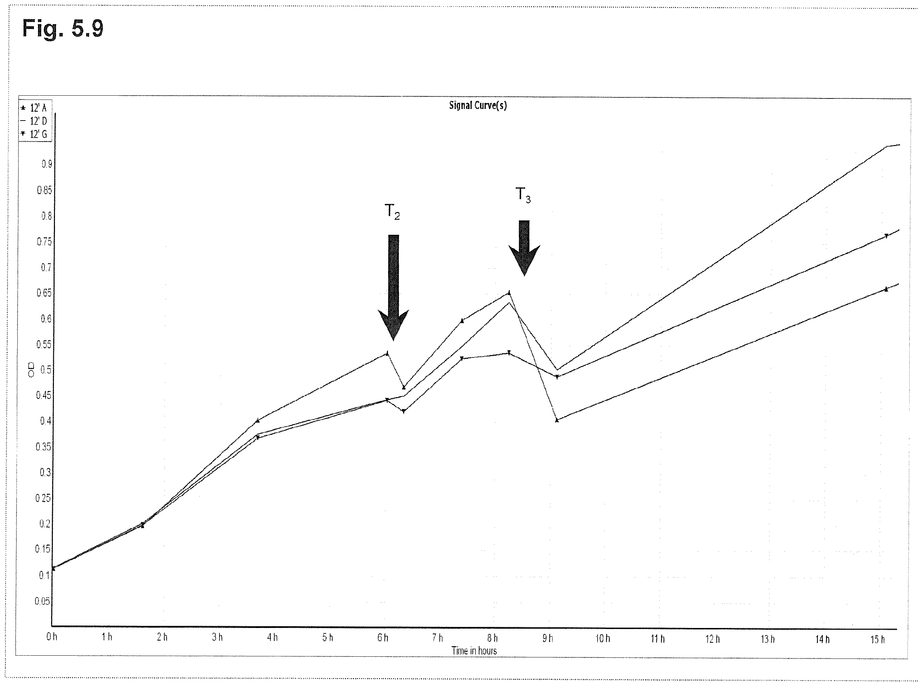

Figure 5.1 to 5.9 show growth curves of bacterial samples treated according toexperiment 9 and with Fig. 5.1 corresponding to sample 1, Fig. 5.2 corresponding to sample 2 etc. In the graphs, the values of optical density at 595-600 nm are plotted on the y-axis against the time in hours on the x-axis. The triangles show treated samples according to pattern A (up triangles) and G (down triangles), and the line without triangles represents the control measurement (D = no irradiation). The sample number 9 is the control without antibiotic (NaCl). The same conclusions can be drawn as discussed above for Experiment 8, including the pyocyanin effect.

### Experiment 10

In experiment 10, the effect of some exemplary photo-activated oncological (antineoplastic) drugs was tested in vitro as active components of the method for killing or reducing the proliferation of chemotherapy resistant neoplastic cells, namely stage 4 (metastatic) small cells lung carcinoma cells:
The following treatments A to F were applied to the samples:
A → Pulse ON 10ms, energy 0.1 J, OFF 90ms during 10 minutes
B → Pulse ON 10ms, energy 0.01-0.001 J, OFF 90ms during 10 minutes
C → Control no Pulse
D → Control no Pulse
E → Pulse ON 10ms energy 0.01-0.001 J, OFF 90ms during 5 minutes
F → Pulse ON 10ms, energy 0.1 J, OFF 90ms during 5 minutes

The neoplastic test cells were grown in RPMI medium without RED phenol + 20% fetal bovine serum

Oncological drugs were added to the samples at concentrations according to the following table:

| Sample number | antibiotic | final concentration (µg/ml) |
|---|---|---|
| 1 | Doxorubicin | 0.25 |
| 2 | Irinotecan | 3 |
| 3 | Topotecan | 0.14 |
| 4 | Topotecan | 0.07 |
| 5 | NaCl | NA |
| 6 | NaCl | NA |

The neoplastic cells are transferred to a well plate with the medium and the chemotherapeutic drug. The experiment was performed for each sample in six well plates, and all the samples 1 to 6 were treated with the two different times and two different intensities including two controls.

The neoplastic cells in the well plate are exposed to a pulse of electromagnetic radiation with a 400nm LED following the protocol as described above. During the ten or five minutes of exposure to electromagnetic radiation the ON time is 10% of the total time of a cycle. 10% ON and 90% OFF is a pulse pattern which is generally useful for some embodiments of the method and the pharmaceuticals.

24 hours after the treatment, the cell metabolic activity where analyzed with luminescence (CellTiter-Glo Promega and analyze of the light generation with the FLUOstar Omega - BMG Labtech).

The results in % after normalization with the controls are shown in the table below.

The results of Experiment 10 are shown in the following table:

| | 1 | 2 | 3 | 4 | 5 | 6 |
|---|---|---|---|---|---|---|
| A | 4 | 6 | 9 | 7 | 32 | 34 |
| B | 17 | 43 | 69 | 52 | 70 | 75 |
| C | 100 | 100 | 100 | 100 | 100 | 100 |
| D | 100 | 100 | 100 | 100 | 100 | 100 |
| E | 46 | 40 | 74 | 47 | 73 | 77 |
| F | 4 | 5 | 6 | 5 | 30 | 32 |

### Conclusion:

The control results are on lines C and D normalizing the light generation in the absence of light treatment 100% (no light treatment → ATP=100%). The results of show that at 24 hours after the treatment, we can see a decrease of the metabolic activity of the neoplastic cells of down to 4 % (sample 1 with doxorubicin) (lines A and F). And even with a very low light power (lines B and E), a decrease of the metabolic activity up to 17% can be observed (sample 1 with doxorubicin).

### Experiment 11:

Experiment 11 was performed according to the same protocol as experiment 10, however, in Experiment 11 two cell lines which are capable of apoptosis were used:
- 3T3 cell (fibroblast)
- ARPE cell (epithelial cell)
grown in DMEM medium without RED phenol + 1% Fetal bovine serum (FBS). The addition of 1% of FBS put the cells in a resting state.

24 hours after the treatment according to the same protocol as above the cells were analyzed optically for the following criteria (density, morphology, apoptosis). The results (not shown) that the cells were affected by the toxicity of the neoplastic drugs, but there was no increased adverse effect on the cells after the exposure to electromagnetic radiation.

### Experiment 12:

15'000'000 multi-resistant bacteriae (0.050 ml of a McFarland of 1) were put on 40 mm diameter petri dishes. The petri dishes comprised MH agar with or without antibiotic.

An LED radiation source with a peak emission at 400 nm was used, energized by a Frequency generator (32.2 V, 2.6 A input power), in a pulsed mode with pulses of 0.02 ms duration and a pulse repetition frequency of 1 kHz. The LED power during 0.02 ms was measured to be 0.63 W on a Thorlabs power sensor with a surface area of 2.2 x 2.2 mm representing a power of 13 mW/cm².

The concentration of all antibiotics was 10 mg/l.

Irradiation took place for a total time of 10 minutes.

After the irradiation, the bacteria were left for 24 hours for incubation, and then number of colony forming units was determined.
- If a significant number (of more than 1'000) of colony forming units could be found, this was taken as indication that the bacteria could replicate.
- If, however, less than 100 (can be countable) were found, the bacteriae were assumed to be not capable of replicating (99.999% of killing).
- If a value between 100-1000 (no important bacterial colony overlapping) was found, the bacteriae were assumed to be capable of partially replicating (99.99 % of killing).

| | A | B | C | D | E | F |
|---|---|---|---|---|---|---|
| 1 | R | PR | R | R | R | R |
| 2 | R | NR | R | NR | R | NR |
| 3 | R | NR | R | NR | R | PR |
| 4 | R | NR | R | NR | R | PR |
| 5 | R | NR | R | NR | R | NR |
| 6 | R | NR | R | NR | R | PR |
| 7 | R | NR | R | R | R | R |
| 8 | R | PR | R | R | R | R |
| 9 | R | NR | R | NR | R | NR |
| 10 | R | NR | R | NR | R | NR |
| 11 | R | NR | R | NR | R | NR |
| 12 | R | NR | R | NR | R | NR |
| 13 | R | NR | R | NR | R | PR |
| 14 | R | NR | R | NR | R | NR |
| 15 | R | NR | R | NR | R | NR |

| | | | | | | |
|---|---|---|---|---|---|---|
| A: Pseudomonas aeruginosa (no irradiation) B: Pseudomonas aeruginosa (irradiation) C: Acinetobacter (no irradiation) D Acinetobacter (irradiation) E: Staphylococcus aureus MU500 (no irradiation) F: Staphylococcus aureus MU500 (irradiation) 1: MH Agar (no antibiotic; control) 2: Moxifloxacin 3: Levofloxacin 4: Ofloxacin 5: Ciprofloxacin 6: Norfloxacin 7: Tobramycin 8: Clarithromycin 9: Tetracycline 10: Doxycycline 11: Minocycline 12: Lymecycline 13: Gatifloxacin (currently partially withdrawn from the market; not for ophthalmologic drops) 14: Oxytetracycline 15: Tigecycline | | | | | | |

The values signify: R: Bacteria can replicate after the treatment; NR: No replication; PR: Bacteria can replicate partially.

With the exception of Tobramycin (which does not absorb the radiation and for which substance no effect is to be expected) and Clarithromycin (for Staphylococcus aureus), the results thus show that the resistant Acinetobacter and Staphylococcus aureus bacteria could be prevented from replicating by the regimen and method according to the invention.

For the Pseudomonas aeruginosa samples the irradiation was observed to be effective independent of the antibiotics, also for Tobramycin (that does not exhibit any substantial absorption) and even in the test group 1 with no antibiotics. The prevention of replication, as explained above, has to be attributed to the presence of an antibiotics (pyocyanin) secreted by pseudomonas (the high toxicity of pyocyanin on pseudomonas when photoactivated with pulse is explained by the invention; pseudomonas secrete pyocyanine for killing the other bacteria but Pseudo is resistant to pyocyanine by the same process as the antibiotics resistances)

### Experiment 13:

After the irradiation treatment according to experiment 12, remaining bacteria were allowed to proliferate and then were subject to the treatment according to experiment 12 again in order to test for possible new resistances to the treatment. The values in the following table are designated as in experiment 12:

| | C | D | E | F |
|---|---|---|---|---|
| 1 | R | R | R | R |
| 2 | R | NR | R | PR |
| 5 | R | NR | R | NR |
| 9 | R | NR | R | NR |
| 10 | R | NR | R | NR |

The results for Levofloxacin, Ciprofloxacin, Doxycycline and Tetracycline on the Acinetobacter and Staphylococcus bacteria show that no resistance to the treatment of the second generation resistant bacteria could be observed - the bacteria could still be prevented from replicating.

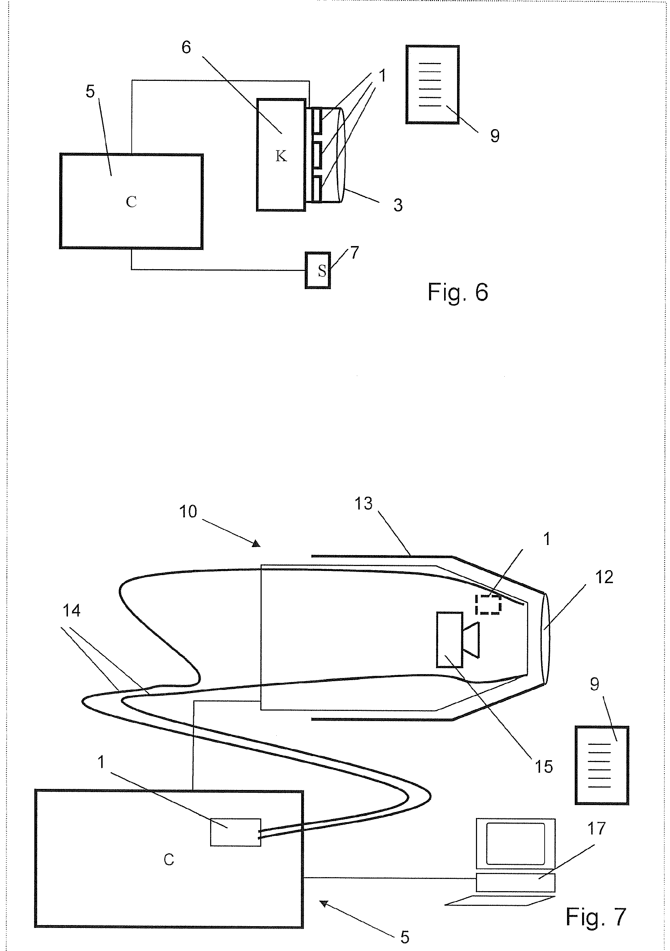

An example of a medical device is shown in **Figure 6**. The device comprises a plurality of radiation sources 1 being LED chips. The device is capable of irradiating for example via an optical system (for example a lens or lens system) 3, tissue that has previously been treated by a pharmaceutical component, for example superficially or by ingestion or both. The radiation sources 1 are mounted on a cooler 6. A control unit 5 controls energizes and controls the radiation sources.

The device further comprises a temperature sensor 7, for example an IR sensor, controlling the temperature of tissue that is irradiated or of tissue that is immediately adjacent an irradiated area. The control unit 5 is connected to the sensor 7 and may be programmed to ascertain that the temperature does not rise above a certain pre-defined or programmable limit, for example of around 40°C. If this temperature is reached, the device may issue a warning and/or switch the radiation source(s) off or reduce their power.

The device further comprises information 9 on the interplay between the radiation and the active component. The information may be written on a leaflet or user manual, or it may be stored in the device's electronics or an electronic manual etc. The information comprises information on which active component is suitable for the radiation produced by the device and absorbs it.
- In accordance with a first possibility, the spectral composition of radiation produced by the radiation source(s) may be fix, and the information may comprise indication of a pre-defined substance or list of substances that are suitable as the active component.
- In accordance with a second possibility, the medical device may comprise a wavelength tunable output. For example, the medical device may comprise different radiation sources with different output spectra, which different radiation sources can be controlled individually. Additionally or alternatively, the radiation sources may themselves be tunable. In accordance with this second possibility, the control unit may make possible that the output spectrum is selected in dependency of the chosen active component. For example, the medical device may comprise a first setting, together with a list of substances that absorb radiation that is produced when the device is operated in the first setting, and a second setting together with a second according list. Or the device may comprise a display (or can for example be remotely controlled via device with a display) on which the active component may be selected, and the control selects an according output.

In the variant of **Figure 7****,** the device is specifically made for treatment of the human eye. It comprises an applicator head 10 that can be brought in immediate vicinity of the eye. In accordance with a possibility, the applicator head 10 does not itself contain the radiation source(s) 1, but the radiation sources are connected to at least one (two in the shown configuration) fiber optic cable 14 the endings of which are at or near the distal end of the applicator head. The fiber optic cables 14 guide radiation emitted by the at least one radiation source 1 to the applicator head and direct it, for example via an optical system 12, onto the cornea when the applicator head is placed. The radiation emitting element(s) may especially be arranged in the control unit 5.

The embodiment of Fig. 7 further shows a disposable outer cover 13 (or 'single use tip') that can be attached to the applicator head casing to protect the cornea from non-sterile components. The outer cover 13 is transparent for visible and near-UV radiation at least in the region towards the distal end and in the depicted embodiment comprises the optional system in the form of a lens 12.

Within the housing the applicator comprises a camera 15 that is placed to capture images (continuously or triggered by certain events) of the cornea. The camera may in embodiments comprise a filter selecting fluorescent radiation emitted by Riboflavin. By an automated analysis or manually, by an operator, one may make sure that the cornea comprises sufficient Riboflavin so that the radiation - that in this embodiment may be UVA radiation, with wavelengths around 360 nm - that would be toxic for the retina or other issue - is sufficiently absorbed and is effective only at the surface of the for example infected (and treated by antibiotics) cornea.

In Fig. 7, as well as in the other figures, the connections between the control unit 5 and the applicator head 10 as well as between the control unit 5 and a computer 17 are depicted only schematically; the skilled person will realize that the connection may include electrical or possibly optical connections for both, power supply and control of the components in the applicator head as well as for the data transmission from the camera 15 and/or other optional sensors to the control unit 5 and ultimately (if present) to the computer 17.

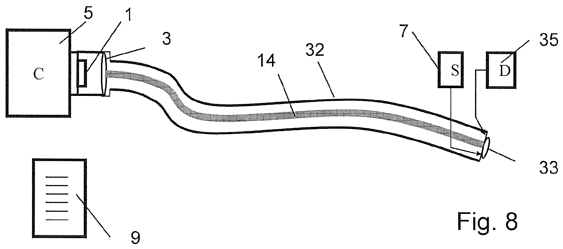

The device of **Figure 8** is especially suited for application of the radiation within the patient's body. It comprises a catheter 35 that can be introduced via an appropriate channel in the human body, for example a vein, the esophagus and/or gastrointestinal tract, the urethra, the vagina/uterus, the trachea, etc. The catheter may comprise steering means for steering within the body. Catheter systems, for example for minimally invasive surgery, are known in the art and will not be described in this text.

The radiation source arrangement comprises an optical fiber or system or optical fibers 14 for guiding the radiation from the radiation source 1 through the catheter to the desired position. A sensor 7 may be present locally at the distal end of the catheter, or it may be arranged in the control unit 5 and connected by the fiber optic system to the distal end.

In addition to the radiation source arrangement, the device may comprise a dosing mechanism 35 that allows to locally dispense the pharmaceutical composition. To this end, the catheter itself may comprise, for example close to the distal end, a compartment for the pharmaceutical composition, and a mechanism for dispensing as soon as the catheter has reached its desired destination. In addition or as an alternative, the catheter may comprise a lumen through which the pharmaceutical composition is applied from the exterior.

The method using this device comprises the steps of applying a dose of the pharmaceutical composition, for example for an oncological treatment, and subsequently locally irradiating with the radiation that is absorbed by the active component of the pharmaceutical composition. The device according to any exemplary embodiment may come as part of a kit that further may comprise at least one of:
- Replacement equipment, such as sterile replacement covers etc.
- Protection equipment protecting the operator. Such protection equipment may especially include protection glasses with a filter absorbing radiation of a spectral composition corresponding to the spectral composition of the radiation generated by the radiation source.
- At least one dose of the pharmaceutical composition.

## Claims

1. Pharmaceutical composition comprising an active component which kills or retards proliferation of target cells, wherein the target cells had previously acquired a resistance to the active component, and wherein the active component absorbs electromagnetic radiation of a wavelength above a lower limit of 190 nm, for use in a method for the preventive or therapeutic treatment of infectious diseases or tumors of a human or non-human individual, **characterized in that** the pharmaceutical composition is administered with a regimen comprising during or after administration of the pharmaceutical composition at least one exposure of the individual to electromagnetic radiation with a wavelength or a range of wavelength some of which is absorbed by the active component, wherein the electromagnetic radiation is applied in short pulses in a range of 0.1 fs to 200 ms.

2. Method of applying a composition to an object not being a human or animal body, wherein the composition comprises an active component which kills or retards proliferation of target cells wherein the target cells had previously acquired a resistance to the active component, and wherein the active component absorbs electromagnetic radiation of a wavelength above a lower limit of 190 nm, wherein after or concomitant with the application or administration of the composition to the object, the surface area is exposed at least once to electromagnetic radiation of a wavelength or a range of wavelengths at least some of which is absorbed by the active component, wherein the electromagnetic radiation is applied in short pulses in a range of 0.1 fs to 200 ms.

3. Pharmaceutical composition according to claim 1 or method according to claim 2, wherein the target cells had previously acquired a resistance to the active component by a pump preventing or reducing the accumulation of the active component in the target cells or by an enzyme degrading the active component or in particular by decreasing the affinity of the targeted molecule of the target cell.

4. Pharmaceutical composition according to claim 1 or 3 or method according to one of claims 2 or 3, wherein the target cells are of one or more pathogen including bacterial, fungal, parasitical, and infected cells or wherein the target cells are tumor, in particular cancer cells.

5. Pharmaceutical composition according to any one of claims 1, 3 or 4 or method according to one of claims 2 to 4, wherein the electromagnetic radiation has a range of wavelength with a lower limit of 193 nm, or of 200 nm, or of 240 nm, or of 280 nm, or of 350 nm or of 365 nm.

6. Pharmaceutical composition according to any one of claims 1 or 3 to 5 or method according to anyone of claims 2 to 5 wherein the electromagnetic radiation has a range of a wavelength with an upper limit of 800 nm, of 700nm, of 600 nm of 500 nm or of 450 nm.

7. Pharmaceutical composition according to anyone of claims 1 or 3 to 6 or method according to anyone of claims 2 to 6 wherein the electromagnetic radiation has a wavelength between 200 nm and 500 nm or in particular in a range with a lower limit between 200 nm and 240 nm or between 200 nm and 280 nm and with upper limit between 450 nm and 500 nm or more particularly with a lower limit of approx. 350 nm or 365 nm or 370 nm and an upper limit of approx. 450 nm.

8. Pharmaceutical composition according to anyone of claims 1 or 3 to 7, wherein the temperature of the tissue exposed to electromagnetic radiation does not increase by more than 2 or 3 or 4 or 5 degrees Celsius and does not increase beyond 40°C in human individuals.

9. Pharmaceutical composition according to anyone of claims 1 or 3 to 8, wherein the dosage of the active component is lowered by a factor of two up to 10 or up to 100 or up to 1000 compared to the regularly prescribed dosage in the absence of a treatment with electromagnetic radiation.

10. Pharmaceutical composition according to anyone of claims 1 or 3 to 9 or method according to anyone of claims 2 to 8, wherein the active component of the composition comprises or consists of a quinolone according to Formula I 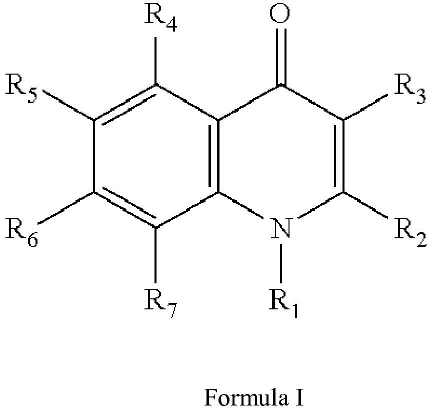 or according to Formula II: 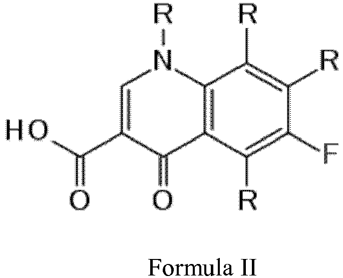 or wherein the active component comprises or consists of a derivative of polycyclic naphthacene carboxamide according to Formula III or IV, in particular an active component belonging to the group of tetracyclines: 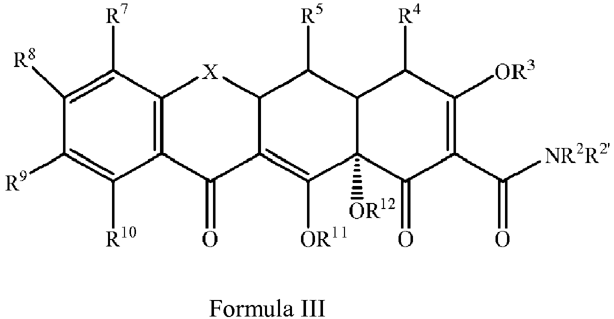 or

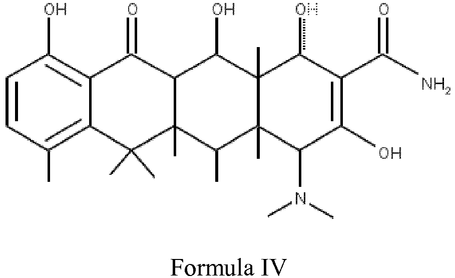

11. Pharmaceutical composition according to anyone of claims 1 or 3 to 10 for the treatment of an eye of a patient, wherein the pharmaceutical composition is administered to the eye, wherein the active component is an antibacterial substance, and wherein the target cells had previously acquired a resistance to the active component.

## Patentansprüche

1. Pharmazeutische Zusammensetzung, umfassend eine Wirkkomponente, die Zielzellen abtötet oder deren Proliferation verzögert, wobei die Zielzellen zuvor eine Resistenz gegen die Wirkkomponente erworben hatten und wobei die Wirkkomponente elektromagnetische Strahlung einer Wellenlänge oberhalb einer Untergrenze von 190 nm absorbiert, zur Verwendung bei einem Verfahren zur vorbeugenden oder therapeutischen Behandlung von Infektionskrankheiten oder Tumoren bei einem menschlichen oder nichtmenschlichen Individuum, **dadurch gekennzeichnet, dass** die pharmazeutische Zusammensetzung innerhalb eines Schemas verabreicht wird, das während oder nach Verabreichung der pharmazeutischen Zusammensetzung wenigstens eine Exposition des Individuums mit einer elektromagnetischen Strahlung mit einer Wellenlänge bzw. einem Wellenlängenbereich umfasst, wovon einiges von der Wirkkomponente absorbiert wird, wobei die elektromagnetische Strahlung in Kurzimpulsen in einem Bereich von 0,1 fs bis 200 ms angewandt wird.

2. Verfahren zur Anwendung einer Zusammensetzung auf einen Gegenstand, bei dem es sich nicht um einen menschlichen oder tierischen Körper handelt, wobei die Zusammensetzung eine Wirkkomponente umfasst, die Zielzellen abtötet oder deren Proliferation verzögert, wobei die Zielzellen zuvor eine Resistenz gegen die Wirkkomponente erworben hatten und wobei die Wirkkomponente elektromagnetische Strahlung einer Wellenlänge oberhalb einer Untergrenze von 190 nm absorbiert, wobei nach oder gleichzeitig mit der Anwendung oder Verabreichung der Zusammensetzung auf bzw. an den Gegenstand die Oberfläche wenigstens einmal elektromagnetischer Strahlung einer Wellenlänge bzw. eines Wellenlängenbereichs ausgesetzt wird, wovon einiges von der Wirkkomponente absorbiert wird, wobei die elektromagnetische Strahlung in Kurzimpulsen in einem Bereich von 0,1 fs bis 200 ms angewandt wird.

3. Pharmazeutische Zusammensetzung nach Anspruch 1 oder Verfahren nach Anspruch 2, wobei die Zielzellen zuvor eine Resistenz gegen die Wirkkomponente erworben hatten, indem eine Pumpe die Ansammlung der Wirkkomponente in den Zielzellen verhinderte oder verringerte oder indem ein Enzym die Wirkkomponente abbaute oder insbesondere indem die Affinität des anvisierten Moleküls der Zielzelle gesenkt wurde.

4. Pharmazeutische Zusammensetzung nach Anspruch 1 oder 3 oder Verfahren nach einem der Ansprüche 2 oder 3, wobei die Zielzellen von einem oder mehreren Krankheitserregern stammen, einschließlich Bakterien-, Pilz-, Parasitenzellen und infizierten Zellen, oder wobei es sich bei den Zielzellen um Tumor-, insbesondere Krebszellen handelt.

5. Pharmazeutische Zusammensetzung nach einem der Ansprüche 1, 3 oder 4 oder Verfahren nach einem der Ansprüche 2 bis 4, wobei die elektromagnetische Strahlung einen Wellenlängenbereich mit einer Untergrenze von 193 nm oder von 200 nm oder von 240 nm oder von 280 nm oder von 350 nm oder von 365 nm aufweist.

6. Pharmazeutische Zusammensetzung nach einem der Ansprüche 1 oder 3 bis 5 oder Verfahren nach einem der Ansprüche 2 bis 5, wobei die elektromagnetische Strahlung einen Wellenlängenbereich mit einer Obergrenze von 800 nm, von 700 nm, von 600 nm, von 500 nm oder von 450 nm aufweist.

7. Pharmazeutische Zusammensetzung nach einem der Ansprüche 1 oder 3 bis 6 oder Verfahren nach einem der Ansprüche 2 bis 6, wobei die elektromagnetische Strahlung eine Wellenlänge zwischen 200 nm und 500 nm oder insbesondere in einem Bereich mit einer Untergrenze zwischen 200 nm und 240 nm oder zwischen 200 nm und 280 nm und mit einer Obergrenze zwischen 450 nm und 500 nm oder ganz besonders mit einer Untergrenze von ca. 350 nm oder 365 nm oder 370 nm und einer Obergrenze von ca. 450 nm aufweist.

8. Pharmazeutische Zusammensetzung nach einem der Ansprüche 1 oder 3 bis 7, wobei die Temperatur des Gewebes, das elektromagnetischer Strahlung ausgesetzt ist, nicht um mehr als 2 oder 3 oder 4 oder 5 Grad Celsius und bei menschlichen Individuen nicht auf über 40°C steigt.

9. Pharmazeutische Zusammensetzung nach einem der Ansprüche 1 oder 3 bis 8, wobei die Dosierung der Wirkkomponente um einen Faktor zwei bis 10 oder bis 100 oder bis 1000 im Vergleich zur regulär verschriebenen Dosierung ohne eine Behandlung mit elektromagnetischer Strahlung herabgesetzt ist.

10. Pharmazeutische Zusammensetzung nach einem der Ansprüche 1 oder 3 bis 9 oder Verfahren nach einem der Ansprüche 2 bis 8, wobei die Wirkkomponente der Zusammensetzung ein Chinolon gemäß der Formel I 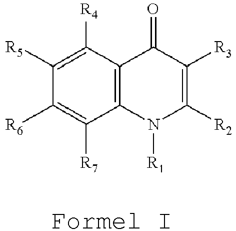 oder gemäß der Formel II 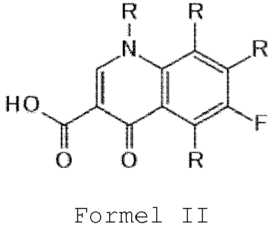 umfasst bzw. daraus besteht oder wobei die Wirkkomponente ein Derivat von polycyclischem Naphthacencarboxamid gemäß der Formel III oder IV umfasst bzw. daraus besteht, insbesondere eine Wirkkomponente, die zur Gruppe der Tetracycline gehört: 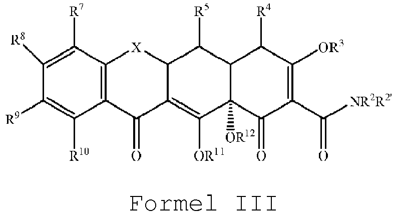 oder

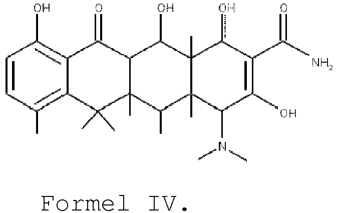

11. Pharmazeutische Zusammensetzung nach einem der Ansprüche 1 oder 3 bis 10 zur Behandlung eines Auges eines Patienten, wobei die pharmazeutische Zusammensetzung an das Auge verabreicht wird, wobei es sich bei der Wirkkomponente um einen antibakteriellen Stoff handelt und wobei die Zielzellen zuvor eine Resistenz gegen die Wirkkomponente erworben hatten.

## Revendications

1. Composition pharmaceutique comprenant un composant actif qui tue ou ralentit la prolifération de cellules cibles,
les cellules cibles ayant antérieurement acquis une résistance au composant actif et le composant actif absorbant le rayonnement électromagnétique dont la longueur d'onde est supérieure à une limite inférieure de 190 nm,
la composition étant destinée à être utilisée dans un procédé de traitement préventif ou thérapeutique de maladies infectieuses ou de tumeurs d'un individu humain ou non humain,
**caractérisée en ce que**
la composition pharmaceutique est administrée en un régime qui comprend pendant ou après l'administration de la composition pharmaceutique au moins une exposition de l'individu à une rayonnement électromagnétique dont la longueur d'onde ou une plage de longueurs d'onde dont certaines sont absorbées par le composant actif, le rayonnement électromagnétique étant appliqué en courtes pulsations comprises dans la plage de 0,1 fs à 200 ms.

2. Procédé d'application d'une composition sur un objet qui n'est pas un corps humain ou animal, la composition comprenant un composant actif qui tue ou ralentit la prolifération de cellules cibles,
les cellules cibles ayant antérieurement acquis une résistance au composant actif et le composant actif absorbant le rayonnement électromagnétique dont la longueur d'onde est supérieure à une limite inférieure de 190 nm,
dans lequel après ou en même temps que l'application ou l'administration de la composition à l'objet, la superficie est exposée au moins une fois à un rayonnement électromagnétique dont la longueur d'onde ou la place de longueurs d'onde dont certaines sont absorbées par le composant actif, le rayonnement électromagnétique étant appliqué en courtes pulsations comprises dans la plage de 0,1 fs à 200 ms.

3. Composition pharmaceutique selon la revendication 1 ou procédé selon la revendication 2, dans lesquels les cellules cibles ont antérieurement acquis une résistance au composant actif par une pompe qui empêche ou réduit l'accumulation du composant actif dans les cellules cibles ou par une enzyme qui dégrade de composant actif, ou en particulier en diminuant l'affinité de la molécule de la cellule cible.

4. Composition pharmaceutique selon les revendications 1 ou 3 ou procédé selon les revendications 2 ou 3, dans lesquels les cellules cibles sont un ou plusieurs pathogènes, notamment des cellules bactériennes, fongiques, parasites ou infectées ou dans lesquels les cellules cibles sont des cellules de tumeur et en particulier des cellules cancéreuses.

5. Composition pharmaceutique selon l'une quelconque des revendications 1, 3 ou 4 ou procédé selon l'une quelconque des revendications 2 à 4, dans lesquels le rayonnement électromagnétique a une plage de longueurs d'onde dont la limite inférieure est de 193 nm, 200 nm, 240 nm, 280 nm, 350 nm ou 365 nm.

6. Composition pharmaceutique selon l'une quelconque des revendications 1 ou 3 à 5 ou procédé selon l'une quelconque des revendications 2 à 5, dans lesquels le rayonnement électromagnétique a une plage de longueurs d'onde dont la limite supérieure est de 800 nm, 700 nm, 600 nm, 500 nm ou 450 nm.

7. Composition pharmaceutique selon l'une quelconque des revendications 1 ou 3 à 6 ou procédé selon l'une quelconque des revendications 2 à 6, dans lesquels le rayonnement électromagnétique a une longueur d'onde comprise entre 200 nm et 500 nm ou en particulier une plage dont la limite inférieure est comprise entre 200 nm et 240 nm ou entre 200 nm et 280 nm et dont la limite supérieure est comprise entre 450 nm et 500 nm ou plus particulièrement dont la limite inférieure est d'environ 350 nm, 365 nm ou 370 nm et la limite supérieure est d'environ 450 nm.

8. Composition pharmaceutique selon l'une quelconque des revendications 1 ou 3 à 7, dans laquelle la température du tissu exposé au rayonnement électromagnétique n'augmente pas de plus de 2, 3, 4 ou 5 degrés Celsius ou n'augmente pas au-delà de 40°C chez des individus humains.

9. Composition pharmaceutique selon l'une quelconque des revendications 1 ou 3 à 8, dans laquelle le dosage du composant actif est diminué d'un facteur de 2 à jusque 10, jusque 100 ou jusque 1 000 par rapport au dosage habituellement prescrit en l'absence d'un traitement par un rayonnement électromagnétique.

10. Composition pharmaceutique selon l'une quelconque des revendications 1 ou 3 à 9 ou procédé selon l'une quelconque des revendications 2 à 8, dans lesquels le composant actif de la composition contient ou est formé d'une quinolone de formule I 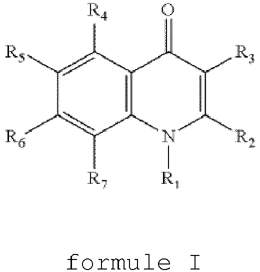 ou de formule II 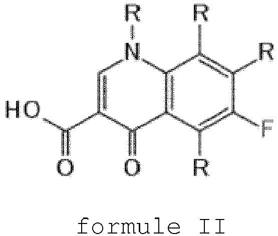 ou dans lesquels le composant actif contient ou est formé d'un dérivé de naphtacène carboxamide polycyclique de formule III ou IV, le composant actif appartenant en particulier au groupe des tétracyclines: 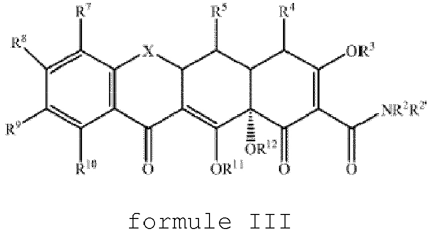 ou

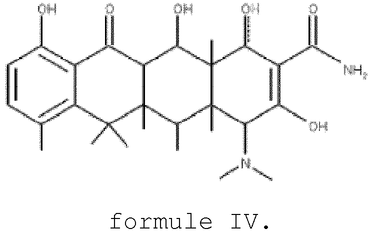

11. Composition pharmaceutique selon l'une quelconque des revendications 1 ou 3 à 10 pour le traitement de l'oeil d'un patient, la composition pharmaceutique étant administrée à l'oeil et de composant actif étant une substance antibactérienne, et dans lesquels les cellules cibles ont acquis antérieurement une résistance au composant actif.
